# EUROPEAN PATENT SPECIFICATION

(11) **EP 1 483 376 B1**
(45) Date of publication and mention of the grant of the patent: **15.10.2008**
(21) Application number: 03744066.6
(22) Date of filing: 08.03.2003
(51) Int. Cl.: C12N 7/01, C12N 15/86

(54) **HIGH THROUGHPUT SYSTEM FOR PRODUCING RECOMBINANT VIRUSES USING SITE-SPECIFIC RECOMBINATION**
HOCHDURCHSATZSYSTEM ZUR HERSTELLUNG REKOMBINANTER VIREN MITTELS STELLENSPEZIFISCHER REKOMBINATION
SYSTEME A HAUT RENDEMENT DE PRODUCTION DE VIRUS DE RECOMBINAISON PAR RECOMBINAISON SPECIFIQUE DE SITE

(30) Priority: 09.03.2002 KR 2002012634
(43) Date of publication of application: 08.12.2004
(73) Proprietor: NEWGEX Inc., Kwanak-gu Seoul (KR)
(72) Inventor: SON, Ho-Sun, Gwanak-gu, Seoul 151-812 (KR); KIM, DO-Hui, Busan 614-858 (KR); JUNG, Neon C., Gwanak-gu, Seoul 151-770 (KR); CHOI, Eun-Wook, Gwanak-gu, Seoul 151-050 (KR); SEEN, Dong-Seung, Seoul 151-812 (KR); KIM, Min-Sung, Gunpo-si, Gyeonggi-do 435-040 (KR); YI, Yong-Weon, Daejeon 302-806 (KR); KIM, Kyung-Jin, Seoul 120-757 (KR)
(74) Representative: W.P. Thompson & Co.
(86) International application number: PCT/KR2003/000454
(87) International publication number: WO 2003/076606

(56) References cited:
- US-A- 6 143 557
- WALHOUT A J M ET AL: "GATEWAY RECOMBINATIONAL CLONING: APPLICATION TO THE CLONING OF LARGE NUMBERS OF OPEN READING FRAMES OR ORFEOMES" METHODS IN ENZYMOLOGY, ACADEMIC PRESS INC, SAN DIEGO, CA, US, vol. 328, 2000, pages 575-592, XP001056139 ISSN: 0076-6879
- HARTLEY ET AL.: 'DNA cloning using in vitro site-specific recombination' GENOME RESEARCH vol. 10, no. 11, November 2000, pages 1788 - 1795, XP002187669
- DWARAKANATH ET AL.: 'The Use of Recombinant Baculoviruses for Sustained Expression of Human Cytomegalovirus Immediate Early Proteins in Fibroblasts' VIROLOGY vol. 284, 2001, pages 297 - 307, XP002987625

## Description

### TECHNICAL FIELD

This invention relates to a method for producing recombinant viruses and vectors for the same. Furthermore, this invention relates to DNA constructs for the method and the recombinant viruses produced by the method.

### BACKGROUND

A recombinant viral vector means a genetically engineered vector, which is used in, for example: the preparation of a vaccine; analysis of function of a gene, a group of genes, or a genomic domain; production of proteins; and gene therapy. Genomic materials, which could be integrated into a viral vector, include any genomic materials such as a gene, cDNA, genomic DNA, DNA sequences encoding peptides or proteins, RNA, anti-sense RNA, siRNA, DNA for si RNA, promoter, enhancer etc. A virus, which is used in the preparation of a vector, includes baculovirus, adenovirus, adeno-associated virus (AAV), retrovirus, Herpes virus, hepatitis B virus (HBV), polioma virus, sindbid virus, and vacxnia virus etc.

Retrovirus is known as one of the most widely used viruses in gene therapy [Clive Patience et al. J Virol, 72:2671-2676, 1998, Marshall, Science, 269: 1050-1055,1995]. Retroviral genome has two LTRs, capsid sequences and 3 (three) coding regions (gag, pol and env), and the method of its preparation and its use *in vitro* and *in vivo* are disclosed (see WO9908692A1 and EP 453242). The clinical application of retroviral vector, however, has the following disadvantages: it forms a replication-competent retrovirus (RCR) (Sharon K. Powell et al. J.Virol., 73:8813-8816, 1999); it has relatively lower level of gene expression, and the level decreases continuously *in vivo;* and the titer of virus is low. Furthermore, the retrovirus cannot transfer a gene to a non-dividing cell [Jaffe, E.M. Cancer Res., 53:2221-2226, 1993; Bender, M.A. et al. J. Virol., 61:1639-1646, 1987].

Adenovirus has a linear genome (sized about 36 kb) and includes replication origin and capsid signals near to the left inverse terminal region (ITR) (103 bp) [Shenk, Adenoviridae: The Viruses and Their Replication. In: Fields BN, Knipe DM Howley PM, ed. Virology. Philadelphia: Raven publishers, 1996: 2111-2148]. Usually, adenoviral vectors have been made by deleting E1 site of the viral genome, which is essential for the viral replication. The viruses including foreign genomic materials substituting E1 site, are transferred to the packaging cells that provide E1 proteins. Thereby, the viral replication occurs only in the packaging cells. Representative packaging cells include, for example, HEK293 cell, 911 cell, and PER. C6 cell, etc. [Hitt MM et al., Advances in Pharmacology, vol. 40, 137-206 (1997), Wang Y. and Huang S., Drug]. Adenoviral vector has advantages such as safty, affinity to various cells, possibility of infecting dividing cells and higher titer (10¹¹ pfu/ml). Thus, adenovirus has been used in the preparation of a vector expressing heterologous genes. Adeno-associated virus (sized about 4700 bp) has ITRs (sized about 145 bp) as replication origins at each terminus. It can be integrated into the genomic DNAs of various types of host cells safely and specifically.

In order to use a recombinant virus as a vector, it should be modified not to replicate themselves in the infected cells. Therefore, defective viruses, which have deletions of some essential regions of genome for viral replication, are usually employed in the preparation of the recombinant viral vectors. As for a retrovirus, for example, gag, pol and/or env genes are deleted, and the regions are replaced with desired genomic materials [Bender et al., J. Virol. 61 (1987) 1639]. Regarding an adenovirus, E1, E2 and/or E4 sites are deleted for the preparation of a viral vector [Levrero et al., Gene 101 (1991) 195; Gosh-Choudhury et al., Gene 50 (1986) 161, Van der Vliet et al., (1975)]. On the other hand, a pseudo-virus vector, which consists of only the essential regions (such as, ITR and capsid sequences) of the replication, has been used also for obtaining recombinant viruses (see WO95/02697).

In addition, baculoviruses, which are known as having circular genomic DNAs, have been used for the preparation of viral vectors. Baculoviridae are infectious on invertebrates, such as insects and crustacea. AcNPV is one of the most widely used baculoviruses, and it contains double strand circular genomic DNAs (sized about 134 kb) (GenBank: NC_001623). Smith et al. developed recombinant baculoviruses by inserting β-interferon genes, and successfully obtained interferon proteins from insect cells using the recombinant baculoviruses (Smith GE., Mol. Cell. Biol., V3, p2156-2165, 1983). Since then, numerous genes were expressed and produced using recombinant baculoviral system. The features of the methods are that: i) it is possible to produce desired recombinant baculoviruses at high efficiency by employing polyhedrin promoter; and ii) it can be used for the expression of genes that do not exhibit their activities when incubated in bacteria. That is because post-translational modification is carried out in insect cells. However, the size of genome of baculovirus made it difficult to engineer the genome through conventional restriction and ligation method. Therefore, homologous recombination has been used in insect cells generally. In order for the homologous recombination, carrier vectors containing desired genes and nucleic acid sequences necessary for the homologous recombination are prepared. Then, insect cells are transfected with the carrier vectors and viral genomic DNAs to induce recombination. In that method, however, since the production efficiency of recombinant viruses is relatively low (0.1-2%), repetitive screening of baculoviral plaques should be performed in order to obtain the desired recombinant viruses. Thus, it is also considered as being an inefficient method.

Kitts PA et al. disclosed an improved method that increased production efficiency of recombinant viruses (Nucleic Acids Res., Oct 1990; 18: 5667 - 5672. Kitts PA et al). They made baculoviral genomic DNAs to be in linear form using restriction enzyme. In Kitts' method, a linker, which includes Bsu36I recognition base sequences, CCTNAGG, or lacZ genes, is inserted to wild-type Autographa californica nuclear polyhedrosis virus (AcNPV) genome at polyherin locus in order to introduce Bsu36I site recognition nucleic acid sequences. Next, the viral DNAs are digested with Bsu36I restriction enzyme, and then transferred to insect cells together with carrier vectors containing desired genomic materials so as to induce homologous recombination. The production efficiency of the desired recombinant viruses is from 26% to 44%. According to that method, linear digested DNA segments, which are not subjected to recombination, are excluded electively from the production of viral vectors. Thus, the desired recombinant viruses expressing desired protein could be generated with relatively high efficiency. However, the method has disadvantages as follows: i) it is not possible to digest Bsu36I recognition sites up to 100% and ii) since the digested DNA segments are fused with each other in insect cells by ligase, selection procedures for the desired viral vectors need be employed.

Peakman TC et al. provided another method using site-specific recombination of cre/loxP system *in vitro* (Peakman TC et al., Nucleic Acids Research, Vol 20, Issue 3 495-500). Briefly, they prepared baculoviruses containing loxP sites and applied them to site-specific recombination *in vitro* with desired genomic materials by reacting the viruses with transfer vectors having the genomic materials flanked with loxP sites, in the presence of cre recombination enzyme. In that method, time-period for producing recombinant viruses is shortened. However, the production efficiency of desired recombinant viruses is still low (0.2% to 49%), thereby it is not suitable to be used as a high-throughput system for the preparation of recombinant viruses.

In addition, Luckow VA et al. reported a method of inserting desired genomic cassette to baculoviral genome by site-specific recombination in a cell (Luckow VA et al., Virol., Aug 1993; 67: 4566 - 4579). According to Luckow's method, firstly, a shuttle vector (sized about 130 kb) is prepared by inserting plasmid replication origin, which replicates in bacteria, kanamycin resistance gene and attTn7 recombination sequences into baculoviral genome. The shuttle was named "Bacmid". DH10Bac was generated by transforming E. coli with Bacmid and helper plasmid, which in turn was transformed using a plasmid containing desired genes and polyhedrin promoter to prepare recombinant Bacmid in E coli. The resulting recombinant Bacmid is isolated and transferred to insect cells to obtain recombinant viruses. According to that method, baculoviruses could be obtained with relatively high efficiency by using site-specific recombination of transposon. That method, however, required complex procedures, for example, cloning desired genomic materials to Tn7 Bacmid carrier vectors; transforming DH10Bac for the recombination of Tn7 baculoviruses; selecting and isolating bacteria having Bacmid that contains desired genomic materials; purifying Bacmid DNAs (sized about 130 kb) from the isolated bacteria; and transferring the DNAs again to insect cells to produce recombinant virues.

US 6143557 discloses recombinational cloning for the use of nucleic acids, vectors and methods, *in vitro* and *in vivo,* for moving or exchanging segments of DNA molecules using engineered recombination sites and recombination proteins to provide chimeric DNA molecules that have the desired characteristic(s) and/or DNA segment(s).

Thus, non of the above-mentioned methods of the prior arts have provided suitable ways to overcome the problems of relatively long time-period in preparing recombinant viruses (about 3 to 6 weeks) and low production efficiency. Therefore, there have been demands for the development of an improved method.

In this regard, we have carried out studies to produce recombinant viruses having desired genomic materials more rapidly and efficiently, thereby we have completed this invention by preparing recombinant viral vectors *in vitro* using site-specific recombination and employing the vectors to produce recombinant viruses. Since the viral vectors obtained *in vitro* according to this invention can be applied to animal cells directly without further procedures like selection, the present invention not only simplifies the total procedures significantly but also provides desired recombinant viruses with up to 100% production efficiency. Thus, higher virus titer can be obtained according to the present invention.

### DETAILED DESCRIPTIONS OF THE INVENTION

Any publications referenced herein are hereby incorporated by reference in this application in order to more fully describe the state of the art to which the present invention pertains.

It is important to understand the present invention to note that all technical and scientific terms used herein, unless otherwise defined, are intended to have the same meaning as commonly understood by one of ordinary skill in the art. The techniques used herein are also those that are known to one of ordinary skill in the art, unless stated otherwise.

Reference to particular buffers, media, reagents, cells, culture conditions and the like, or to some subclass of same, is not intended to be limiting, but should be read to include all such related materials that one of ordinary skill in the art would recognize as being of interest or value in the particular context in which that discussion is presented. For example, it is often possible to substitute one buffer system or culture medium for another, such that a different but known way is used to achieve the same goals as those to which the use of suggested method, material or composition is directed.

It is an object of this invention to provide more rapid and efficient method of producing recombinant viruses.

In order to accomplish this object, this invention employs recombinant viral vectors that are prepared in vitro by site-specific recombination.

In accordance with the present invention, there is provided a method for preparing expressive circular recombinant viral vectors, characterized in that the method comprises the steps of:
i) providing a linearized viral DNA fragment containing at least two site-specific recombination sites and at least one restriction enzyme recognition site;
ii) providing a gene cassette containing desired genomic material flanked with at least two site-specific recombination sites for homologous recombination with the linearized viral DNA fragment at the two site specific recombination sites; and
iii) reacting the linearized viral DNA fragment and the gene cassette together under conditions effective to allow in vitro homologous recombination to take place so as to form a recombinant viral vector; and
iv) preventing the expression of a viral vector that does not contain the desired genomic material.

In accordance with a further aspect of the present invention, there is provided a method of screening recombinant viruses containing desired genomic materials, characterized in that the method comprises the steps of:
i) providing a linearised viral DNA fragment containing at least two site-specific recombination sites and at least one restriction enzyme recognition site;
ii) providing a gene cassette containing desired genomic material flanked with at least two site-specific recombination sites for homologous recombination with the linearized viral DNA fragment at the two site specific recombination sites;
iii) reacting the linearized viral DNA fragment and the gene cassette together under conditions effective to allow the in vitro homologous recombination to take place so as to form a recombinant virusin vitro to produce reaction mixture including expressive circular recombinant viral vectors formed in vitro;
iv) preventing the expression of a virus that does not contain the desired genomic material; and
v) transferring the reaction mixture of the iii) to host cells in multi-well plate.

In accordance with yet a further aspect of the present invention, there is provided the method method of preparing recombinant viruses having circular genomic DNAs, characterized in that the method comprises the steps of:
i) providing a linearised viral DNA fragment containing at least two site-specific recombination sites and at least one restriction enzyme recognition site;
ii) providing a gene cassette containing desired genomic material flanked with at least two site-specific recombination sites for homologous recombination with the linearized DNA fragment at the two site specific recombination sites;
iii) reacting the linearized viral DNA fragment and the gene cassette to create a homologous recombination in vitro to produce reaction mixture including expressive circular recombinant viral vectors;
iv) preventing the expression of a viral vector that does not contain the desired genomic material; and
v) transferring the reaction mixture of the iii) to host cells in multi-well plate.

In the present invention, viral genomic DNAs are made in linear form by restriction enzymes before being applied to site-specific recombination *in vitro*. Since the linear viral genomic DNAs, which are not subjected to the site-specific recombination, cannot replicate by themselves, almost all of the expressed viruses are believed to be those from the circular viral genomic DNAs. Thus, according to the present invention, it is possible to obtain recombinant viruses at high efficiency without the procedures for selecting and isolating the desired recombinant viruses. Accordingly, the method of the present invention can be applied to a virus having circular genomic DNAs, for example, polioma virus, papiloma virus and hepatitis B virus, etc.

Referring to site-specific recombination, it is an event occurring naturally in various living organisms. Enzymes for the site-specific recombination contain not only endonuclease activity but also ligase activity, so they recognize a certain part of DNA sequences and replace it with any other corresponding DNA sequences [Yang W. and Mizuuchi K., Structure, 1997, Vol. 5, 1401-1406(9)]. Int/att system from bacterio λ phage, Cre/LoxP system from P1 bacterio phage and FLP-FRT system from yeast are well developed site-specific recombination systems.

Site-specific recombination system has been used in i) expression control of desired genomic materials cloned into recombinant viruses, ii) excising helper-virus when preparing helper-viral dependent viruses, iii) increasing the efficiency of homologous recombination in packaging cells, or iv) inducing site-specific recombination between carrier vectors and viral genome in packaging cells to prepare recombinant viruses [Philip NG et al., Biotechniques, vol. 29. 524-528 (2000), Philip NG et al., Human Gene therapy, vol. 10., 2667-2672 (1999), Hardy S et al., Journal of Viology, vol. 71., 1842-1849 (1997), Parks RJ et al., Proc. Natl. Acad. Sci USA,, vol 93., 13565-13570 (1996)]. However, the said applications of the site-specific recombination disclosed in the prior arts were merely indirect and supplementary uses *in vivo* or in a cell line, not *in vitro.*

In addition, applications of site-specific recombination in the preparation of recombinant viruses having circular genomic DNAs were reported: i) in the preparation of recombinant baculoviruses in bacteria using Tn7 recombinantion, and ii) in the preparation of recombinant baculoviruses using cre/lox-P system *in vitro.*

However, there have been no applications of site-specific recombination *in vitro* for specific insertion of desired genomic materials into viral genomic DNAs in order to obtain recombinant viral vectors. In particular, there have been no applications to prevent the expression of viral genomic DNAs that do not contain desired genomic materials. Thus, the present invention is characterized by employing site-specific recombination in preparing recombinant viral vectors *in vitro*. The present invention has unexpected advantages compared to the prior arts as follows: i) it is possible to obtain recombinant viral vectors more easily and more rapidly by employing site-specific recombinant *in vitro*; ii) it does not require any further procedure of selecting desired recombinant viruses from intermediate host cells, since the viral genomic DNAs are made in linear form and thereby the linear viral DNAs, which do not form in circular form by integration of the desired genomic materials, cannot be expressed; and iii) it is possible to generate the same kind of genomic materials from various types of viruses or to generate various genomic materials from the same kind of viral type muti-well (e.g. 96-well or 384-well) by employing common transfer vector containing expression cassette that does not have DNAs from viral genome.

Furthermore, according to the present invention, homologous recombination in the packaging cells is not required any more. Nor, particular cell lines for inducing homologous recombination are required. Table 1 and Table 2 show the results of the comparisons between the method of the present invention (BacHTS system) and the methods of the prior arts (BacPAK6 system and BacToBac system) with regard to the processes. With the results, it is clear that the desired recombinant viruses can be obtained even more rapidly by using the method of this invention in comparison to the prior arts.

**Table 1. The comparisons of the number of procedures between the method of the present invention and the methods of prior arts**

| | the number of procedures | days | time-period |
|---|---|---|---|
| BacPAK6 system | 11 | 6 | 10 |
| BacToBac system | 13 | 8 | 12 |
| BacHTS system | 5 | 3 | 7 |

**Table 2. The comparisons of the processes required for preparation of baculoviruses**

| BacPAK system | BacToBac system | BacHTS system |
|---|---|---|
| 1. RE cut | 1. LR reaction | 1. Recombination |
| 2. Agarose gel elution | 2. Transformation | 2. Transfection |
| | 3. Colony seeding | 3. Infection |
| 3. Ligaion | 4. Plasmid purification | 4. Harvest and virus storage |
| 5. Colony seeding | | 5. RE analysis |
| 6. Plasmid purification | 6. DH10Bac | |
| | transformation | |
| 7. RE analysis | 7. Streaking | |
| 8. Transfection | 8. Seeding | |
| 9. Infection | 9. Bacmid purification | |
| 10. Harvest and virus storage | | |
| | 10. Transfection | |
| 11. Protein expression analysis | 11. Infection | |
| | 12. Harvest and virus storage | |
| | 13. Protein expression analysis | |

Furthermore, according to the present invention, since promoter for the expression of the desired genomic materials, and fusion system and polyadenyl sequences for the preparation of fusion protein are provided from parent viral genome, the gene cassette cloned to common carrier vector could be applied to produce various types of viruses.

Thus, the present invention provides significantly useful tools for studying functions of genes of which demands have been increasing greatly since the completion of genome project [Wang Y. and Huang S., Drug Discovery Today, vol. 5, 10-16 (2000)].

In one embodiment, the present invention employs bacuovirus having circular genomic DNAs, and Int/att system from bacteriophage λ (Genebank: NC 001416) as a site-specific recombination system. In the Int/att system, attR site-specific recombination sites within viral genomic DNA fragment react with corresponding attL site-specific recombination sites including desired genomic materials from carrier vector, in the presence of λ integrase recombination enzyme, IHF as a co-factor and Xis, and result in vector DNA fragment containing attB and attP sites [see Hartley JL. Genome Research, 2000, Vol. 10, 1788-1795]. Other recombination systems from various organisms, for example, the Cre/loxP system from bacteriophage P1, and the FLP/FRT system from the Saccharomyces cerevisiae 2 µ circle plasmid, can be employed.

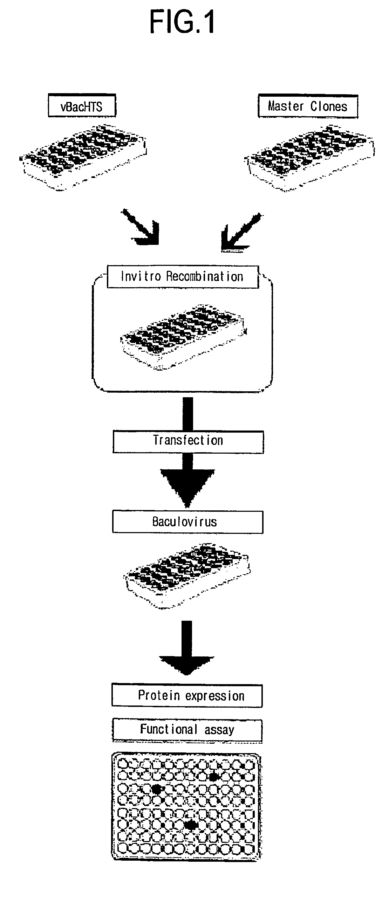

When the mixture is transferred directly to suitable host cells, which are expected to express desired recombinant viruses, only the circular viral genomic DNAs containing attB1-[desired genomic materials]-attB2 can replicate and form viral particles. The linear viral DNA fragment and the vector DNAs themselves, which do not react with each other, and the circular viral DNAs having attP sites cannot replicate and cannot form viral particles. In FIG.1, site-specific recombination between gene cassette and vBacHTS baculoviral DNAs in vitro is presented.

In another embodiment, vBacHTS viral DNAs are constructed to include site-specific recombination sequences and Bsu36I recognition base sequences, which in turn are made to linear form viral DNAs flanked by 2 (two) site-specific sites with treatment of Bsu36I restriction enzyme. The linear vBacHTS DNAs cannot form viral particles since the vBacHTS viral DNAs are digested at 2 (two) Bsu36I recognition sites having nucleic acid sequences different from each other, while the circular form DNAs generated by site-specific recombination formed viral particles efficiently.

In another embodiment, the reaction mixture containing viral DNAs having desired genomic materials, which are integrated by site-specific recombination, is transferred to Sf21 insect cells, and then viral packaging is detected. Thus, according to the present invention, recombinant viruses can be obtained without employing intermediate host cells, such as E. coli or yeast, for selecting and amplifying suitable genomic DNAs. Therefore, the recombinant viruses are suitable for the purpose of expressing desired genes from host cells. The activity measurement of GFP and GUS proteins indicates that the method of the present invention is more efficient than the method of the prior arts.

### BRIEF DESCRIPTIONS OF FIGURES

FIG.1 illustrates gene-screening processes using BacHTS system.
FIG.2 depicts the cleavage map of pBacHTS.
FIG.3 depicts the cleavage map of pBacHTS2.
FIG.4 depicts the cleavage map of pBacPAK8_attR1.
FIG.5 depicts the cleavage map of pBacPAK_attR1R2.
FIG.6 depicts the cleavage map of pBacHTS_GFP.
FIG.7 depicts the cleavage map of pBacHTS_Flag.
FIG.8 depicts the cleavage map of pBacHTS_His.
FIG.9 depicts the cleavage map of pBacHTS_HisGst.
FIG.10 depicts the cleavage map of pBacHTS_Gst.
FIG.11 depicts the cleavage map of pBacHTS2_EGFP.

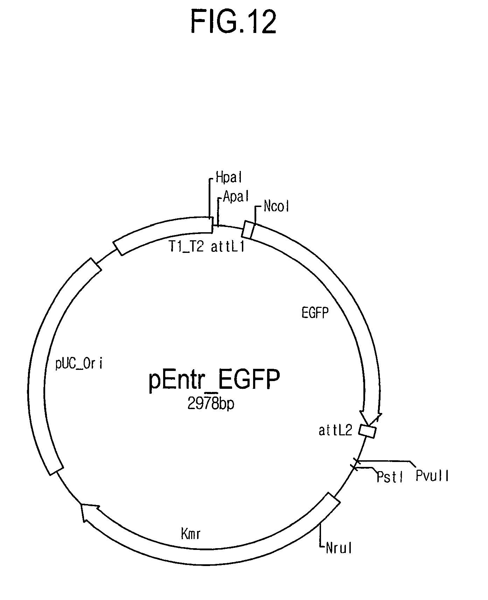

FIG.12 depicts the cleavage map of pEntr_EGFP.
FIG.13 illustrates the process of preparation of vBacHTS virus.

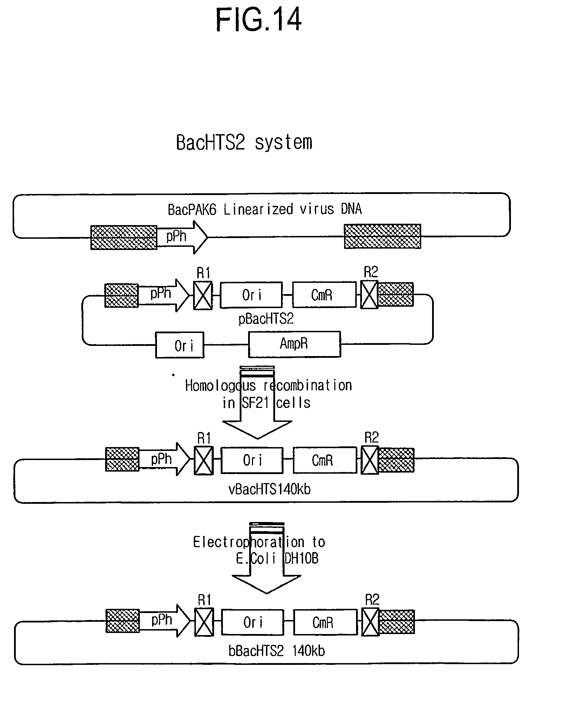

FIG.14 illustrates the process of preparation of bBacHTS2 clone.
FIG.15 shows the map of baculoviral polyhedrin locus.
FIG.16 illustrates agarose gel electrophoretic analysis of vBacHTS viral DNAs and bBacHTS2 viral DNAs.

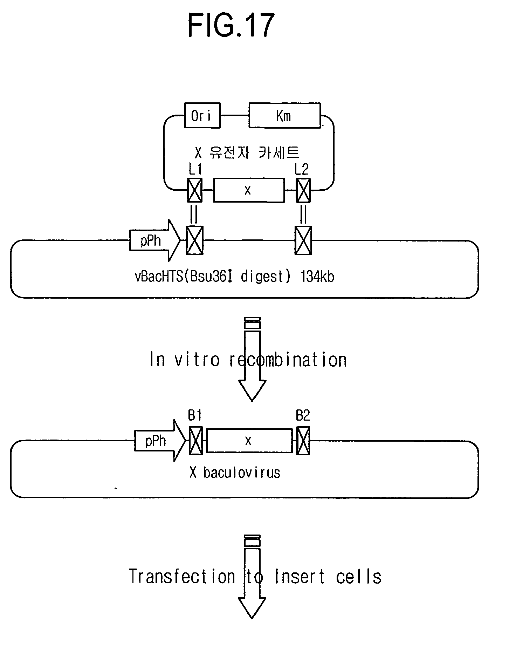

FIG.17 illustrates procedures for the preparation of desired recombinant viruses using vBacHTS and gene cassette.
FIG.18 shows the results of activity assays of GFP baculoviruses and GUS recombinant viruses in vBacHTS system.
FIG.19 shows plaque assays for GFP baculoviruses and GUS recombinant viruses in vBacHTS system.

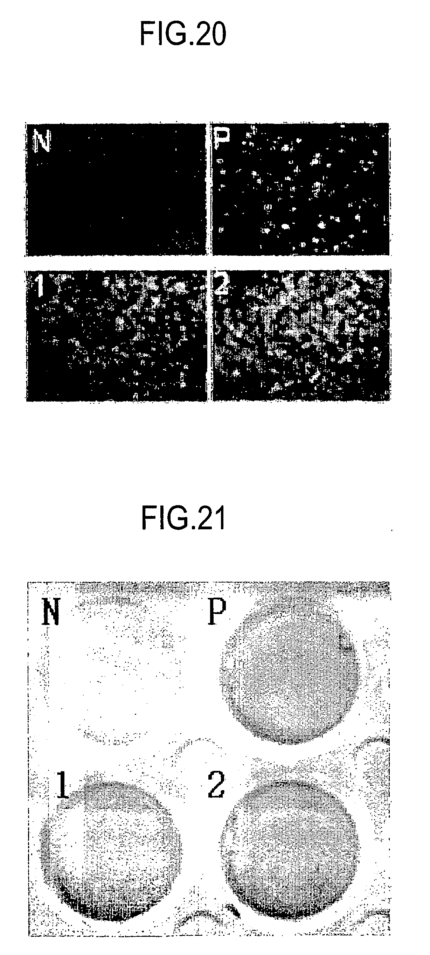

FIG.20 shows the results of activity assays of GFP baculoviruses in bBacHTS2 system.
FIG. 21 shows the results of activity assays of GUS recombinant baculoviruses in bBacHTS2 system.
FIG.22 illustrates GFP image in multi-well plate and the results of western blot.

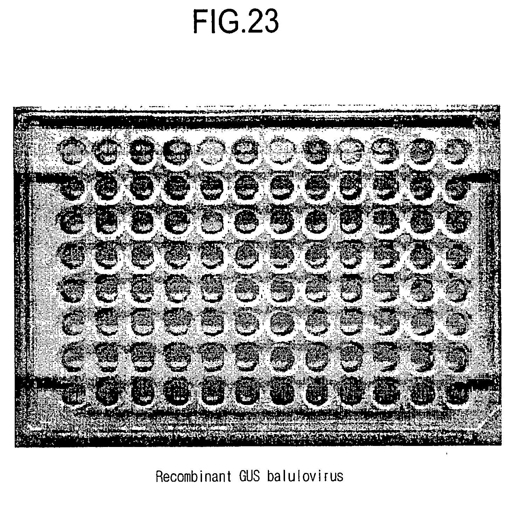

FIG.23 shows enzyme activity using X-Gluc of GUS recombinant viruses in multi-well plate.
FIG.24 shows the results of site-specific recombination *in vitro.*
FIG.25 depicts expression of GFP-LacZ fusion proten in Sf21 cells after 4 days from the transfer of recombinant DNAs to the cells.
FIG.26 shows the results of western blot of the expression of GFP-LacZ fusion protein using SDS-PAGE and GFP antibodies.
FIG.27 depicts LacZ enzyme activity in recombinant virus (GFP-LacZ) using X-Gal substrates.
FIG.28 shows the image of Sf21 cells infected with GFP-YPK baculoviruses from multi-well plate.
FIG.29 shows the results of screening of protein kinase (Rb, Histone H1, MBP).
FIG.30 depicts phosphorylation of Rb (retinoblastoma) protein.
FIG.31 illustrates phosphorylation of Rb protein by #2-A9 protein kinase.

Preferred embodiments of this invention are described in the following examples. Other embodiments within the scope of the claims herein will be apparent to those skilled in the art from consideration of the specification or practice of the invention as disclosed herein. It is intended that the specification, together with the examples, be considered exemplary only, with the scope and the spirit of the invention being indicated by the claims that follow the example. The examples herein are meant to exemplify the various aspects of carrying out the invention and not intended to limit the scope of the invention in any way. The examples do not include detailed descriptions of conventional methods employed, such as in the performance of genomic DNA isolation, PCR, and sequencing procedures. Such methods are well known to those skilled in the art and are described in numerous publications. In addition, all the publications referred herein are integrated hereto as references.

### EXAMPLES

### EXAMPLE 1: Preparation of pBacHTS

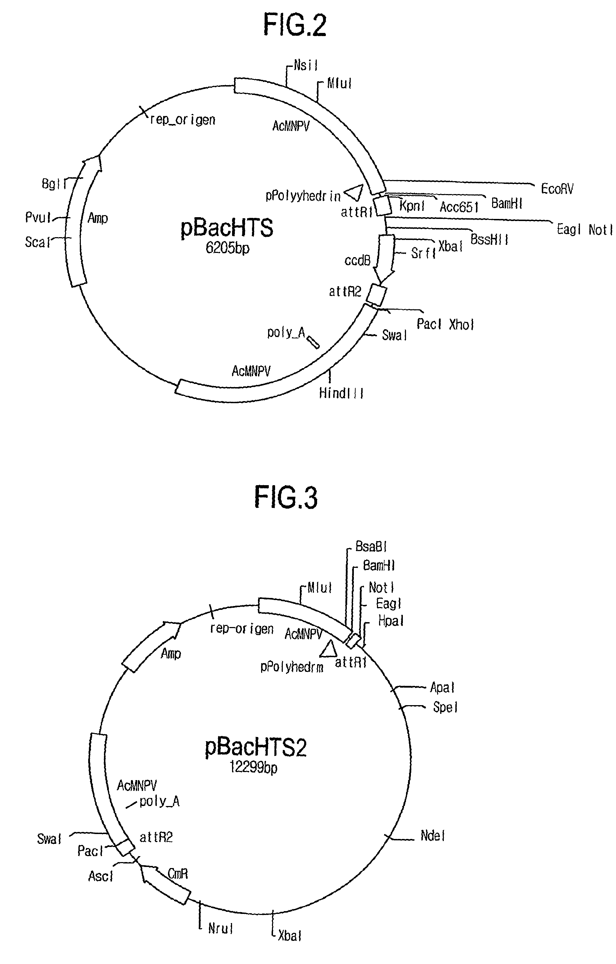

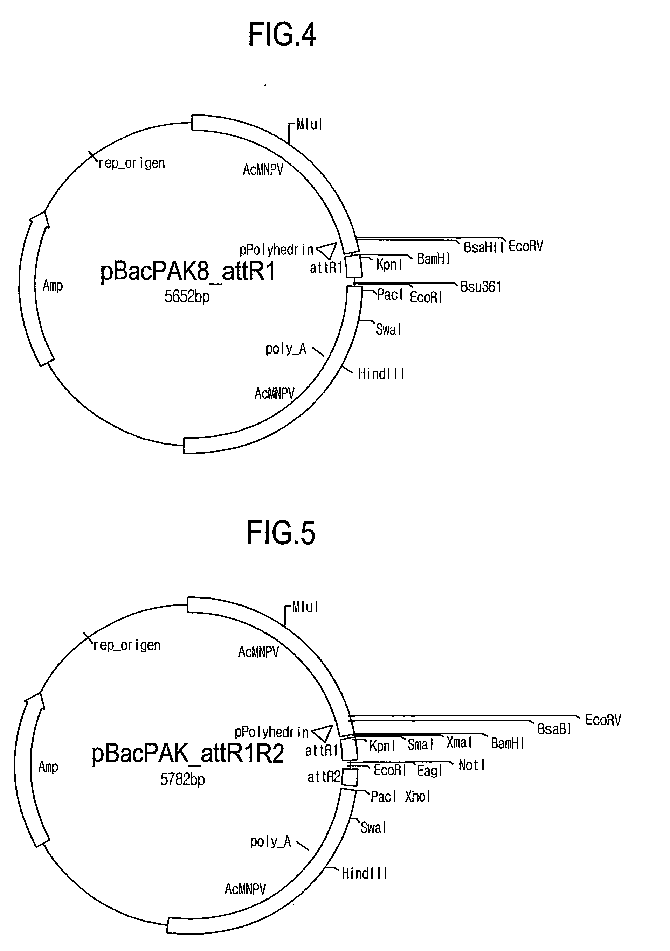

pBacHTS viral vectors were prepared. For the preparation, firstly, pBacPAK8_R1 (see FIG.4) was provided by cloning amplified attR1 sites and Bsu36I recognition base sequences at BamHI/EcoRI sites of a shuttle vector of pBacPAK8 (Genebank: U2446, from Clontech) and transforming DH5α (from LTI, Life Technologies Inc.) with the cloned vector, and then by incubating and screening in agar medium including ampicilin. Likewise, pBacPAK8_R1R2 was prepared by cloning PCR amplified attR2 sites and Bsu36I recognition site sequences at EcoRI/PacI site sequences of pBacPAK8_R1 (see FIG.5). In order to obtain pBacHTS, DNA fragment (427 bp) including ccdB gene from EcoR1 digested fragments of pEntr4 plasmid (from Invitrogen) was inserted to the pBacPAK8_R1R2, and then the resulting plasmid was transferred to DB3.1 cell (from LTI). pBacHTS contained two different Bsu36I recognition sequences between attR1 site and attR2 site (FIG.2). The attR1 sites and the attR2 sites included site-specific recombinase target sequences that reacted with attL1 and attL2 sites, respectively, in the presence of integrase from λ bacteriophage, Xis, IHF-α and IHF-β.

### EXAMPLE 2: Preparation of pBacHTS 6His, pBacHTS_GST, pBacHTS_HisGst, pBacHTS_GFP and pBacHTS_Flag

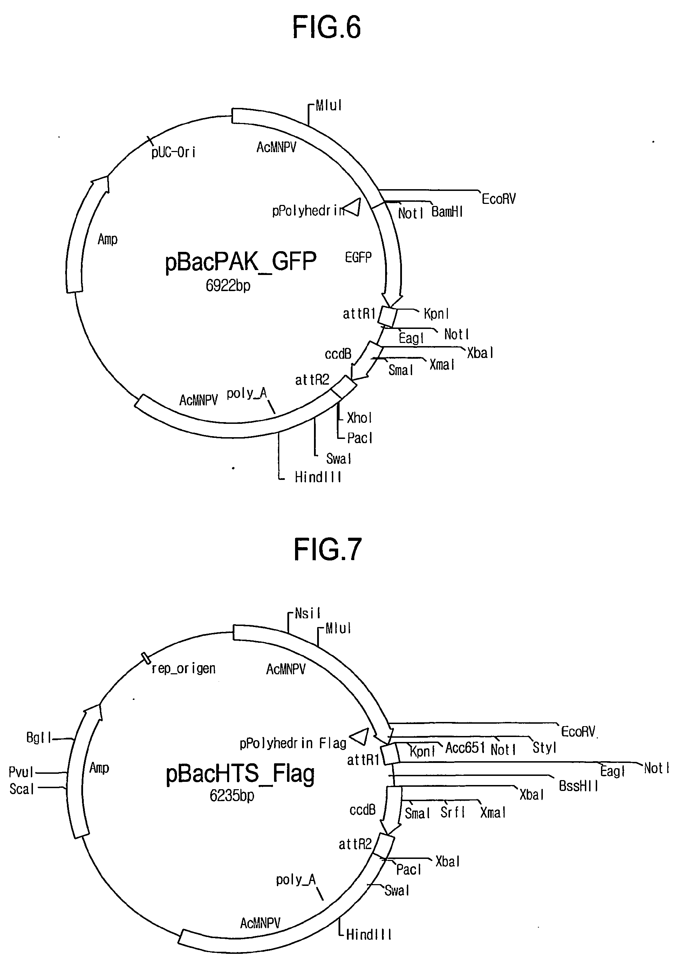

Fusion tag was inserted to the sites in back of polyhedrin promoter in order to prepare recombinant baculovirus expressing fusion proteins. pBacHTS_Gst was designed for the preparation of expression vector for GST fusion protein. In order for this, pGEX-2T (Genbank:U13850) was PCR amplified using a primer including Bg1II and a primer including BamH1 linker, which in turn was treated with BglII and BamHI and then was inserted to pBacHTS/BamHI restriction sites. In addition, pBacHTS_His was designed for the preparation of a vector expressing His tag fusion protein by inserting 6 His linker to BamHI site of pBacHTS. Furthermore, pBacHTS_HisGST was designed by inserting BglII and BamHI treated GST genes, which were PCR amplified, to BamHI site of pBacHTS_His. On the other hand, GFP gene was PCR amplified using a primer containing BamHI linker and a primer containing KpnI linker, which in turn was digested with BamHI/KpnI. After that, the digested gene was integrated into BamHI/KpnI restriction sites of pBacHTS to generate pBacHTS_GFP. Furthermore, pBacHTS_Flag was prepared by inserting a linker expressing Flag tag at BamHI/KpnI sites of pBacHTS (see FIG.7).

### EXAMPLE 3: Preparation of DNAs of vBacHTS, vBacHTS_His, vBacHTS_HisGst, vBacHTS_GST and vBacHTS_GFP viruses

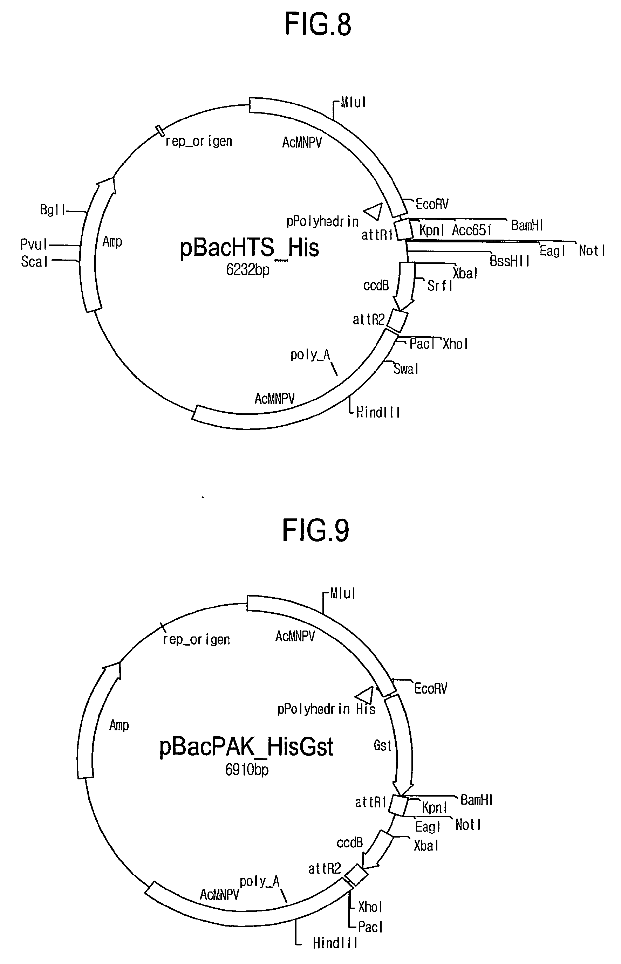

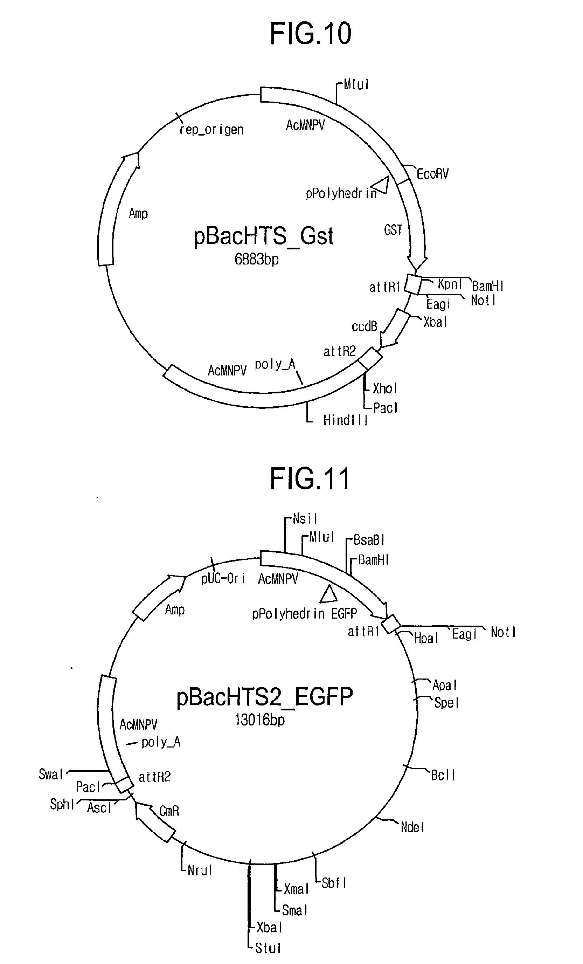

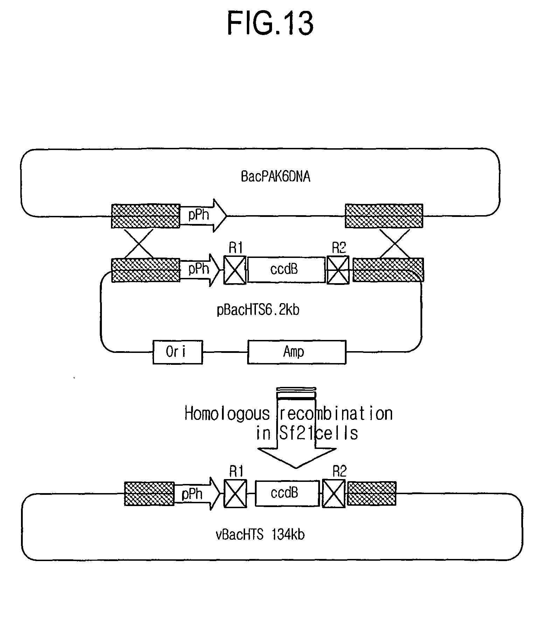

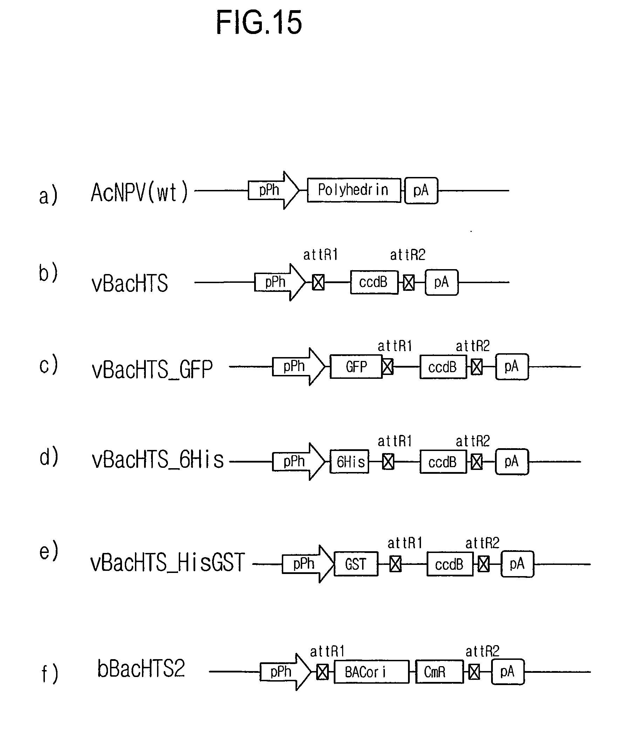

Baculoviruses of which polyhedrin loci were replaced by pBacHTS plasmids were prepared using homologous recombination. Briefly, Sf21 insect cells were transfected with a mixture of pBacHTS plasmid, Bsu36I digested BacPAK6 viral DNAs (from Clontech#6144-1) and Lipofectin (from LTI) to induce homologous recombination in the cells. The recombinant viruses were isolated through twice repeated plaque assays, which in turn were subjected to PCR. The purified viral clone was named vBacHTS virus (SEQ. ID No.1: the sequences of its polyhedrin locus) (see FIG.15). As disclosed in FIG.13, pBacHTS_His (see FIG.8), pBacHTS_HisGst (see FIG.9) pBacHTS_GST (see FIG.10), pBacHTS_GFP (see FIG.6) were respectively transferred together with BacPAK6 virus to generate vBacHTS_His (SEQ. ID No.2: the sequences of its polyhedrin locus), vBacHTS_HisGst (SEQ. ID No.3: the sequences of its polyhedrin locus), vBacHTS_GST (SEQ. ID No.4: the sequences of its polyhedrin locus) and vBacHTS_GFP (SEQ. ID No.5: the sequences of its polyhedrin locus) (see FIG.15). The sequences of the obtained viruses are the same as those of the wild type AcNPV (GenBank: NC_001623) except for the above-designated sequences of the polyhedrin loci. Viral plaques were selected from agarose medium.

### EXAMPLE 4: Purification of viral DNAs of vBacHTS, vBacHTS_His, vBacHTS_HisGst, vBacHTS_GFP, and vBacHTS

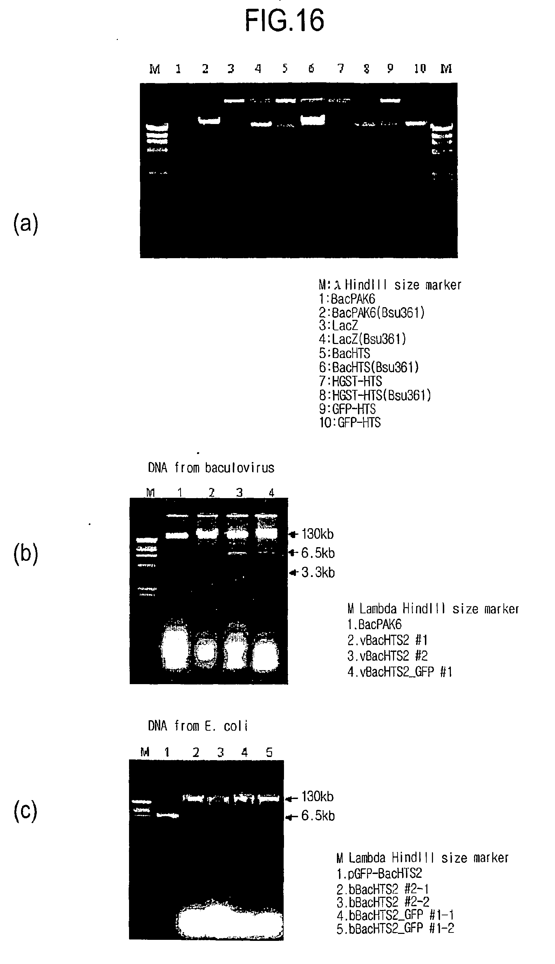

Viral culture medium having 1.2x10⁸ pfu/ml of viral titer was produced after 3 days from infecting Sf21 cells on cell culture dish (100 mm diameter) in order to isolate vBacHTS viral DNAs. Then, 1.25x10⁷ of Sf21 cells were plated again on cell culture dish (150 mm diameter), which in turn were infected with 20 times larger numbers of viruses (MOI=20). After incubating for 48 hrs, about 25 ml of cell culture medium per 1 dish was obtained. A total of 150 ml of cell culture medium from 6 dishes (150 mm diameter) was centrifugated at 20,000 rpm for 90 min, and the resulting precipitated viral particles were recovered (with Hanil, Supra22k). The recovered viral particles were suspended in 2ml of TE (10mM Tris-HCl pH=8.0, 1mM EDTA), and then were treated with sucrose concentration gradient ultra-centrifuge (Beckman SW41 rotor) at 30,000 rpm. Viral particles were purified from the layer between 50% and 40% sucrose solution. The purified viruses were subjected to centrifugation again at 18,000 rpm for 90 min. After that, the precipitates of viral particles were suspended again with 2 ml of TE (10mM Tris-HCl pH=8.0, 1mM EDTA), and were left at 42°C for 2 hrs after adding 0.5% SDS, 1% beta-mercaptoethanol and 0.2 mg of ProteinaseK thereto, so as to isolate viral envelopes. The viral lysate was extracted twice with the same amount of phenol/chloroform solution, and ethanol was added to the extract, thereby pure viral DNAs were obtained as precipitates. Then, 50 units of restriction enzyme Bsu36I (from New England Bio labs Inc. NEB#524) were added to the purified DNAs (10 ug). The mixture was left at 37°C for 2 hrs before used in the following studies (see FIG.16).

### EXAMPLE 5: Preparation of gene cassette

A pEntr_GFP gene cassette was prepared by inserting PCR products of GFP between attL1 site and attL2 site. pEntr_Gus gene cassette from LTI was employed.

### EXAMPLE 6: Preparation of the desired recombinant viruses having GFP gene or GUS gene

Recombinant viruses expressing GFP and Gus were generated in this study. The pEntr_GFP gene cassette (100 ng) or the pEntr_Gus gene cassette (100 ng) was transferred to a test-tube, and reacted with 100 ng of Bsu36I treated vBacHTS viral DNAs at 25°C for 2 hrs, in the presence of integrase mixture comprising 4 ul of integrase, Xis, IHF-α and IHF-β. Then, Sf21 cells were infected with the reaction mixture together with lipofectin (from LTI), thereby recombinant viruses were generated. After 2 days, green fluorescence was observed from the insect cells including GFP gene cassette. Furthermore, after 4 days, almost every cell showed green fluorescence. Furthermore, fluorescence and symptoms of viral infection were observed in the infected cells infected.

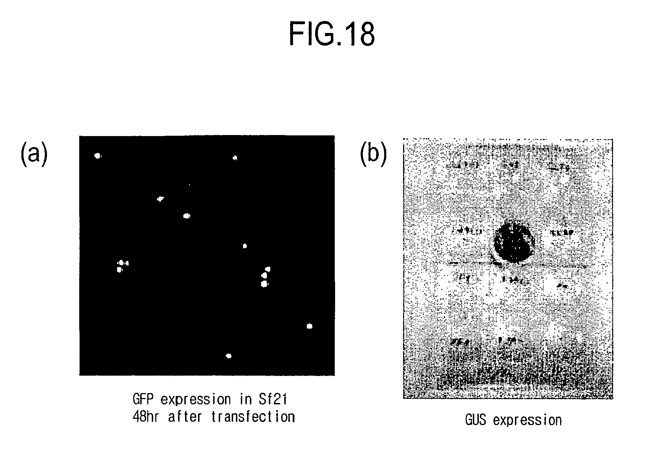

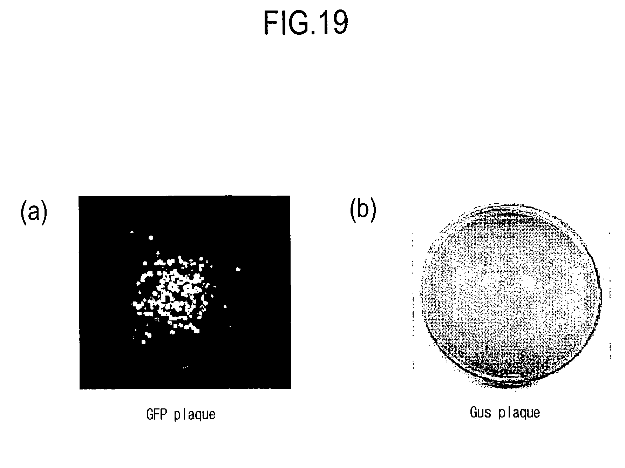

On the other hand, a mixture containing pEntr_Gus gene cassette was transferred to Sf21 cells. After 4 days, X-Gluc was added to the cells. After 2 hrs of the reaction, the Sf21 cells became blue, which supported the expression of GUS (see FIG.18). In order to determine the productivity of the recombinant viruses, 4-day viral culture was dilated and transferred to insect cells. Next, plaques were detected 4 days after adding culture medium containing 1% low-melting point agarose to the infected cells. As the result, most plaques represented blue fluorescence (see FIG.19).

### EXAMPLE 7: Determination of the production efficiency of recombinant viruses

Recombinant viruses expressing GFP were prepared in this study. In order for this, 100 ng of pEntr_GFP gene cassette containing GFP genes and 200 ng of viral DNAs of Bsu36I treated vBacHTS, vBacHTS_His or vBacHTS_HisGST were reacted in the presence of 4ul of integrase mixture comprising integrase, Xis (exisionase), IHF-α and IHF-β at 25 °C for 6 hrs. The resulting mixture was transferred to Sf21 cells together with lipofectin (from LTI) in order to generate recombinant baculoviruses. After incubating at 27 °C for 48 hrs, the expression of GFP was detected under fluorescence microscope. Since the cells, in which baculoviruses replicated, represented fluorescence, the efficiency of recombinant viral expression was determined by counting the cells showing green fluorescence. In the meantime, BacPAK6 viral DNAs (from Clontech) treated with Bsu36I and 500 ng of pBacPAK_GFP were transferred to Sf21 cells, then the expression of GFP in the Sf21 cells was monitored.

**Table 3. The expression efficiency of GFP baculoviruses**

| Virus DNA | Bsu36I | gene cassette | GFP positive cell# |
|---|---|---|---|
| BacPAK6 | Yes | pBacPAK8 EGFP | 154 |
| VbacHTS | | pEntr EGFP | 1752 |
| VbacHTS | yes | pEntr EGFP | 572 |
| VbacHTS His | | pEntr EGFP | 2236 |
| VbacHTS His | yes | pEntr EGFP | 654 |
| VbacHTS HisGST | | pEntr EGFP | 1433 |
| VbacHTS HisGST | yes | pEntr EGFP | 475 |

As the result, the efficiency of the viral expression is much higher in the method of the present invention compared to the prior arts (see Table 3).

### EXAMPLE 8: Detection of the efficiency of recombinant viral expression

Recombinant viruses expressing Gus were prepared, and the number of plaques expressing Gus genes was counted. In order for this, firstly, 100 ng of pEntr_Gus gene cassette containing Gus genes and 200 ng of each of viral DNAs of Bsu36I treated vBacHTS, vBacHTS_His or vBacHTS_HisGST were reacted in the presence of 4 ul of integrase mixture comprising integrase, exisionase, IHF-α and IHF-β at 25°C for 6 hrs. The resulting mixture was transferred to Sf21 cells together with lipofectin (from LTI) to generate recombinant baculobiruses. Plaque assays were carried out for the viral culture medium. On the other hand, in order to detect productivity of the recombinant viruses, 4-day viral culture medium was dilated and transferred to insect cells. Subsequently, the cells were incubated for 4 days after adding medium containing 1% low-melting point agarose thereto. The numbers of plaques expressing Gus genes were counted after 4 days from the addition of 100 ul of 0.33% nutral red water solution and 25 ul of X-Gluc (20 mg/ml in DMSO) and afer subsequent dye treatment (see Table 4).

**Table 4. The efficiency of GUS recombinant viral expression**

| Virus DNA | Bsu36I | gene cassette | Total | Recombinant | rate % |
|---|---|---|---|---|---|
| BacPAK6 | yes | pBacPAK8 Gus | 46 | 41 | 89% |
| VbacHTS | | pEntr Gus | 28 | 25 | 89% |
| VbacHTS | yes | pEntr Gus | 57 | 57 | 100% |
| VbacHTS His | | pEntr Gus | 44 | 36 | 82% |
| VbacHTS His | yes | pEntr Gus | 46 | 45 | 98% |
| VbacHTS HisGST | | pEntr Gus | 82 | 57 | 70% |
| VbacHTS HisGST | yes | pEntr Gus | 32 | 32 | 100% |
| VbacHTS GFP | | pEntr Gus | 27 | 22 | 81% |
| VbacHTS GFP | yes | pEntr Gus | 21 | 20 | 95% |

As the result, most of the plaques represented blue color when they were treated with X-Gluc, which supported that the efficiency of GUS viral generation was very high in comparison to the prior arts.

### EXAMPLE 9: High-throughput preparation of recombinant viruses in multi-well plate

Desired recombinant viruses were prepared simultaneously from multi-well plate. For this purpose, the vBacHTS viral DNAs were treated with Bsu36I restriction enzyme. In this study, GFP gene cassette was employed to confirm the generation of recombinant baculoviruses and gene expression. The GFP gene cassettes were incubated in 96-deep well plate simultaneously and purified using automatic device. All the processes were carried out in multi-well plate while recombination reaction, insect cell incubation, and viral infection were performed using 8-channel pipette. About 50 ng of gene cassette and 200 ng of vBacHTS baculoviral DNAs were reacted in the presence of 2 ul of recombinase and 4 ul of buffer at 25 °C for 12 hrs. A total of 20 ul of reaction mixture was used in this reaction. In order to detect the insertion of gene cassette, PCR amplification was performed using primers for polyhedrin locus amplification.

Baculo-Forward primer: 5-actgttttcgtaacagttttg-3 (SEQ. ID No.: 6)

Baculo-Reverse primer: 5-acaacgcacagaatctagc-3 (SEQ. ID No.: 7)

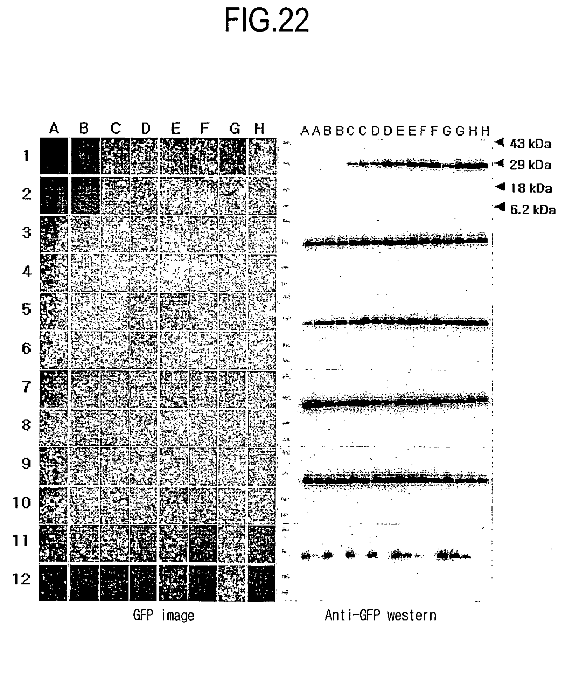

As the result, recombinant viral DNAs were generated with very high efficiency. Meanwhile, liposome was prepared by mixing 5 ul of the recombinant reaction mixture with 5 ul of 10% lipofectin dilates, which in turn was transferred to 50, 000 of Sf21 cells in 96-well plate (SPL) followed by the incubation of the cells for 4 days. Protein expression was assayed after 3-day incubation of 50,000 of the Sf21 cells that were infected again with 10 ul of viruses. As for GFP viruses, the expression of green fluorescence was detected under the fluorescence microscope. And, the exhibition of symptoms of GFP viral infection was detected also. The cells were isolated from each of the wells with SDS-PAGE, and the expression of GFP was assayed using GFP-antibody with western blot. With regard to GFP proteins, the measured value showed similar pattern as detected under the fluorescence microscope (see FIG.22).

### EXAMPLE 10: High-throughput preparation and enzyme activity screening of LacZ gene mutants in 96-well plate using GFP recombinant baculoviruses

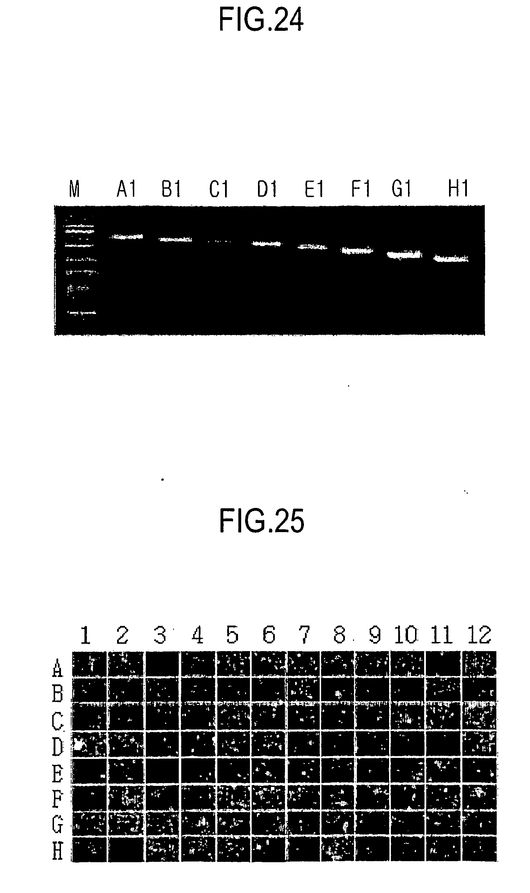

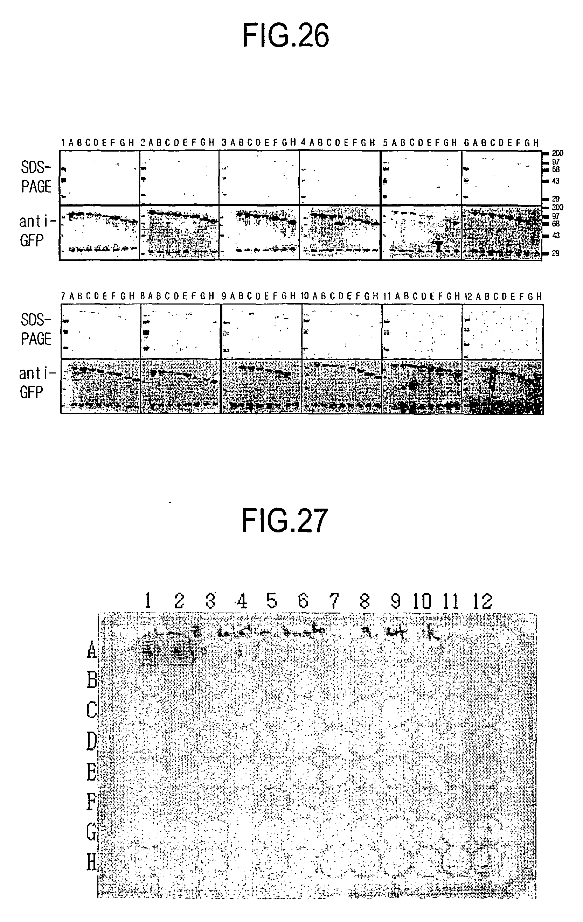

In order to prepare desired recombinant baculoviruses (sized about 1-3.2 kb) from multi-well plate simultaneously, the vBacHTS_GFP viral DNAs were treated with Bsu36I restriction enzyme. Gene cassettes of pEntr_LacZdel were employed in this study. The gene cassettes were prepared in 96-well plate by PCR cloning of the gene of β-galactosidase from E. coli (sized about 3.2 kb), wherein the gene was deleted of 3'-terminus. More specifically, pEntr_LacZ was prepared by PCR cloning of gene of LacZ. Afterward, the pEntr_LacZ was treated with ExoIII/S1 deletion kit (#K0421) (from Fermentas) to generate of pEntr_LacZdel. All the processes were carried out in multi-well plate, while the recombination reaction, insect cell incubation and viral infection were performed with 8-channel pipette. 50 ng of gene cassettes and 200 ng of vBacHTS baculoviral DNAs were reacted in the presence of 2 ul of recombines and 4 ul of buffer at 25 °C for 12 hrs. A total of 20 ul of reaction mixture was used in this reaction. In order to detect the insertion of gene cassette, PCR amplification was performed using a pair of primers (actgttttcgtaacagttttg and acaacgcacagaatctagc) for the amplification of polyhedrin locus. As the result, recombinant viruses were generated at very high efficiency (see FIG.25). Meanwhile, liposome was prepared by mixing 5 ul of the recombinant reaction mixture and 5 ul of 10% lipofectin dilates, which in turn was transferred to 50,000 of Sf21 cells in 96-well plate (SPL) followed by incubation of the cells for 4 days (see FIG.26). In order to assay the expression of GFP fusion proteins, SDS-PAGE analysis and western blotting using GFP antibodies were carried out (see FIG.26).

In order to screen the gene activities, 1.5 ul of X-Gal (25 mg/ml in DMSO) of developer of β-galactosidase was added to each well that contained viruses incubated for 3 days after the primary infection. After 12 hrs of reaction, viruses containing β-galactosidase activity were detected in 2 (two) wells (see FIG.27).

### EXAMPLE 11: High-throughput heterologous gene expression using recombinant baculoviruses in 96-well plate and the screening of enzyme activity using the same

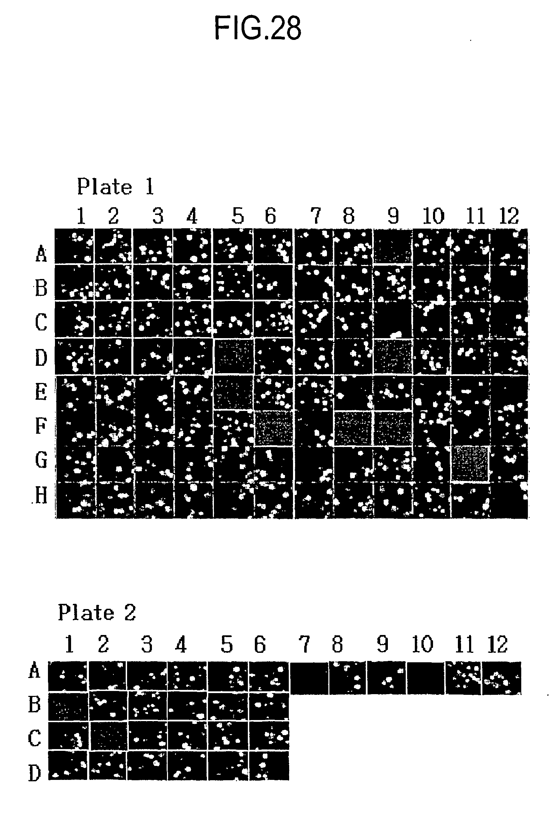

*S. cerevisie,* which includes 124 protein kinase genes, was employed in this study. PCR amplification and cloning were carried out using gene-specific primers to obtain 112 gene cassettes from the *S*. *cerevisie* genome. Recombinant baculoviruses were prepared by the recombination of the gene cassettes and vBacHTS_GFP vector DNA. The respective recombinant viruses represented particular fluorescence according to the proteins fused to GFP (see FIG.28). Also, the recombinant baculoviruses having 32 human cDNA were prepared in the same manner.

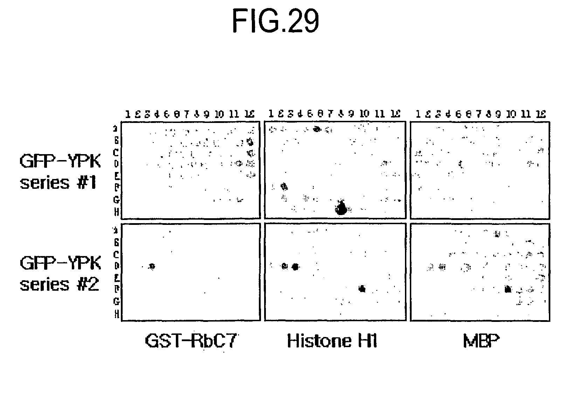

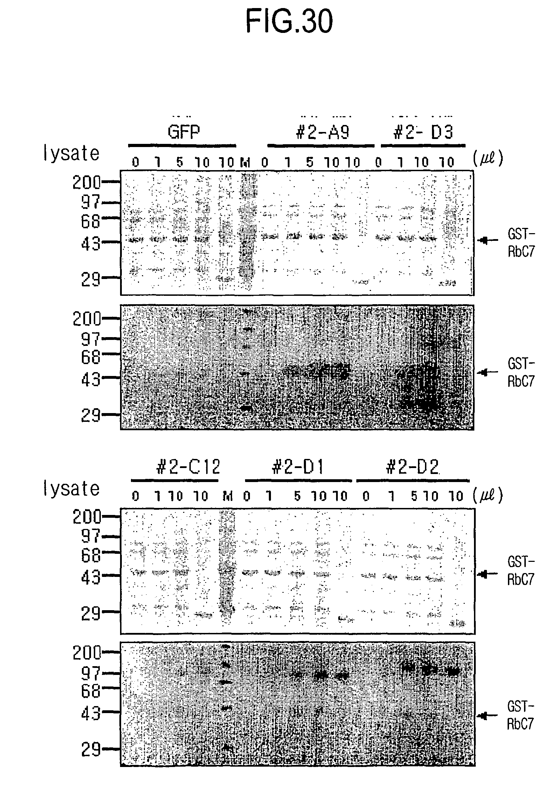

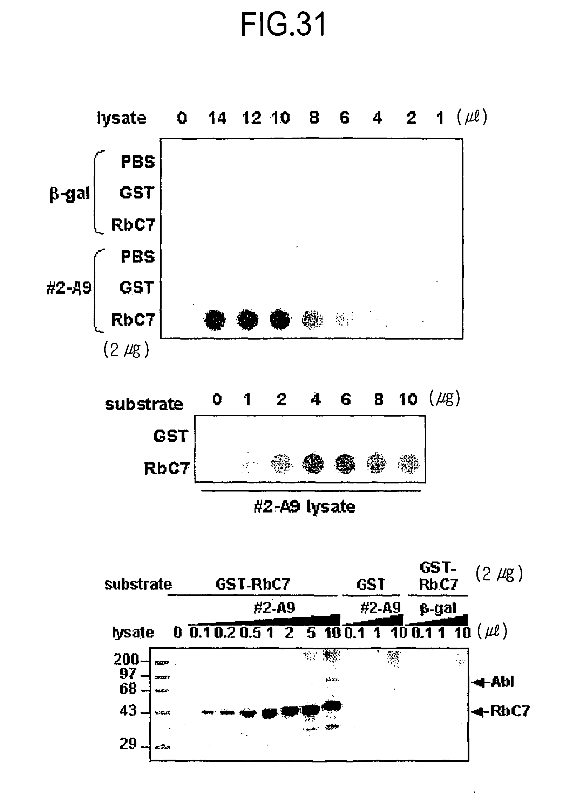

Firstly, cells were infected with these viruses and incubated for 3 days. The cell lysate was generated using a buffer to lyse cells. Viruses, which exhibited protein kinase activities to substrates of Histon H1, MBP (myelin basic protein) and Rb were screened. 2 ug of protein substrates (histon H1, MBP and Rb) were reacted with 10 ul of cell extracts at 30 °C for 10 min, in the presence of 10uM ATP, 0.2 uCi of P32-δ-labeled ATP (gamma, P32 ATP), and 2 ul of phosphate buffer [200 mM Tris-HCl (pH=8.0), 100 mM of MgCl₂, 10 mM of EGTA and 10 mM of DTT]. After stopping the reaction by adding 120 ul of 1% phosphoric acid solution, the reaction mixture was transferred to PVDF membrane (from Milipore, #MAIP-N45). Afterward, the membrane was washed with detergent [10 mM Tris-HCl (pH=8.0), 1 mM EDTA and 150 mM NaCl] four times and dried, which in turn was exposed to phosphoscreen at room temperature, and then signals were detected with Phosphoimager (from MD or Fuji). As the result, it was observed that protein kinase activities were increased in some viruses (see FIG.29). Subsequently, SDS-PAGE electophoretic analysis was carried out to confirm the specificity of protein phosphorylation. At that time, the level of Rb protein phosphorylation with regard to the concentration of protein kinase included in cell extracts was detected, while varying the concentrations of cell extracts. As the result, only the cell extracts of the baculoviruses expressing plate#2-D3 protein and plate#2-A9 protein increased Rb protein phosphorylation in a concentration dependent manner (see FIG.30). Meanwhile, proteins of plate#2-D1 and plate#2-D2 exhibited very weak phosphorylation activities. And, it was acknowledged that the positive signals from the proteins of plate#2-D1 and plate#2-D2 were resulted from auto-phosphorylation activities rather than from Rb protein phosphorylation. Rb protein phosphorylations by plate#2-D3 and plate#2-A9 proteins exhibited a typical protein phosphorylation pattern in that phosphorylation of Rb protein was dependent on the concentrations of the substrate and the enzyme (FIG.31). Thus, it is possible to find out new useful proteins from baculoviral libraries obtained from the high-throughput system of the present invention according to the present invention.

### EXAMPLE 12: The preparation of pBacHTS2 and pBacHTS2_GFP

Baculoviral transfer vector of pBacHTS2 was prepared in order to generate vBacHTS2 viruses (SEQ. ID No.8: the sequences of its polyhedrin locus) that can replicate and can be applied to recombination reaction *in vitro*. The sequences of the obtained vBacHTS2 virus are the same as those of the wild type AcNPV (GenBank: NC_001623) except for the above-designated sequences of the polyhedrin locus. The pBacHTS2 had BAC vector originated replication origin and CmR (chloramphenichol resistance gene) at Bsu36I restriction enzyme recognition site located between attR1 site and attR2 site thereof. Thus, it was a kind of BAC vectors that can replicate in bacteria. Replacing the BAC vector originated replication origin and CmR with desired gene cassette generated vBacHTS viruses *in vitro.*

In order to prepare pBacHTS2, firstly, 6.5 kb of DNA fragment containing BAC (Bacterial artificial chromosome) vector replication origin and CmR was generated using pBACe3.6 (Genbank:U80929) as a template. Then, PCR amplification was carried out using 10 pmole of a pair of sequences having Bsu36I recognition sequences, 200 uM of dNTPs and 2.5 U of Pfu turbo polymerase (Stratagene) with PCR cycler (Applied Biosystem, Gene Amp PCR System 2700). A total of 50 ul of reaction mixture was used. PCR was carried out for 20 cycles (DNA denaturation at 95 °C, 30 sec, DNA extension at 60 °C, 30 sec, DNA amplification at 72 °C, 7 min). PCR products of 6.5 kb of DNA fragment and vBacHTS vector were digested by Bsu36I, and were reacted in the presence of T4 ligase at 16 °C overnight. E. coli (DH5a) was transformed using the resulting mixture, which in turn was incubated with Cm and Amp medium (for selection) to produce pBacHTS2. Likewise, Bsu36I treated PCR products of 6.5 kb of DNA fragment were inserted to Bsu36I site of the pBacHTS_GFP vector so as to obtain pBacHTS2_GFP vector.

### EXAMPLE 13: Preparation of viruses having site-specific recombination sites

Baculoviruses were prepared using homologous recombination, which replicated in bacteria. Briefly, the homologous recombination was induced in Sf21 cells by transferring a mixture of pBacHTS2 plasmid, Bsu36I digested BacPAK66 DNA and Lipofectin (from LTI). The infected cells were incubated at 27 °C for 4 days, and were transferred to 5x10⁶ of Sf21 cells (in 100 mm of dish). Thereby, high titer of viruses was obtained. Then, precipitates of viral particles were produced by centrifugation after adding 2ml of 50% PEG6000 to the cell culture medium. Next, proteinase K was added to the mixture at 42 °C and left the mixture alone for 2 hrs to dismantle viral envelope. The viral lysate was extracted twice using the same amount of phenol/chloroform, and ethanol was added thereto. Thereby, precipitated and purified vBacHTS2 viral DNAs were obtained. Then, DH10B cell lines (from LTI) were transformed with the purified DNAs using MicroPulser (from Bio-Rad), which are in turn incubated in Cm medium to form bacterial colony. The bacteria were incubated in 1 liter of 2× YT medium (10 g of Yeast extract powder, 16 g of tryptone, 5 g of NaCl within per 1 (one) Liter), then DNAs were purified and named as bBacHTS2 (FIG.16). Thus, according to this method, it is possible to reduce the required time-period and cost significantly, since the viral DNAs can be obtained from bacterial culture without the need of incubation in insect cells. Purified 10 ug of DNAs was digested with 50 unit of Bsu36I at 37 °C for 5 hrs, which in turn was heat treated at 80 °C for 20 min to inactivate Bsu36I enzyme. Likewise, vBacHTS2_GFP (SEQ. ID No.9: the sequences of its polyhedrin locus) was prepared from pBacHTS2_GFP vector by homologous recombination, and then was transferred to DH10B by electrophoration. The DNAs were purified and named as bBacHTS2_GFP (see FIG.16). The sequences of the vBacHTS2_GFP virus are the same as those of the wild type AcNPV (GenBank: NC_001623) except for the above-designated sequences of the polyhedrin locus.

### EXAMPLE 14: Preparation of the desired recombinant viruses using vBacHTS2 and vBacHTS2_GFP DNAs generated from bacteria

50 ng of the pEntr_GFP and the pEntr_Gus gene cassettes were reacted with 200 ng of Bsu36I treated vBacHTS2 viral DNAs in the presence of 1 ul of integrase mixture in vitro at 25 °C for 12 hrs. The same reaction was carried out except for the use of the pEntr_Gus gene cassette instead of pEntr_GFP gene cassette. PCR was performed as disclosed in the Examples mentioned above. PCR amplification was carried out using the reaction mixture, as templates, and a pair of primers amplifying viral polyhedrin locus to confirm whether recombination reaction occurred. A total of 20 cycles of PCR were carried out using each of 10 pmole of the primers and 1 tube of Bioneer premix kit, in which one cycle of PCR was performed at 95 °C for 30 sec, 50 °C for 30 sec and 72 °C for 3 min. After completing PCR reaction, electrophoresis was carried out for the PCR products on 1% agarose gel to confirm efficient performance of recombination. The gene cassette reaction mixture was transferred to Sf21 cells together with 5 ul of Lipofectin (from LTI) so as to obtain baculoviruses. Green fluorescence was observed in insect cells, after 2 days from transferring GFP gene cassette reaction mixture thereto. In addition, most of the cells represented green fluorescence after 4 days from transfer (FIG.25). Sf21 cells were infected again with the viral culture medium to confirm green fluorescence and the symptoms of infection. Likewise, pEntr_Gus gene cassette was reacted with Bsu36I treated vBacHTS2 DNAs at 25 °C for 12 hrs. Then, the obtained reaction mixture was transferred to Sf21 cells and was incubated at 27 °C for 4 days. When 6 ul of X-Gluc (20 mg/ml in DMSO) was added to incubated cells, all the culture medium represented strong blue, which supported not only the expressions of Gus genes but also efficient generation of recombinant viruses containing Gus genes.

### INDUSTRIAL APPLICABILITY

As mentioned above, according to the present invention, it is possible to prepare recombinant viruses more rapidly and more easily. Also, it is possible to obtain high titer viruses at high efficiency. In the present invention, since site-specific recombination is employed *in vitro*, the desired genes can be inserted into any target site of viral genome accurately, and also numerous viruses can be generated simultaneously. Accordingly, the present invention can be applied to high-throughput system to produce hundreds or thousands of viruses to study functions of genes at the same time, as well as to obtain several recombinant viruses. Furthermore, recombinant baculoviral library can be produced easily according to the present invention, and the library can be used in the screening of the desired genomic materials.
## Claims

1. A method for preparing expressive circular recombinant viral vectors, **characterized in that** the method comprises the steps of:
i) providing a linearized viral DNA fragment containing at least two site-specific recombination sites and at least one restriction enzyme recognition site;
ii) providing a gene cassette containing desired genomic material flanked with at least two site-specific recombination sites for homologous recombination with the linearized viral DNA fragment at the two site specific recombination sites; and
iii) reacting the linearized viral DNA fragment, and the gene cassette together under conditions effective to allow *in vitro* homologous recombination to take place so as to form a recombinant viral vector; and
iv) preventing the expression of a viral vector that does not contain the desired genomic material.

2. The method for preparing expressive circular recombinant viral vectors as claimed in claim 1, wherein one or more viral DNA fragments are digested with restriction enzymes at one or more sites to generate the linearized DNA fragments.

3. The method for preparing expressive circular recombinant viral vectors as claimed in claim 2, wherein said one or more restriction enzyme sites comprise(s) the nucleotide sequences of CCTNAGG.

4. The method for preparing expressive circular recombinant viral vectors as claimed in claim 1, wherein the recombinant viral vector is obtained from a group consisting of baculovirus, poliomavirus, papilomavirus and hepatitis B virus (HBV).

5. A method of screening recombinant viruses containing desired genomic materials, **characterized in that** the method comprises the steps of:
i) providing a linearised viral DNA fragment containing at least two site-specific recombination sites and at least one restriction enzyme recognition site;
ii) providing a gene cassette containing desired genomic material flanked with at least two site-specific recombination sites for homologous recombination with the linearized viral DNA fragment at the two site specific recombination sites;
iii) reacting the linearized viral DNA fragment and the gene cassette together under conditions effective to allow the *in vitro* homologous recombination to take place so as to form a recombinant virus *in vitro* to produce reaction mixture including expressive circular recombinant viral vectors formed *in vitro*;
iv) preventing the expression of a virus that does not contain the desired genomic material; and
v) transferring the reaction mixture of the iii) to host cells in multi-well plate.

6. A method of screening recombinant viruses as claimed in claim 4, wherein the method further comprises the step of:
vi) identifying the features expressed in host cells.

7. The method of screening recombinant viruses as claimed in claim 5 or 6, wherein the virus is baculovis, poliomavirus, papilomavirus, and hepatitis B virus (HBV).

8. A method of preparing recombinant viruses having circular genomic DNAs, **characterized in that** the method comprises the steps of:
i) providing a linearised viral DNA fragment containing at least two site-specific recombination sites and at least one restriction enzyme recognition site;
ii) providing a gene cassette containing desired genomic material flanked with at least two site-specific recombination sites for homologous recombination with the linearized DNA fragment at the two site specific recombination sites;
iii) reacting the linearized viral DNA fragment and the gene cassette to create a homologous recombination *in vitro* to produce reaction mixture including expressive circular recombinant viral vectors;
iv) preventing the expression of a viral vector that does not contain the desired genomic material; and
v) transferring the reaction mixture of the iii) to host cells in multi-well plate.

9. The method of preparing recombinant viruses having circular genomic DNAs as claimed in claim 8,
wherein the linearized viral DNA fragment is non-infectious to animal cells or cannot replicate, while includes bacterial replication origin and antibiotics resistance sequences.

10. The method of preparing recombinant viruses having circular genomic DNAs as claimed in claim 8,
wherein the transfer of the reaction mixture to host cells is carried out without the need for a selection process to isolate the circular genomic DNA.

11. The method of preparing recombinant viruses having circular genomic DNAs as claimed in claim 7, wherein viral genomic DNAs of i) are digested with restriction enzyme at one or more sites to generate the linearized viral DNA fragments.

12. The method of preparing Recombinant viruses having circular genomic DNAs as claimed in claim 10, wherein said one or more sites comprise(s) the nucleotide sequences of CCTNAGG.

13. The method of preparing recombinant viruses having circular genomic DNAs as claimed in claim 8, wherein the recombinant viral vectors is obtained from a group consisting of baculovirus, poliomavirus, papilomavirus, and hepatitis B virus (HBV).

## Patentansprüche

1. Verfahren zum Herstellen expressiver, zirkulärer, rekombinanter Virusvektoren, **dadurch gekennzeichnet, dass** das Verfahren die folgenden Schritte umfasst:
i) Bereitstellen eines linearisierten Virus-DNA-Fragments, enthaltend mindestens zwei ortsspezifische Rekombinationsstellen und mindestens eine Restriktionsenzym-Erkennungsstelle;
ii) Bereitstellen einer Genkassette, enthaltend das angestrebte genomische Material, das von mindestens zwei ortsspezifischen Rekombinationsstellen für die homologe Rekombination mit dem linearisierten Virus-DNA-Fragment an den beiden ortsspezifischen Rekombinationsstellen flankiert ist; und
iii) zusammen zur Reaktion bringen des linearisierten Virus-DNA-Fragments und der Genkassette unter wirksamen Bedingungen, um zu erlauben, dass die homologe Rekombination *in vitro* stattfinden kann, um auf diese Weise einen rekombinanten Virusvektor zu bilden; und
iv) Verhindern der Expression eines Virusvektors, der nicht das angestrebte genomische Material enthält.

2. Verfahren zum Herstellen expressiver, zirkulärer, rekombinanter Virusvektoren nach Anspruch 1, worin ein oder mehr Virus-DNA-Fragment(e) mit Restriktionsenzymen an einer oder mehr Stelle(n) zum Herbeiführen der linearisierten DNA-Fragmente verdaut wird/werden.

3. Verfahren zum Herstellen expressiver, zirkulärer, rekombinanter Virusvektoren nach Anspruch 2, worin eine oder mehr Restriktionsenzymstelle(n) die Nukleotidsequenzen von CCTNAGG umfasst/umfassen.

4. Verfahren zum Herstellen expressiver, zirkulärer, rekombinanter Virusvektoren nach Anspruch 1, worin der rekombinante Virusvektor aus einer Gruppe erhalten wird, bestehend aus dem Baculovirus, Polyomavirus, Papillomavirus und Hepatitis B-virus (HBV).

5. Verfahren zum Screening rekombinanter Viren, enthaltend die angestrebten genomischen Materialien, **dadurch gekennzeichnet, dass** das Verfahren die folgenden Schritte umfasst:
i) Bereitstellen eines linearisierten Virus-DNA-Fragments, enthaltend mindestens zwei ortsspezifische Rekombinationsstellen und mindestens eine Restriktionsenzym-Erkennungsstelle;
ii) Bereitstellen einer Genkassette, enthaltend das angestrebte genomische Material, das von mindestens zwei ortsspezifischen Rekombinationsstellen für die homologe Rekombination mit dem linearisierten Virus-DNA-Fragment an den beiden ortsspezifischen Rekombinationsstellen flankiert ist;
iii) zusammen zur Reaktion bringen des linearisierten Virus-DNA-Fragments und der Genkassette unter wirksamen Bedingungen, um zu erlauben, dass die homologe Rekombination *in vitro* stattfinden kann, um auf diese Weise ein rekombinantes Virus *in vitro* zum Herstellen eines Reaktionsgemischs, einschließlich *in vitro* gebildeter expressiver, zirkulärer, rekombinanter Virusvektoren, zu bilden;
iv) Verhindern der Expression eines Virus, das nicht das angestrebte genomische Material enthält; und
v) Transferieren des Reaktionsgemischs von iii) an Wirtszellen in Multiwell-Platten.

6. Verfahren zum Screening rekombinanter Viren nach Anspruch 4, worin das Verfahren weiter die folgenden Schritte umfasst:
vi) Identifizierten der in Wirtszellen exprimierten Merkmale.

7. Verfahren zum Screening rekombinanter Viren nach Anspruch 5 oder 6, worin das Virus ein Baculovirus, Polyomavirus, Papillomavirus und Hepatitis B-Virus (HBV) darstellt.

8. Verfahren zum Herstellen rekombinanter Viren mit zirkulären genomischen DNAs, **dadurch gekennzeichnet, dass** das Verfahren die folgenden Schritte umfasst:
i) Bereitstellen eines linearisierten Virus-DNA-Fragments, enthaltend mindestens zwei ortsspezifische Rekombinationsstellen und mindestens eine Restriktionsenzym-Erkennungsstelle;
ii) Bereitstellen einer Genkassette, enthaltend das angestrebte genomische Material, das von mindestens zwei ortsspezifischen Rekombinationsstellen für die homologe Rekombination mit dem linearisierten DNA-Fragment an den beiden ortsspezifischen Rekombinationsstellen flankiert ist;
iii) zur Reaktion bringen des linearisierten Virus-DNA-Fragments und der Genkassette zum Herbeiführen einer homologen Rekombination *in vitro* zum Herstellen eines Reaktionsgemischs, einschließlich expressiver, zirkulärer, rekombinanter Virusvektoren;
iv) Verhindern der Expression eines Virusvektors, der nicht das angestrebte genomische Material enthält; und
v) Transferieren des Reaktionsgemischs von iii) an Wirtszellen in Multiwell-Platten.

9. Verfahren zum Herstellen rekombinanter Viren mit zirkulären genomischen DNAs nach Anspruch 8, worin das linearisierte Virus-DNA-Fragment für Tierzellen nicht infektiös ist oder nicht replizieren kann, obgleich es den bakteriellen Rcplikationsursprung und Antibiotikaresistenzsquenzen einschließt.

10. Verfahren zum Herstellen rekombinanter Viren mit zirkulären genomischen DNAs nach Anspruch 8, worin der Transfer des Reaktionsgemischs an Wirtszellen ohne die Notwendigkeit für einen Selektionsprozess zum Isolieren der zirkulären genomischen DNA durchgeführt wird.

11. Verfahren zum Herstellen rekombinanter Viren mit zirkulären genomischen DNAs nach Anspruch 7, worin die genomischen Virus-DNAs von i) mit Restriktionsenzym an einer oder mehr Stelle(n) zum Herbeiführen der linearisierten Virus-DNA-Fragmente verdaut werden.

12. Verfahren zum Herstellen rekombinanter Viren mit zirkulären genomischen DNAs nach Anspruch 10, worin eine oder mehr Stelle(n) die Nukleotidsequenzen von CCTNAGG umfasst/umfassen.

13. Verfahren zum Herstellen rekombinanter Viren mit zirkulären genomischen DNAs nach Anspruch 8, worin die rekombinanten Virusvektoren aus einer Gruppe erhalten werden, bestehend aus dem Baculovirus, Polyomavirus, Papillomavirus und Hepatitis B-Virus (HBV).

## Revendications

1. Méthode de préparation de vecteurs viraux recombinés circulaires expressifs, **caractérisée en ce que** la méthode comprend les étapes consistant à :
i) fournir un fragment d'ADN viral linéarisé contenant au moins deux sites de recombinaison site-spécifiques et au moins un site de reconnaissance d'enzyme de restriction ;
ii) fournir une cassette génétique contenant le matériau génomique désiré flanqué d'au moins deux sites de recombinaison site-spécifiques pour une recombinaison homologue avec le fragment d'ADN viral linéarisé au niveau des deux sites de recombinaison site-spécifiques ; et
iii) faire réagir ensemble le fragment d'ADN viral linéarisé et la cassette génétique dans des conditions efficaces pour permettre une recombinaison homologue *in vitro,* de façon à former un vecteur viral recombiné ; et
iv) empêcher l'expression d'un vecteur viral qui ne contient pas le matériau génomique désiré.

2. Méthode de préparation de vecteurs viraux recombinés circulaires expressifs selon la revendication 1, dans laquelle un ou plusieurs fragments d'ADN viral sont digérés par des enzymes de restriction au niveau d'un ou plusieurs sites afin de générer les fragments d'ADN linéarisés.

3. Méthode de préparation de vecteurs viraux recombinés circulaires expressifs selon la revendication 2, dans laquelle lesdits un ou plusieurs sites d'enzyme de restriction comprennent les séquences nucléotidiques de CCTNAGG.

4. Méthode de préparation de vecteurs viraux recombinés circulaires expressifs selon la revendication 1, dans laquelle le vecteur viral recombiné est obtenu à partir du groupe constitué par le baculovirus, le polyomavirus, le papillomavirus et le virus de l'hépatite B (HBV).

5. Méthode de criblage de virus recombinés contenant des matériaux génomiques désirés, **caractérisée en ce que** la méthode comprend les étapes consistant à :
i) fournir un fragment d'ADN viral linéarisé contenant au moins deux sites de recombinaison site-spécifiques et au moins un site de reconnaissance d'enzyme de restriction ;
ii) fournir une cassette génétique contenant le matériau génomique désiré flanqué d'au moins deux sites de recombinaison site-spécifiques pour une recombinaison homologue avec le fragment d'ADN viral linéarisé au niveau des deux sites de recombinaison site-spécifiques ;
iii) faire réagir ensemble le fragment d'ADN viral linéarisé et la cassette génétique dans des conditions efficaces pour permettre une recombinaison homologue *in vitro,* de façon à former un virus recombiné *in vitro* afin de produire un mélange réactionnel comportant des vecteurs viraux recombinés circulaires expressifs formés *in vitro* ;
iv) empêcher l'expression d'un virus qui ne contient pas le matériau génomique désiré ; et
v) transférer le mélange réactionnel de iii) dans des cellules hôtes dans une plaque à puits multiples.

6. Méthode de criblage de virus recombinés selon la revendication 4, dans laquelle la méthode comprend en outre l'étape consistant à :
vi) identifier les caractéristiques exprimées dans les cellules hôtes.

7. Méthode de criblage de virus recombinés selon la revendication 5 ou 6, dans laquelle le virus est le baculovirus, le polyomavirus, le papillomavirus et le virus de l'hépatite B (HBV).

8. Méthode de préparation de virus recombinés possédant des ADN génomiques circulaires, **caractérisée en ce que** la méthode comprend les étapes consistant à :
i) fournir un fragment d'ADN viral lméarisé contenant au moins deux sites de recombinaison site-spécifiques et au moins un site de reconnaissance d'enzyme de restriction ;
ii) fournir une cassette génétique contenant le matériau génomique désiré flanqué d'au moins deux sites de recombinaison site-spécifiques pour une recombinaison homologue avec le fragment d'ADN linéarisé au niveau des deux sites de recombinaison site-spécifiques ;
iii) faire réagir le fragment d'ADN viral linéarisé et la cassette génétique pour créer une recombinaison homologue *in vitro,* afin de produire un mélange réactionnel comportant des vecteurs viraux recombinés circulaires expressifs ;
iv) empêcher l'expression d'un vecteur viral qui ne contient pas le matériau génomique désiré ; et
v) transférer le mélange réactionnel de iii) dans des cellules hôtes dans une plaque à puits multiples.

9. Méthode de préparation de virus recombinés possédant des ADN génomiques circulaires selon la revendication 8, dans laquelle le fragment d'ADN viral linéarisé n'est pas infectieux vis-à-vis de cellules animales ou ne peut se répliquer, tout en comportant une origine de réplication bactérienne et des séquences de résistance aux antibiotiques.

10. Méthode de préparation de virus recombinés possédant des ADN génomiques circulaires selon la revendication 8, dans laquelle le transfert du mélange réactionnel dans des cellules hôtes est réalisé sans qu'un processus de sélection de l'ADN génomiques circulaire ne soit nécessaire.

11. Méthode de préparation de virus recombinés possédant des ADN génomiques circulaires selon la revendication 7, dans laquelle les ADN génomiques viraux de i) sont digérés par une enzyme de restriction au niveau d'un ou plusieurs sites afin de générer les fragments d'ADN viral linéarisés.

12. Méthode de préparation de virus recombinés possédant des ADN génomiques circulaires selon la revendication 10, dans laquelle lesdits un ou plusieurs sites comprennent les séquences nucléotidiques de CCTNAGG.

13. Méthode de préparation de virus recombinés possédant des ADN génomiques circulaires selon la revendication 8, dans laquelle les vecteurs viraux recombinés sont obtenus à partir du groupe constitué par le baculovirus, le polyomavirus, le papillomavirus et le virus de l'hépatite B (HHV).


<110> Neurogenex Co., Ltd.
<120> HIGH THROUGHPUT SYSTEM FOR PRODUCING RECOMBINANT VIRUSES USING SITE-SPECIFIC RECOMBINATION
<130> PN22782.00
<150> KR10-2002-0012634
   <151> 2002-03-09
<160> 9
<170> Kopatentln 1.71
<210> 1
   <211> 10000
   <212> DNA
   <213> Artificial Sequence
<220>
   <223> Polyhedrin locus of vBacHTS virus
<220>
   <221> - promoter
   <222> (4428)..(4520)
   <223> F pPolyhedrin
<220>
   <221> misc_feature
   <222> (4084)..(5109)
   <223> F ccdB
<220>
   <221> misc_feature
   <222> (5126)..(5250)
   <223> R attR2
<220>
   <221> misc_feature
   <222> (4537)..(4661)
   <223> F attR1
<400> 1
   gaattctacc cgtaaagcga gtttagtttt gaaaaacaaa tgacatcatt tgtataatga      60
   catcatcccc tgattgtgtt ttacaagtag aattctatcc gtaaagcgag ttcagttttg     120
   aaaacaaatg agtcatacct aaacacgtta ataatcttct gatatcagct tatgactcaa     180
   gttatgagcc gtgtgcaaaa catgagataa gtttatgaca tcatccactg atcgtgcgtt     240
   acaagtagaa ttctactcgt aaagccagtt cggttatgag ccgtgtgcaa aacatgacat     300
   cagcttatga ctcatacttg attgtgtttt acgcgtagaa ttctactcgt aaagcgagtt     360
   cggttatgag ccgtgtgcaa aacatgacat cagcttatga gtcataatta atcgtgcgtt     420
   acaagtagaa ttctactcgt aaagcgagtt gaaggatcat atttagttgc gtttatgaga     480
   taagattgaa agcacgtgta aaatgtttcc cgcgcgttgg cacaactatt tacaatgcgg     540
   ccaagttata aaagattcta atctgatatg ttttaaaaca cctttgcggc ccgagttgtt     600
   tgcgtacgtg actagcgaag aagatgtgtg gaccgcagaa cagatagtaa aacaaaaccc     660
   tagtattgga gcaataatcg atttaaccaa cacgtctaaa tattatgatg gtgtgcattt     720
   tttgcgggcg ggcctgttat acaaaaaaat tcaagtacct ggccagactt tgccgcctga 780
   aagcatagtt caagaattta ttgacacggt aaaagaattt acagaaaagt gtcccggcat 840
   gttggtgggc gtgcactgca cacacggtat taatcgcacc ggttacatgg tgtgcagata 900
   tttaatgcac accctgggta ttgcgccgca ggaagccata gatagattcg aaaaagccag 960
   aggtcacaaa attgaaagac aaaattacgt tcaagattta ttaatttaat taatattatt 1020
   tgcattcttt aacaaatact ttatcctatt ttcaaattgt tgcgcttctt ccagcgaacc 1080
   aaaactatgc ttcgcttgct ccgtttagct tgtagccgat cagtggcgtt gttccaatcg 1140
   acggtaggat taggccggat attctccacc acaatgttgg caacgttgat gttacgttta 1200
   tgcttttggt tttccacgta cgtcttttgg ccggtaatag ccgtaaacgt agtgccgtcg 1260
   cgcgtcacgc acaacaccgg atgtttgcgc ttgtccgcgg ggtattgaac cgcgcgatcc 1320
   gacaaatcca ccactttggc aactaaatcg gtgacctgcg cgtctttttt ctgcattatt 1380
   tcgtctttct tttgcatggt ttcctggaag ccggtgtaca tgcggtttag atcagtcatg 1440
   acgcgcgtga cctgcaaatc tttggcctcg atctgcttgt ccttgatggc aacgatgcgt 1500
   tcaataaact cttgtttttt aacaagttcc tcggtttttt gcgccaccac cgcttgcagc 1560
   gcgtttgtgt gctcggtgaa tgtcgcaatc agcttagtca ccaactgttt gctctcctcc 1620
   tcccgttgtt tgatcgcggg atcgtacttg ccggtgcaga gcacttgagg aattacttct 1680
   tctaaaagcc attcttgtaa ttctatggcg taaggcaatt tggacttcat aatcagctga 1740
   atcacgccgg atttagtaat gagcactgta tgcggctgca aatacagcgg gtcgcccctt 1800
   ttcacgacgc tgttagaggt agggccccca ttttggatgg tctgctcaaa taacgatttg 1860
   tatttattgt ctacatgaac acgtatagct ttatcacaaa ctgtatattt taaactgtta 1920
   gcgacgtcct tggccacgaa ccggacctgt tggtcgcgct ctagcacgta ccgcaggttg 1980
   aacgtatctt ctccaaattt aaattctcca attttaacgc gagccatttt gatacacgtg 2040
   tgtcgatttt gcaacaacta ttgtttttta acgcaaacta aacttattgt ggtaagcaat 2100
   aattaaatat gggggaacat gcgccgctac aacactcgtc gttatgaacg cagacggcgc 2160
   cggtctcggc gcaagcggct aaaacgtgtt gcgcgttcaa cgcggcaaac atcgcaaaag 2220
   ccaatagtac agttttgatt tgcatattaa cggcgatttt ttaaattatc ttatttaata 2280
   aatagttatg acgcctacaa ctccccgccc gcgttgactc gctgcacctc gagcagttcg 2340
   ttgacgcctt cctccgtgtg gccgaacacg tcgagcgggt ggtcgatgac cagcggcgtg 2400
   ccgcacgcga cgcacaagta tctgtacacc gaatgatcgt cgggcgaagg cacgtcggcc 2460
   tccaagtggc aatattggca aattcgaaaa tatatacagt tgggttgttt gcgcatatct 2520
   atcgtggcgt tgggcatgta cgtccgaacg ttgatttgca tgcaagccga aattaaatca 2580
   ttgcgattag tgcgattaaa acgttgtaca tcctcgcttt taatcatgcc gtcgattaaa 2640
   tcgcgcaatc gagtcaagtg atcaaagtgt ggaataatgt tttctttgta ttcccgagtc 2700
   aagcgcagcg cgtattttaa caaactagcc atcttgtaag ttagtttcat ttaatgcaac 2760
   tttatccaat aatatattat gtatcgcacg tcaagaatta acaatgcgcc cgttgtcgca 2820
   tctcaacacg actatgatag agatcaaata aagcgcgaat taaatagctt gcgacgcaac 2880
   gtgcacgatc tgtgcacgcg ttccggcacg agctttgatt gtaataagtt tttacgaagc 2940
   gatgacatga cccccgtagt gacaacgatc acgcccaaaa gaactgccga ctacaaaatt 3000
   accgagtatg tcggtgacgt taaaactatt aagccatcca atcgaccgtt agtcgaatca 3060
   ggaccgctgg tgcgagaagc cgcgaagtat ggcgaatgca tcgtataacg tgtggagtcc 3120
   gctcattaga gcgtcatgtt tagacaagaa agctacatat ttaattgatc ccgatgattt 3180
   tattgataaa ttgaccctaa ctccatacac ggtattctac aatggcgggg ttttggtcaa 3240
   aatttccgga ctgcgattgt acatgctgtt aacggctccg cccactatta atgaaattaa 3300
   aaattccaat tttaaaaaac gcagcaagag aaacatttgt atgaaagaat gcgtagaagg 3360
   aaagaaaaat gtcgtcgaca tgctgaacaa caagattaat atgcctccgt gtataaaaaa 3420
   aatattgaac gatttgaaag aaaacaatgt accgcgcggc ggtatgtaca ggaagaggtt 3480
   tatactaaac tgttacattg caaacgtggt ttcgtgtgcc aagtgtgaaa accgatgttt 3540
   aatcaaggct ctgacgcatt tctacaacca cgactccaag tgtgtgggtg aagtcatgca 3600
   tcttttaatc aaatcccaag atgtgtataa accaccaaac tgccaaaaaa tgaaaactgt 3660
   cgacaagctc tgtccgtttg ctggcaactg caagggtctc aatcctattt gtaattattg 3720
   aataataaaa caattataaa tgctaaattt gttttttatt aacgatacaa accaaacgca 3780
   acaagaacat ttgtagtatt atctataatt gaaaacgcgt agttataatc gctgaggtaa 3840
   tatttaaaat cattttcaaa tgattcacag ttaatttgcg acaatataat tttattttca 3900
   cataaactag acgccttgtc gtcttcttct tcgtattcct tctctttttc atttttctcc 3960
   tcataaaaat taacatagtt attatcgtat ccatatatgt atctatcgta tagagtaaat 4020
   tttttgttgt cataaatata tatgtctttt ttaatggggt gtatagtacc gctgcgcata 4080
   gtttttctgt aatttacaac agtgctattt tctggtagtt cttcggagtg tgttgcttta 4140
   attattaaat ttatataatc aatgaatttg ggatcgtcgg ttttgtacaa tatgttgccg 4200
   gcatagtacg cagcttcttc tagttcaatt acaccatttt ttagcagcac cggattaaca 4260
   taactttcca aaatgttgta cgaaccgtta aacaaaaaca gttcacctcc cttttctata 4320
   ctattgtctg cgagcagttg tttgttgtta aaaataacag ccattgtaat gagacgcaca 4380
   aactaatatc acaaactgga aatgtctatc aatatatagt tgctgatatc atggagataa 4440
   ttaaaatgat aaccatctcg caaataaata agtattttac tgttttcgta acagttttgt 4500
   aataaaaaaa cctataaata cggatcccgg ggtaccacaa gtttgtacaa aaaagctgaa 4560
   cgagaaacgt aaaatgatat aaatatcaat atattaaatt agattttgca taaaaaacag 4620
   actacataat actgtaaaac acaacatatc cagtcactat ggcggccgca ttaggcaccc 4680
   cttaggaatt cgcttactaa aagccagata acagtatgcg tatttgcgcg ctgatttttg 4740
   cggtataaga atatatactg atatgtatac ccgaagtatg tcaaaaagag gtgtgcttct 4800
   agaatgcagt ttaaggttta cacctataaa agagagagcc gttatcgtct gtttgtggat 4860
   gtacagagtg atattattga cacgcccggg cgacggatgg tgatccccct ggccagtgca 4920
   cgtctgctgt cagataaagt ctcccgtgaa ctttacccgg tggtgcatat cggggatgaa 4980
   agctggcgca tgatgaccac cgatatggcc agtgtgccgg tctccgttat cggggaagaa 5040
   gtggctgatc tcagccaccg cgaaaatgac atcaaaaacg ccattaacct gatgttctgg 5100
   ggaatataga attcctcagg tcgaccatag tgactggata tgttgtgttt tacagtatta 5160
   tgtagtctgt tttttatgca aaatctaatt taatatattg atatttatat cattttacgt 5220
   ttctcgttca gctttcttgt acaaagtggt ctcgagttaa ttaattgatc cgggttatta 5280
   gtacatttat taagcgctag attctgtgcg ttgttgattt acagacaatt gttgtacgta 5340
   ttttaataat tcattaaatt tataatcttt agggtggtat gttagagcga aaatcaaatg 5400
   attttcagcg tctttatatc tgaatttaaa tattaaatcc tcaatagatt tgtaaaatag 5460
   gtttcgatta gtttcaaaca agggttgttt ttccgaaccg atggctggac tatctaatgg 5520
   attttcgctc aacgccacaa aacttgccaa atcttgtagc agcaatctag ctttgtcgat 5580
   attcgtttgt gttttgtttt gtaataaagg ttcgacgtcg ttcaaaatat tatgcgcttt 5640
   tgtatttctt tcatcactgt cgttagtgta caattgactc gacgtaaaca cgttaaataa 5700
   agcttggaca tatttaacat cgggcgtgtt agctttatta ggccgattat cgtcgtcgtc 5760
   ccaaccctcg tcgttagaag ttgcttccga agacgatttt gccatagcca cacgacgcct 5820
   attaattgtg tcggctaaca cgtccgcgat caaatttgta gttgagcttt ttggaattat 5880
   ttctgattgc gggcgttttt gggcgggttt caatctaact gtgcccgatt ttaattcaga 5940
   caacacgtta gaaagcgatg gtgcaggcgg tggtaacatt tcagacggca aatctactaa 6000
   tggcggcggt ggtggagctg atgataaatc taccatcggt ggaggcgcag gcggggctgg 6060
   cggcggaggc ggaggcggag gtggtggcgg tgatgcagac ggcggtttag gctcaaatgt 6120
   ctctttaggc aacacagtcg gcacctcaac tattgtactg gtttcgggcg ccgtttttgg 6180
   tttgaccggt ctgagacgag tgcgattttt ttcgtttcta atagcttcca acaattgttg 6240
   tctgtcgtct aaaggtgcag cgggttgagg ttccgtcggc attggtggag cgggcggcaa 6300
   ttcagacatc gatggtggtg gtggtggtgg aggcgctgga atgttaggca cgggagaagg 6360
   tggtggcggc ggtgccgccg gtataatttg ttctggttta gtttgttcgc gcacgattgt 6420
   gggcaccggc gcaggcgccg ctggctgcac aacggaaggt cgtctgcttc gaggcagcgc 6480
   ttggggtggt ggcaattcaa tattataatt ggaatacaaa tcgtaaaaat ctgctataag 6540
   cattgtaatt tcgctatcgt ttaccgtgcc gatatttaac aaccgctcaa tgtaagcaat 6600
   tgtattgtaa agagattgtc tcaagctcgg atcccgcacg ccgataacaa gccttttcat 6660
   ttttactaca gcattgtagt ggcgagacac ttcgctgtcg tcgacgtaca tgtatgcttt 6720
   gttgtcaaaa acgtcgttgg caagctttaa aatatttaaa agaacatctc tgttcagcac 6780
   cactgtgttg tcgtaaatgt tgtttttgat aatttgcgct tccgcagtat cgacacgttc 6840
   aaaaaattga tgcgcatcaa ttttgttgtt cctattattg aataaataag attgtacaga 6900
   ttcatatcta cgattcgtca tggccaccac aaatgctacg ctgcaaacgc tggtacaatt 6960
   ttacgaaaac tgcaaaaacg tcaaaactcg gtataaaata atcaacgggc gctttggcaa 7020
   aatatctatt ttatcgcaca agcccactag caaattgtat ttgcagaaaa caatttcggc 7080
   gcacaatttt aacgctgacg aaataaaagt tcaccagtta atgagcgacc acccaaattt 7140
   tataaaaatc tattttaatc acggttccat caacaaccaa gtgatcgtga tggactacat 7200
   tgactgtccc gatttatttg aaacactaca aattaaaggc gagctttcgt accaacttgt 7260
   tagcaatatt attagacagc tgtgtgaagc gctcaacgat ttgcacaagc acaatttcat 7320
   acacaacgac ataaaactcg aaaatgtctt atatttcgaa gcacttgatc gcgtgtatgt 7380
   ttgcgattac ggattgtgca aacacgaaaa ctcacttagc gtgcacgacg gcacgttgga 7440
   gtattttagt ccggaaaaaa ttcgacacac aactatgcac gtttcgtttg actggtacgc 7500
   cgtcggcgtg ttaacataca agttgctaac cggcggccga cacccatttg aaaaaagcga 7560
   agacgaaatg ttggacttga atagcatgaa gcgtcgtcag caatacaatg acattggcgt 7620
   tttaaaacac gttcgtaacg ttaacgctcg tgactttgtg tactgcctaa caagatacaa 7680
   catagattgt agactcacaa attacaaaca aattataaaa catgagtttt tgtcgtaaaa 7740
   atgccacttg ttttacgagt agaattctac gtgtaacaca cgatctaaaa gatgatgtca 7800
   ttttttatca atgactcatt tgttttaaaa cagacttgtt ttacgagtag aattctacgt 7860
   gtaaagcatg atcgtgagtg gtgttaataa aatcataaaa attattgtaa atgtttatta 7920
   tttaaaaacg attcaaatat ataataaaaa caatctacat ctatttcttc acaatccata 7980
   acacacaaca ggtccatcaa tgagtttttg tctttatccg acatactatg tgcatgtaac 8040
   aaatcaaata catcttttaa atttttatac acatctttac attgtctacc aaaatcttta 8100
   ataaccctat aacaaggaaa agacttttct tcttgcgtgg ttttgccgcg cagatattga 8160
   aataaaatgt gcatgcacga caacttgtgt ttactaaaat gctccttgcc tataccgcaa 8220
   aaccggccat acatttcggc gattacacgc ggacaattgt acgattcgtc tacgtgtaaa 8280
   cgatcatcat aatcactctt gcgcaaacga ataaattttt tcaccgcttc cgacaaacga 8340
   ggcaccaatt cggcgggcac gcttcgatac attattctgt gcacataagt taccacacaa 8400
   aatttattgt accaccatcc gacaacgtcg ttattagggt tgaacacgtt ggcgatgcgc 8460
   agcagtttcc cgtttctcat gaaatattca aagcggccca aaataatttg caagcaatcc 8520
   aacatgtctt gagaaatttc tcgttcaaaa ttgttcaaag agaatatctg ccatccgttt 8580
   tgaacgcgca cgctgacggg aaccaccgca tcgatttgct ccaacacttc acggacgtta 8640
   tcgtcgatgc ccatcgtttc gctggtgctg aaccaatggg aaaggctctt gatggaatcg 8700
   cccgcgtcta tcatcttgac cgcttcgtca aaggtgcaac tgccgctctt caaacgccgc 8760
   atagcggtca cgtcccgctc tatgcacgac ataccgttta cgtacgattc tgataggtat 8820
   tcctgaacta tacggtaatg gtgatacgac tcgccataca cgtcgtgcac ctcattgtat 8880
   ttagcataat aattgtaaat tattaacttt gcagcgagag acatgttgtc agtaaagcgg 8940
   tgctaggctc aataatactg atgtacaggc acgcgtgcta tttatatata atttcgcaag 9000
   gaggggagct gttatcggtt gctattatta aagaatggcc gtctgttttt atcacaagct 9060
   tggcagcctc aaccatgaag cgtcgtcatt gtaaattaaa ttctctgcct caagaattat 9120
   ttgacaagat tgtcgagtat ttatctttat ctgattactg caatttggtg cttgtctgta 9180
   aaagaccttc tagtaaatat aacgtgatat ttgatagtac taatcaccaa catttgaaag 9240
   gcgtgtacaa aaagacagac gtgcaaataa caagctacaa cgaatacatc aactgtattt 9300
   gcaacgaact gagacaagac gaattctatg ccaaatcatc atggattgcg agtatttgcg 9360
   gtcaccagag agcgacaatt tttagtgtaa caaataaaca agtagaaatg aaatatcatt 9420
   tgtataatat agcaattgtg gaaagtgaag attgcaacgg attttaccca tttgagccaa 9480
   cgcgcgattg tttaatatgc aaacaaaaaa accaatgtcc tcgtaattca tttattgttt 9540
   cgttgtgtaa atatttagaa aaacaaaatg tacaatcaaa ctttatatat tatttatacg 9600
   aaataaatac ataataataa ctattataca tgtttttatt ttacaatact tcctgtataa 9660
   cctctctaac tacattagga gtacaatcca cgtcaattac acgtttagct atttttctaa 9720
   ttttgtaatg tttatcgtag agtttttcgt taatacattg aatagccaac aagggatttg 9780
   ggtgcacacc gtcatagagt acttccatgt cgtcttcaaa gcgcattttt cgcttgcgaa 9840
   aatgccgctc ttggcccaaa acaaaagcga gtttgatgcg gtcgtcgatg cgttccgaaa 9900
   atacggccaa atgctggtgt ttggtgatgt cgcgcggaaa cgtcaccgtg ccatttttgc 9960
   tttccgccac gacggcggtt ttcaattttt cggccgactg 10000
<210> 2
   <211> 10000
   <212> DNA
   <213> Artificial Sequence
<220>
   <223> Polyhedrin locus of vBacHTS_His
<220>
   <221> source
   <222> (4523)..(4553)
   <223> F 6His
<220>
   <221> misc_feature
   <222> (4831)..(5136)
   <223> F ccdB
<220>
   <221> promoter
   <222> (4428)..(4520)
   <223> F pPolyhedrin
<220>
   <221> misc_feature
   <222> (5153)..(5277)
   <223> R attR2
<220>
   <221> misc_feature
   <222> (4564)..(4688)
   <223> F attR1
<400> 2
   gaattctacc cgtaaagcga gtttagtttt gaaaaacaaa tgacatcatt tgtataatga 60
   catcatcccc tgattgtgtt ttacaagtag aattctatcc gtaaagcgag ttcagttttg 120
   aaaacaaatg agtcatacct aaacacgtta ataatcttct gatatcagct tatgactcaa 180
   gttatgagcc gtgtgcaaaa catgagataa gtttatgaca tcatccactg atcgtgcgtt 240
   acaagtagaa ttctactcgt aaagccagtt cggttatgag ccgtgtgcaa aacatgacat 300
   cagcttatga ctcatacttg attgtgtttt acgcgtagaa ttctactcgt aaagcgagtt 360
   cggttatgag ccgtgtgcaa aacatgacat cagcttatga gtcataatta atcgtgcgtt 420
   acaagtagaa ttctactcgt aaagcgagtt gaaggatcat atttagttgc gtttatgaga 480
   taagattgaa agcacgtgta aaatgtttcc cgcgcgttgg cacaactatt tacaatgcgg 540
   ccaagttata aaagattcta atctgatatg ttttaaaaca cctttgcggc ccgagttgtt 600
   tgcgtacgtg actagcgaag aagatgtgtg gaccgcagaa cagatagtaa aacaaaaccc 660
   tagtattgga gcaataatcg atttaaccaa cacgtctaaa tattatgatg gtgtgcattt 720
   tttgcgggcg ggcctgttat acaaaaaaat tcaagtacct ggccagactt tgccgcctga 780
   aagcatagtt caagaattta ttgacacggt aaaagaattt acagaaaagt gtcccggcat 840
   gttggtgggc gtgcactgca cacacggtat taatcgcacc ggttacatgg tgtgcagata 900
   tttaatgcac accctgggta ttgcgccgca ggaagccata gatagattcg aaaaagccag 960
   aggtcacaaa attgaaagac aaaattacgt tcaagattta ttaatttaat taatattatt 1020
   tgcattcttt aacaaatact ttatcctatt ttcaaattgt tgcgcttctt ccagcgaacc 1080
   aaaactatgc ttcgcttgct ccgtttagct tgtagccgat cagtggcgtt gttccaatcg 1140
   acggtaggat taggccggat attctccacc acaatgttgg caacgttgat gttacgttta 1200
   tgcttttggt tttccacgta cgtcttttgg ccggtaatag ccgtaaacgt agtgccgtcg 1260
   cgcgtcacgc acaacaccgg atgtttgcgc ttgtccgcgg ggtattgaac cgcgcgatcc 1320
   gacaaatcca ccactttggc aactaaatcg gtgacctgcg cgtctttttt ctgcattatt 1380
   tcgtctttct tttgcatggt ttcctggaag ccggtgtaca tgcggtttag atcagtcatg 1440
   acgcgcgtga cctgcaaatc tttggcctcg atctgcttgt ccttgatggc aacgatgcgt 1500
   tcaataaact cttgtttttt aacaagttcc tcggtttttt gcgccaccac cgcttgcagc 1560
   gcgtttgtgt gctcggtgaa tgtcgcaatc agcttagtca ccaactgttt gctctcctcc 1620
   tcccgttgtt tgatcgcggg atcgtacttg ccggtgcaga gcacttgagg aattacttct 1680
   tctaaaagcc attcttgtaa ttctatggcg taaggcaatt tggacttcat aatcagctga 1740
   atcacgccgg atttagtaat gagcactgta tgcggctgca aatacagcgg gtcgcccctt 1800
   ttcacgacgc tgttagaggt agggccccca ttttggatgg tctgctcaaa taacgatttg 1860
   tatttattgt ctacatgaac acgtatagct ttatcacaaa ctgtatattt taaactgtta 1920
   gcgacgtcct tggccacgaa ccggacctgt tggtcgcgct ctagcacgta ccgcaggttg 1980
   aacgtatctt ctccaaattt aaattctcca attttaacgc gagccatttt gatacacgtg 2040
   tgtcgatttt gcaacaacta ttgtttttta acgcaaacta aacttattgt ggtaagcaat 2100
   aattaaatat gggggaacat gcgccgctac aacactcgtc gttatgaacg cagacggcgc 2160
   cggtctcggc gcaagcggct aaaacgtgtt gcgcgttcaa cgcggcaaac atcgcaaaag 2220
   ccaatagtac agttttgatt tgcatattaa cggcgatttt ttaaattatc ttatttaata 2280
   aatagttatg acgcctacaa ctccccgccc gcgttgactc gctgcacctc gagcagttcg 2340
   ttgacgcctt cctccgtgtg gccgaacacg tcgagcgggt ggtcgatgac cagcggcgtg 2400
   ccgcacgcga cgcacaagta tctgtacacc gaatgatcgt cgggcgaagg cacgtcggcc 2460
   tccaagtggc aatattggca aattcgaaaa tatatacagt tgggttgttt gcgcatatct 2520
   atcgtggcgt tgggcatgta cgtccgaacg ttgatttgca tgcaagccga aattaaatca 2580
   ttgcgattag tgcgattaaa acgttgtaca tcctcgcttt taatcatgcc gtcgattaaa 2640
   tcgcgcaatc gagtcaagtg atcaaagtgt ggaataatgt tttctttgta ttcccgagtc 2700
   aagcgcagcg cgtattttaa caaactagcc atcttgtaag ttagtttcat ttaatgcaac 2760
   tttatccaat aatatattat gtatcgcacg tcaagaatta acaatgcgcc cgttgtcgca 2820
   tctcaacacg actatgatag agatcaaata aagcgcgaat taaatagctt gcgacgcaac 2880
   gtgcacgatc tgtgcacgcg ttccggcacg agctttgatt gtaataagtt tttacgaagc 2940
   gatgacatga cccccgtagt gacaacgatc acgcccaaaa gaactgccga ctacaaaatt 3000
   accgagtatg tcggtgacgt taaaactatt aagccatcca atcgaccgtt agtcgaatca 3060
   ggaccgctgg tgcgagaagc cgcgaagtat ggcgaatgca tcgtataacg tgtggagtcc 3120
   gctcattaga gcgtcatgtt tagacaagaa agctacatat ttaattgatc ccgatgattt 3180
   tattgataaa ttgaccctaa ctccatacac ggtattctac aatggcgggg ttttggtcaa 3240
   aatttccgga ctgcgattgt acatgctgtt aacggctccg cccactatta atgaaattaa 3300
   aaattccaat tttaaaaaac gcagcaagag aaacatttgt atgaaagaat gcgtagaagg 3360
   aaagaaaaat gtcgtcgaca tgctgaacaa caagattaat atgcctccgt gtataaaaaa 3420
   aatattgaac gatttgaaag aaaacaatgt accgcgcggc ggtatgtaca ggaagaggtt 3480
   tatactaaac tgttacattg caaacgtggt ttcgtgtgcc aagtgtgaaa accgatgttt 3540
   aatcaaggct ctgacgcatt tctacaacca cgactccaag tgtgtgggtg aagtcatgca 3600
   tcttttaatc aaatcccaag atgtgtataa accaccaaac tgccaaaaaa tgaaaactgt 3660
   cgacaagctc tgtccgtttg ctggcaactg caagggtctc aatcctattt gtaattattg 3720
   aataataaaa caattataaa tgctaaattt gttttttatt aacgatacaa accaaacgca 3780
   acaagaacat ttgtagtatt atctataatt gaaaacgcgt agttataatc gctgaggtaa 3840
   tatttaaaat cattttcaaa tgattcacag ttaatttgcg acaatataat tttattttca 3900
   cataaactag acgccttgtc gtcttcttct tcgtattcct tctctttttc atttttctcc 3960
   tcataaaaat taacatagtt attatcgtat ccatatatgt atctatcgta tagagtaaat 4020
   tttttgttgt cataaatata tatgtctttt ttaatggggt gtatagtacc gctgcgcata 4080
   gtttttctgt aatttacaac agtgctattt tctggtagtt cttcggagtg tgttgcttta 4140
   attattaaat ttatataatc aatgaatttg ggatcgtcgg ttttgtacaa tatgttgccg 4200
   gcatagtacg cagcttcttc tagttcaatt acaccatttt ttagcagcac cggattaaca 4260
   taactttcca aaatgttgta cgaaccgtta aacaaaaaca gttcacctcc cttttctata 4320
   ctattgtctg cgagcagttg tttgttgtta aaaataacag ccattgtaat gagacgcaca 4380
   aactaatatc acaaactgga aatgtctatc aatatatagt tgctgatatc atggagataa 4440
   ttaaaatgat aaccatctcg caaataaata agtattttac tgttttcgta acagttttgt 4500
   aataaaaaaa cctataaata cggatctatg catcatcacc atcatcacgg atcccggggt 4560
   accacaagtt tgtacaaaaa agctgaacga gaaacgtaaa atgatataaa tatcaatata 4620
   ttaaattaga ttttgcataa aaaacagact acataatact gtaaaacaca acatatccag 4680
   tcactatggc ggccgcatta ggcacccctt aggaattcgc ttactaaaag ccagataaca 4740
   gtatgcgtat ttgcgcgctg atttttgcgg tataagaata tatactgata tgtatacccg 4800
   aagtatgtca aaaagaggtg tgcttctaga atgcagttta aggtttacac ctataaaaga 4860
   gagagccgtt atcgtctgtt tgtggatgta cagagtgata ttattgacac gcccgggcga 4920
   cggatggtga tccccctggc cagtgcacgt ctgctgtcag ataaagtctc ccgtgaactt 4980
   tacccggtgg tgcatatcgg ggatgaaagc tggcgcatga tgaccaccga tatggccagt 5040
   gtgccggtct ccgttatcgg ggaagaagtg gctgatctca gccaccgcga aaatgacatc 5100
   aaaaacgcca ttaacctgat gttctgggga atatagaatt cctcaggtcg accatagtga 5160
   ctggatatgt tgtgttttac agtattatgt agtctgtttt ttatgcaaaa tctaatttaa 5220
   tatattgata tttatatcat tttacgtttc tcgttcagct ttcttgtaca aagtggtctc 5280
   gagttaatta attgatccgg gttattagta catttattaa gcgctagatt ctgtgcgttg 5340
   ttgatttaca gacaattgtt gtacgtattt taataattca ttaaatttat aatctttagg 5400
   gtggtatgtt agagcgaaaa tcaaatgatt ttcagcgtct ttatatctga atttaaatat 5460
   taaatcctca atagatttgt aaaataggtt tcgattagtt tcaaacaagg gttgtttttc 5520
   cgaaccgatg gctggactat ctaatggatt ttcgctcaac gccacaaaac ttgccaaatc 5580
   ttgtagcagc aatctagctt tgtcgatatt cgtttgtgtt ttgttttgta ataaaggttc 5640
   gacgtcgttc aaaatattat gcgcttttgt atttctttca tcactgtcgt tagtgtacaa 5700
   ttgactcgac gtaaacacgt taaataaagc ttggacatat ttaacatcgg gcgtgttagc 5760
   tttattaggc cgattatcgt cgtcgtccca accctcgtcg ttagaagttg cttccgaaga 5820
   cgattttgcc atagccacac gacgcctatt aattgtgtcg gctaacacgt ccgcgatcaa 5880
   atttgtagtt gagctttttg gaattatttc tgattgcggg cgtttttggg cgggtttcaa 5940
   tctaactgtg cccgatttta attcagacaa cacgttagaa agcgatggtg caggcggtgg 6000
   taacatttca gacggcaaat ctactaatgg cggcggtggt ggagctgatg ataaatctac 6060
   catcggtgga ggcgcaggcg gggctggcgg cggaggcgga ggcggaggtg gtggcggtga 6120
   tgcagacggc ggtttaggct caaatgtctc tttaggcaac acagtcggca cctcaactat 6180
   tgtactggtt tcgggcgccg tttttggttt gaccggtctg agacgagtgc gatttttttc 6240
   gtttctaata gcttccaaca attgttgtct gtcgtctaaa ggtgcagcgg gttgaggttc 6300
   cgtcggcatt ggtggagcgg gcggcaattc agacatcgat ggtggtggtg gtggtggagg 6360
   cgctggaatg ttaggcacgg gagaaggtgg tggcggcggt gccgccggta taatttgttc 6420
   tggtttagtt tgttcgcgca cgattgtggg caccggcgca ggcgccgctg gctgcacaac 6480
   ggaaggtcgt ctgcttcgag gcagcgcttg gggtggtggc aattcaatat tataattgga 6540
   atacaaatcg taaaaatctg ctataagcat tgtaatttcg ctatcgttta ccgtgccgat 6600
   atttaacaac cgctcaatgt aagcaattgt attgtaaaga gattgtctca agctcggatc 6660
   ccgcacgccg ataacaagcc ttttcatttt tactacagca ttgtagtggc gagacacttc 6720
   gctgtcgtcg acgtacatgt atgctttgtt gtcaaaaacg tcgttggcaa gctttaaaat 6780
   atttaaaaga acatctctgt tcagcaccac tgtgttgtcg taaatgttgt ttttgataat 6840
   ttgcgcttcc gcagtatcga cacgttcaaa aaattgatgc gcatcaattt tgttgttcct 6900
   attattgaat aaataagatt gtacagattc atatctacga ttcgtcatgg ccaccacaaa 6960
   tgctacgctg caaacgctgg tacaatttta cgaaaactgc aaaaacgtca aaactcggta 7020
   taaaataatc aacgggcgct ttggcaaaat atctatttta tcgcacaagc ccactagcaa 7080
   attgtatttg cagaaaacaa tttcggcgca caattttaac gctgacgaaa taaaagttca 7140
   ccagttaatg agcgaccacc caaattttat aaaaatctat tttaatcacg gttccatcaa 7200
   caaccaagtg atcgtgatgg actacattga ctgtcccgat ttatttgaaa cactacaaat 7260
   taaaggcgag ctttcgtacc aacttgttag caatattatt agacagctgt gtgaagcgct 7320
   caacgatttg cacaagcaca atttcataca caacgacata aaactcgaaa atgtcttata 7380
   tttcgaagca cttgatcgcg tgtatgtttg cgattacgga ttgtgcaaac acgaaaactc 7440
   acttagcgtg cacgacggca cgttggagta ttttagtccg gaaaaaattc gacacacaac 7500
   tatgcacgtt tcgtttgact ggtacgccgt cggcgtgtta acatacaagt tgctaaccgg 7560
   cggccgacac ccatttgaaa aaagcgaaga cgaaatgttg gacttgaata gcatgaagcg 7620
   tcgtcagcaa tacaatgaca ttggcgtttt aaaacacgtt cgtaacgtta acgctcgtga 7680
   ctttgtgtac tgcctaacaa gatacaacat agattgtaga ctcacaaatt acaaacaaat 7740
   tataaaacat gagtttttgt cgtaaaaatg ccacttgttt tacgagtaga attctacgtg 7800
   taacacacga tctaaaagat gatgtcattt tttatcaatg actcatttgt tttaaaacag 7860
   acttgtttta cgagtagaat tctacgtgta aagcatgatc gtgagtggtg ttaataaaat 7920
   cataaaaatt attgtaaatg tttattattt aaaaacgatt caaatatata ataaaaacaa 7980
   tctacatcta tttcttcaca atccataaca cacaacaggt ccatcaatga gtttttgtct 8040
   ttatccgaca tactatgtgc atgtaacaaa tcaaatacat cttttaaatt tttatacaca 8100
   tctttacatt gtctaccaaa atctttaata accctataac aaggaaaaga cttttcttct 8160
   tgcgtggttt tgccgcgcag atattgaaat aaaatgtgca tgcacgacaa cttgtgttta 8220
   ctaaaatgct ccttgcctat accgcaaaac cggccataca tttcggcgat tacacgcgga 8280
   caattgtacg attcgtctac gtgtaaacga tcatcataat cactcttgcg caaacgaata 8340
   aattttttca ccgcttccga caaacgaggc accaattcgg cgggcacgct tcgatacatt 8400
   attctgtgca cataagttac cacacaaaat ttattgtacc accatccgac aacgtcgtta 8460
   ttagggttga acacgttggc gatgcgcagc agtttcccgt ttctcatgaa atattcaaag 8520
   cggcccaaaa taatttgcaa gcaatccaac atgtcttgag aaatttctcg ttcaaaattg 8580
   ttcaaagaga atatctgcca tccgttttga acgcgcacgc tgacgggaac caccgcatcg 8640
   atttgctcca acacttcacg gacgttatcg tcgatgccca tcgtttcgct ggtgctgaac 8700
   caatgggaaa ggctcttgat ggaatcgccc gcgtctatca tcttgaccgc ttcgtcaaag 8760
   gtgcaactgc cgctcttcaa acgccgcata gcggtcacgt cccgctctat gcacgacata 8820
   ccgtttacgt acgattctga taggtattcc tgaactatac ggtaatggtg atacgactcg 8880
   ccatacacgt cgtgcacctc attgtattta gcataataat tgtaaattat taactttgca 8940
   gcgagagaca tgttgtcagt aaagcggtgc taggctcaat aatactgatg tacaggcacg 9000
   cgtgctattt atatataatt tcgcaaggag gggagctgtt atcggttgct attattaaag 9060
   aatggccgtc tgtttttatc acaagcttgg cagcctcaac catgaagcgt cgtcattgta 9120
   aattaaattc tctgcctcaa gaattatttg acaagattgt cgagtattta tctttatctg 9180
   attactgcaa tttggtgctt gtctgtaaaa gaccttctag taaatataac gtgatatttg 9240
   atagtactaa tcaccaacat ttgaaaggcg tgtacaaaaa gacagacgtg caaataacaa 9300
   gctacaacga atacatcaac tgtatttgca acgaactgag acaagacgaa ttctatgcca 9360
   aatcatcatg gattgcgagt atttgcggtc accagagagc gacaattttt agtgtaacaa 9420
   ataaacaagt agaaatgaaa tatcatttgt ataatatagc aattgtggaa agtgaagatt 9480
   gcaacggatt ttacccattt gagccaacgc gcgattgttt aatatgcaaa caaaaaaacc 9540
   aatgtcctcg taattcattt attgtttcgt tgtgtaaata tttagaaaaa caaaatgtac 9600
   aatcaaactt tatatattat ttatacgaaa taaatacata ataataacta ttatacatgt 9660
   ttttatttta caatacttcc tgtataacct ctctaactac attaggagta caatccacgt 9720
   caattacacg tttagctatt tttctaattt tgtaatgttt atcgtagagt ttttcgttaa 9780
   tacattgaat agccaacaag ggatttgggt gcacaccgtc atagagtact tccatgtcgt 9840
   cttcaaagcg catttttcgc ttgcgaaaat gccgctcttg gcccaaaaca aaagcgagtt 9900
   tgatgcggtc gtcgatgcgt tccgaaaata cggccaaatg ctggtgtttg gtgatgtcgc 9960
   gcggaaacgt caccgtgcca tttttgcttt ccgccacgac 10000
<210> 3
   <211> 10000
   <212> DNA
   <213> Artificial Sequence
<220>
   <223> Polyhedrin locus of vBacHTS_HisGst
<220>
   <221> source
   <222> (4523)..(4553)
   <223> F 6His
<220>
   <221> source
   <222> (4523)..(5231)
   <223> F HGst
<220>
   <221> promoter
   <222> (4428)..(4520)
   <223> F pPolyhedrin
<220>
   <221> misc_feature
   <222> (5831)..(5955)
   <223> R attR2
<220>
   <221> misc_feature
   <222> (5242)..(5366)
   <223> F attR1
<400> 3
   gaattctacc cgtaaagcga gtttagtttt gaaaaacaaa tgacatcatt tgtataatga      60
   catcatcccc tgattgtgtt ttacaagtag aattctatcc gtaaagcgag ttcagttttg     120
   aaaacaaatg agtcatacct aaacacgtta ataatcttct gatatcagct tatgactcaa     180
   gttatgagcc gtgtgcaaaa catgagataa gtttatgaca tcatccactg atcgtgcgtt     240
   acaagtagaa ttctactcgt aaagccagtt cggttatgag ccgtgtgcaa aacatgacat     300
   cagcttatga ctcatacttg attgtgtttt acgcgtagaa ttctactcgt aaagcgagtt     360
   cggttatgag ccgtgtgcaa aacatgacat cagcttatga gtcataatta atcgtgcgtt     420
   acaagtagaa ttctactcgt aaagcgagtt gaaggatcat atttagttgc gtttatgaga     480
   taagattgaa agcacgtgta aaatgtttcc cgcgcgttgg cacaactatt tacaatgcgg     540
   ccaagttata aaagattcta atctgatatg ttttaaaaca cctttgcggc ccgagttgtt     600
   tgcgtacgtg actagcgaag aagatgtgtg gaccgcagaa cagatagtaa aacaaaaccc     660
   tagtattgga gcaataatcg atttaaccaa cacgtctaaa tattatgatg gtgtgcattt     720
   tttgcgggcg ggcctgttat acaaaaaaat tcaagtacct ggccagactt tgccgcctga     780
   aagcatagtt caagaattta ttgacacggt aaaagaattt acagaaaagt gtcccggcat     840
   gttggtgggc gtgcactgca cacacggtat taatcgcacc ggttacatgg tgtgcagata     900
   tttaatgcac accctgggta ttgcgccgca ggaagccata gatagattcg aaaaagccag 960
   aggtcacaaa attgaaagac aaaattacgt tcaagattta ttaatttaat taatattatt 1020
   tgcattcttt aacaaatact ttatcctatt ttcaaattgt tgcgcttctt ccagcgaacc 1080
   aaaactatgc ttcgcttgct ccgtttagct tgtagccgat cagtggcgtt gttccaatcg 1140
   acggtaggat taggccggat attctccacc acaatgttgg caacgttgat gttacgttta 1200
   tgcttttggt tttccacgta cgtcttttgg ccggtaatag ccgtaaacgt agtgccgtcg 1260
   cgcgtcacgc acaacaccgg atgtttgcgc ttgtccgcgg ggtattgaac cgcgcgatcc 1320
   gacaaatcca ccactttggc aactaaatcg gtgacctgcg cgtctttttt ctgcattatt 1380
   tcgtctttct tttgcatggt ttcctggaag ccggtgtaca tgcggtttag atcagtcatg 1440
   acgcgcgtga cctgcaaatc tttggcctcg atctgcttgt ccttgatggc aacgatgcgt 1500
   tcaataaact cttgtttttt aacaagttcc tcggtttttt gcgccaccac cgcttgcagc 1560
   gcgtttgtgt gctcggtgaa tgtcgcaatc agcttagtca ccaactgttt gctctcctcc 1620
   tcccgttgtt tgatcgcggg atcgtacttg ccggtgcaga gcacttgagg aattacttct 1680
   tctaaaagcc attcttgtaa ttctatggcg taaggcaatt tggacttcat aatcagctga 1740
   atcacgccgg atttagtaat gagcactgta tgcggctgca aatacagcgg gtcgcccctt 1800
   ttcacgacgc tgttagaggt agggccccca ttttggatgg tctgctcaaa taacgatttg 1860
   tatttattgt ctacatgaac acgtatagct ttatcacaaa ctgtatattt taaactgtta 1920
   gcgacgtcct tggccacgaa ccggacctgt tggtcgcgct ctagcacgta ccgcaggttg 1980
   aacgtatctt ctccaaattt aaattctcca attttaacgc gagccatttt gatacacgtg 2040
   tgtcgatttt gcaacaacta ttgtttttta acgcaaacta aacttattgt ggtaagcaat 2100
   aattaaatat gggggaacat gcgccgctac aacactcgtc gttatgaacg cagacggcgc 2160
   cggtctcggc gcaagcggct aaaacgtgtt gcgcgttcaa cgcggcaaac atcgcaaaag 2220
   ccaatagtac agttttgatt tgcatattaa cggcgatttt ttaaattatc ttatttaata 2280
   aatagttatg acgcctacaa ctccccgccc gcgttgactc gctgcacctc gagcagttcg 2340
   ttgacgcctt cctccgtgtg gccgaacacg tcgagcgggt ggtcgatgac cagcggcgtg 2400
   ccgcacgcga cgcacaagta tctgtacacc gaatgatcgt cgggcgaagg cacgtcggcc 2460
   tccaagtggc aatattggca aattcgaaaa tatatacagt tgggttgttt gcgcatatct 2520
   atcgtggcgt tgggcatgta cgtccgaacg ttgatttgca tgcaagccga aattaaatca 2580
   ttgcgattag tgcgattaaa acgttgtaca tcctcgcttt taatcatgcc gtcgattaaa 2640
   tcgcgcaatc gagtcaagtg atcaaagtgt ggaataatgt tttctttgta ttcccgagtc 2700
   aagcgcagcg cgtattttaa caaactagcc atcttgtaag ttagtttcat ttaatgcaac 2760
   tttatccaat aatatattat gtatcgcacg tcaagaatta acaatgcgcc cgttgtcgca 2820
   tctcaacacg actatgatag agatcaaata aagcgcgaat taaatagctt gcgacgcaac 2880
   gtgcacgatc tgtgcacgcg ttccggcacg agctttgatt gtaataagtt tttacgaagc 2940
   gatgacatga cccccgtagt gacaacgatc acgcccaaaa gaactgccga ctacaaaatt 3000
   accgagtatg tcggtgacgt taaaactatt aagccatcca atcgaccgtt agtcgaatca 3060
   ggaccgctgg tgcgagaagc cgcgaagtat ggcgaatgca tcgtataacg tgtggagtcc 3120
   gctcattaga gcgtcatgtt tagacaagaa agctacatat ttaattgatc ccgatgattt 3180
   tattgataaa ttgaccctaa ctccatacac ggtattctac aatggcgggg ttttggtcaa 3240
   aatttccgga ctgcgattgt acatgctgtt aacggctccg cccactatta atgaaattaa 3300
   aaattccaat tttaaaaaac gcagcaagag aaacatttgt atgaaagaat gcgtagaagg 3360
   aaagaaaaat gtcgtcgaca tgctgaacaa caagattaat atgcctccgt gtataaaaaa 3420
   aatattgaac gatttgaaag aaaacaatgt accgcgcggc ggtatgtaca ggaagaggtt 3480
   tatactaaac tgttacattg caaacgtggt ttcgtgtgcc aagtgtgaaa accgatgttt 3540
   aatcaaggct ctgacgcatt tctacaacca cgactccaag tgtgtgggtg aagtcatgca 3600
   tcttttaatc aaatcccaag atgtgtataa accaccaaac tgccaaaaaa tgaaaactgt 3660
   cgacaagctc tgtccgtttg ctggcaactg caagggtctc aatcctattt gtaattattg 3720
   aataataaaa caattataaa tgctaaattt gttttttatt aacgatacaa accaaacgca 3780
   acaagaacat ttgtagtatt atctataatt gaaaacgcgt agttataatc gctgaggtaa 3840
   tatttaaaat cattttcaaa tgattcacag ttaatttgcg acaatataat tttattttca 3900
   cataaactag acgccttgtc gtcttcttct tcgtattcct tctctttttc atttttctcc 3960
   tcataaaaat taacatagtt attatcgtat ccatatatgt atctatcgta tagagtaaat 4020
   tttttgttgt cataaatata tatgtctttt ttaatggggt gtatagtacc gctgcgcata 4080
   gtttttctgt aatttacaac agtgctattt tctggtagtt cttcggagtg tgttgcttta 4140
   attattaaat ttatataatc aatgaatttg ggatcgtcgg ttttgtacaa tatgttgccg 4200
   gcatagtacg cagcttcttc tagttcaatt acaccatttt ttagcagcac cggattaaca 4260
   taactttcca aaatgttgta cgaaccgtta aacaaaaaca gttcacctcc cttttctata 4320
   ctattgtctg cgagcagttg tttgttgtta aaaataacag ccattgtaat gagacgcaca 4380
   aactaatatc acaaactgga aatgtctatc aatatatagt tgctgatatc atggagataa 4440
   ttaaaatgat aaccatctcg caaataaata agtattttac tgttttcgta acagttttgt 4500
   aataaaaaaa cctataaata cggatctatg catcatcacc atcatcacgg atctatgtcc 4560
   cctatactag gttattggaa aattaagggc cttgtgcaac ccactcgact tcttttggaa 4620
   tatcttgaag aaaaatatga agagcatttg tatgagcgcg atgaaggtga taaatggcga 4680
   aacaaaaagt ttgaattggg tttggagttt cccaatcttc cttattatat tgatggtgat 4740
   gttaaattaa cacagtctat ggccatcata cgttatatag ctgacaagca caacatgttg 4800
   ggtggttgtc caaaagagcg tgcagagatt tcaatgcttg aaggagcggt tttggatatt 4860
   agatacggtg tttcgagaat tgcatatagt aaagactttg aaactctcaa agttgatttt 4920
   cttagcaagc tacctgaaat gctgaaaatg ttcgaagatc gtttatgtca taaaacatat 4980
   ttaaatggtg atcatgtaac ccatcctgac ttcatgttgt atgacgctct tgatgttgtt 5040
   ttatacatgg acccaatgtg cctggatgcg ttcccaaaat tagtttgttt taaaaaacgt 5100
   attgaagcta tcccacaaat tgataagtac ttgaaatcca gcaagtatat agcatggcct 5160
   ttgcagggct ggcaagccac gtttggtggt ggcgaccatc ctccaaaatc ggatctggtt 5220
   ccgcgtggat cccggggtac cacaagtttg tacaaaaaag ctgaacgaga aacgtaaaat 5280
   gatataaata tcaatatatt aaattagatt ttgcataaaa aacagactac ataatactgt 5340
   aaaacacaac atatccagtc actatggcgg ccgcattagg caccccttag gaattcgctt 5400
   actaaaagcc agataacagt atgcgtattt gcgcgctgat ttttgcggta taagaatata 5460
   tactgatatg tatacccgaa gtatgtcaaa aagaggtgtg cttctagaat gcagtttaag 5520
   gtttacacct ataaaagaga gagccgttat cgtctgtttg tggatgtaca gagtgatatt 5580
   attgacacgc ccgggcgacg gatggtgatc cccctggcca gtgcacgtct gctgtcagat 5640
   aaagtctccc gtgaacttta cccggtggtg catatcgggg atgaaagctg gcgcatgatg 5700
   accaccgata tggccagtgt gccggtctcc gttatcgggg aagaagtggc tgatctcagc 5760
   caccgcgaaa atgacatcaa aaacgccatt aacctgatgt tctggggaat atagaattcc 5820
   tcaggtcgac catagtgact ggatatgttg tgttttacag tattatgtag tctgtttttt 5880
   atgcaaaatc taatttaata tattgatatt tatatcattt tacgtttctc gttcagcttt 5940
   cttgtacaaa gtggtctcga gttaattaat tgatccgggt tattagtaca tttattaagc 6000
   gctagattct gtgcgttgtt gatttacaga caattgttgt acgtatttta ataattcatt 6060
   aaatttataa tctttagggt ggtatgttag agcgaaaatc aaatgatttt cagcgtcttt 6120
   atatctgaat ttaaatatta aatcctcaat agatttgtaa aataggtttc gattagtttc 6180
   aaacaagggt tgtttttccg aaccgatggc tggactatct aatggatttt cgctcaacgc 6240
   cacaaaactt gccaaatctt gtagcagcaa tctagctttg tcgatattcg tttgtgtttt 6300
   gttttgtaat aaaggttcga cgtcgttcaa aatattatgc gcttttgtat ttctttcatc 6360
   actgtcgtta gtgtacaatt gactcgacgt aaacacgtta aataaagctt ggacatattt 6420
   aacatcgggc gtgttagctt tattaggccg attatcgtcg tcgtcccaac cctcgtcgtt 6480
   agaagttgct tccgaagacg attttgccat agccacacga cgcctattaa ttgtgtcggc 6540
   taacacgtcc gcgatcaaat ttgtagttga gctttttgga attatttctg attgcgggcg 6600
   tttttgggcg ggtttcaatc taactgtgcc cgattttaat tcagacaaca cgttagaaag 6660
   cgatggtgca ggcggtggta acatttcaga cggcaaatct actaatggcg gcggtggtgg 6720
   agctgatgat aaatctacca tcggtggagg cgcaggcggg gctggcggcg gaggcggagg 6780
   cggaggtggt ggcggtgatg cagacggcgg tttaggctca aatgtctctt taggcaacac 6840
   agtcggcacc tcaactattg tactggtttc gggcgccgtt tttggtttga ccggtctgag 6900
   acgagtgcga tttttttcgt ttctaatagc ttccaacaat tgttgtctgt cgtctaaagg 6960
   tgcagcgggt tgaggttccg tcggcattgg tggagcgggc ggcaattcag acatcgatgg 7020
   tggtggtggt ggtggaggcg ctggaatgtt aggcacggga gaaggtggtg gcggcggtgc 7080
   cgccggtata atttgttctg gtttagtttg ttcgcgcacg attgtgggca ccggcgcagg 7140
   cgccgctggc tgcacaacgg aaggtcgtct gcttcgaggc agcgcttggg gtggtggcaa 7200
   ttcaatatta taattggaat acaaatcgta aaaatctgct ataagcattg taatttcgct 7260
   atcgtttacc gtgccgatat ttaacaaccg ctcaatgtaa gcaattgtat tgtaaagaga 7320
   ttgtctcaag ctcggatccc gcacgccgat aacaagcctt ttcattttta ctacagcatt 7380
   gtagtggcga gacacttcgc tgtcgtcgac gtacatgtat gctttgttgt caaaaacgtc 7440
   gttggcaagc tttaaaatat ttaaaagaac atctctgttc agcaccactg tgttgtcgta 7500
   aatgttgttt ttgataattt gcgcttccgc agtatcgaca cgttcaaaaa attgatgcgc 7560
   atcaattttg ttgttcctat tattgaataa ataagattgt acagattcat atctacgatt 7620
   cgtcatggcc accacaaatg ctacgctgca aacgctggta caattttacg aaaactgcaa 7680
   aaacgtcaaa actcggtata aaataatcaa cgggcgcttt ggcaaaatat ctattttatc 7740
   gcacaagccc actagcaaat tgtatttgca gaaaacaatt tcggcgcaca attttaacgc 7800
   tgacgaaata aaagttcacc agttaatgag cgaccaccca aattttataa aaatctattt 7860
   taatcacggt tccatcaaca accaagtgat cgtgatggac tacattgact gtcccgattt 7920
   atttgaaaca ctacaaatta aaggcgagct ttcgtaccaa cttgttagca atattattag 7980
   acagctgtgt gaagcgctca acgatttgca caagcacaat ttcatacaca acgacataaa 8040
   actcgaaaat gtcttatatt tcgaagcact tgatcgcgtg tatgtttgcg attacggatt 8100
   gtgcaaacac gaaaactcac ttagcgtgca cgacggcacg ttggagtatt ttagtccgga 8160
   aaaaattcga cacacaacta tgcacgtttc gtttgactgg tacgccgtcg gcgtgttaac 8220
   atacaagttg ctaaccggcg gccgacaccc atttgaaaaa agcgaagacg aaatgttgga 8280
   cttgaatagc atgaagcgtc gtcagcaata caatgacatt ggcgttttaa aacacgttcg 8340
   taacgttaac gctcgtgact ttgtgtactg cctaacaaga tacaacatag attgtagact 8400
   cacaaattac aaacaaatta taaaacatga gtttttgtcg taaaaatgcc acttgtttta 8460
   cgagtagaat tctacgtgta acacacgatc taaaagatga tgtcattttt tatcaatgac 8520
   tcatttgttt taaaacagac ttgttttacg agtagaattc tacgtgtaaa gcatgatcgt 8580
   gagtggtgtt aataaaatca taaaaattat tgtaaatgtt tattatttaa aaacgattca 8640
   aatatataat aaaaacaatc tacatctatt tcttcacaat ccataacaca caacaggtcc 8700
   atcaatgagt ttttgtcttt atccgacata ctatgtgcat gtaacaaatc aaatacatct 8760
   tttaaatttt tatacacatc tttacattgt ctaccaaaat ctttaataac cctataacaa 8820
   ggaaaagact tttcttcttg cgtggttttg ccgcgcagat attgaaataa aatgtgcatg 8880
   cacgacaact tgtgtttact aaaatgctcc ttgcctatac cgcaaaaccg gccatacatt 8940
   tcggcgatta cacgcggaca attgtacgat tcgtctacgt gtaaacgatc atcataatca 9000
   ctcttgcgca aacgaataaa ttttttcacc gcttccgaca aacgaggcac caattcggcg 9060
   ggcacgcttc gatacattat tctgtgcaca taagttacca cacaaaattt attgtaccac 9120
   catccgacaa cgtcgttatt agggttgaac acgttggcga tgcgcagcag tttcccgttt 9180
   ctcatgaaat attcaaagcg gcccaaaata atttgcaagc aatccaacat gtcttgagaa 9240
   atttctcgtt caaaattgtt caaagagaat atctgccatc cgttttgaac gcgcacgctg 9300
   acgggaacca ccgcatcgat ttgctccaac acttcacgga cgttatcgtc gatgcccatc 9360
   gtttcgctgg tgctgaacca atgggaaagg ctcttgatgg aatcgcccgc gtctatcatc 9420
   ttgaccgctt cgtcaaaggt gcaactgccg ctcttcaaac gccgcatagc ggtcacgtcc 9480
   cgctctatgc acgacatacc gtttacgtac gattctgata ggtattcctg aactatacgg 9540
   taatggtgat acgactcgcc atacacgtcg tgcacctcat tgtatttagc ataataattg 9600
   taaattatta actttgcagc gagagacatg ttgtcagtaa agcggtgcta ggctcaataa 9660
   tactgatgta caggcacgcg tgctatttat atataatttc gcaaggaggg gagctgttat 9720
   cggttgctat tattaaagaa tggccgtctg tttttatcac aagcttggca gcctcaacca 9780
   tgaagcgtcg tcattgtaaa ttaaattctc tgcctcaaga attatttgac aagattgtcg 9840
   agtatttatc tttatctgat tactgcaatt tggtgcttgt ctgtaaaaga ccttctagta 9900
   aatataacgt gatatttgat agtactaatc accaacattt gaaaggcgtg tacaaaaaga 9960
   cagacgtgca aataacaagc tacaacgaat acatcaactg 10000
<210> 4
   <211> 10000
   <212> DNA
   <213> Artificial Sequence
<220>
   <223> Polyhedrin locus of vBacHTS_GST
<220>
   <221> CDS
   <222> (4528)..(5181)
   <223> F GST
<220>
   <221> misc_feature
   <222> (5482)..(5787)
   <223> F ccdB
<220>
   <221> promoter
   <222> (4428)..(4520)
   <223> F pPolyhedrin
<220>
   <221> misc_feature
   <222> (5215)..(5339)
   <223> F attR1
<220>
   <221> misc_feature
   <222> (5804)..(5928)
   <223> R attR2
<400> 4
   gaattctacc cgtaaagcga gtttagtttt gaaaaacaaa tgacatcatt tgtataatga 60
   catcatcccc tgattgtgtt ttacaagtag aattctatcc gtaaagcgag ttcagttttg 120
   aaaacaaatg agtcatacct aaacacgtta ataatcttct gatatcagct tatgactcaa 180
   gttatgagcc gtgtgcaaaa catgagataa gtttatgaca tcatccactg atcgtgcgtt 240
   acaagtagaa ttctactcgt aaagccagtt cggttatgag ccgtgtgcaa aacatgacat 300
   cagcttatga ctcatacttg attgtgtttt acgcgtagaa ttctactcgt aaagcgagtt 360
   cggttatgag ccgtgtgcaa aacatgacat cagcttatga gtcataatta atcgtgcgtt 420
   acaagtagaa ttctactcgt aaagcgagtt gaaggatcat atttagttgc gtttatgaga 480
   taagattgaa agcacgtgta aaatgtttcc cgcgcgttgg cacaactatt tacaatgcgg 540
   ccaagttata aaagattcta atctgatatg ttttaaaaca cctttgcggc ccgagttgtt 600
   tgcgtacgtg actagcgaag aagatgtgtg gaccgcagaa cagatagtaa aacaaaaccc 660
   tagtattgga gcaataatcg atttaaccaa cacgtctaaa tattatgatg gtgtgcattt 720
   tttgcgggcg ggcctgttat acaaaaaaat tcaagtacct ggccagactt tgccgcctga 780
   aagcatagtt caagaattta ttgacacggt aaaagaattt acagaaaagt gtcccggcat 840
   gttggtgggc gtgcactgca cacacggtat taatcgcacc ggttacatgg tgtgcagata 900
   tttaatgcac accctgggta ttgcgccgca ggaagccata gatagattcg aaaaagccag 960
   aggtcacaaa attgaaagac aaaattacgt tcaagattta ttaatttaat taatattatt 1020
   tgcattcttt aacaaatact ttatcctatt ttcaaattgt tgcgcttctt ccagcgaacc 1080
   aaaactatgc ttcgcttgct ccgtttagct tgtagccgat cagtggcgtt gttccaatcg 1140
   acggtaggat taggccggat attctccacc acaatgttgg caacgttgat gttacgttta 1200
   tgcttttggt tttccacgta cgtcttttgg ccggtaatag ccgtaaacgt agtgccgtcg 1260
   cgcgtcacgc acaacaccgg atgtttgcgc ttgtccgcgg ggtattgaac cgcgcgatcc 1320
   gacaaatcca ccactttggc aactaaatcg gtgacctgcg cgtctttttt ctgcattatt 1380
   tcgtctttct tttgcatggt ttcctggaag ccggtgtaca tgcggtttag atcagtcatg 1440
   acgcgcgtga cctgcaaatc tttggcctcg atctgcttgt ccttgatggc aacgatgcgt 1500
   tcaataaact cttgtttttt aacaagttcc tcggtttttt gcgccaccac cgcttgcagc 1560
   gcgtttgtgt gctcggtgaa tgtcgcaatc agcttagtca ccaactgttt gctctcctcc 1620
   tcccgttgtt tgatcgcggg atcgtacttg ccggtgcaga gcacttgagg aattacttct 1680
   tctaaaagcc attcttgtaa ttctatggcg taaggcaatt tggacttcat aatcagctga 1740
   atcacgccgg atttagtaat gagcactgta tgcggctgca aatacagcgg gtcgcccctt 1800
   ttcacgacgc tgttagaggt agggccccca ttttggatgg tctgctcaaa taacgatttg 1860
   tatttattgt ctacatgaac acgtatagct ttatcacaaa ctgtatattt taaactgtta 1920
   gcgacgtcct tggccacgaa ccggacctgt tggtcgcgct ctagcacgta ccgcaggttg 1980
   aacgtatctt ctccaaattt aaattctcca attttaacgc gagccatttt gatacacgtg 2040
   tgtcgatttt gcaacaacta ttgtttttta acgcaaacta aacttattgt ggtaagcaat 2100
   aattaaatat gggggaacat gcgccgctac aacactcgtc gttatgaacg cagacggcgc 2160
   cggtctcggc gcaagcggct aaaacgtgtt gcgcgttcaa cgcggcaaac atcgcaaaag 2220
   ccaatagtac agttttgatt tgcatattaa cggcgatttt ttaaattatc ttatttaata 2280
   aatagttatg acgcctacaa ctccccgccc gcgttgactc gctgcacctc gagcagttcg 2340
   ttgacgcctt cctccgtgtg gccgaacacg tcgagcgggt ggtcgatgac cagcggcgtg 2400
   ccgcacgcga cgcacaagta tctgtacacc gaatgatcgt cgggcgaagg cacgtcggcc 2460
   tccaagtggc aatattggca aattcgaaaa tatatacagt tgggttgttt gcgcatatct 2520
   atcgtggcgt tgggcatgta cgtccgaacg ttgatttgca tgcaagccga aattaaatca 2580
   ttgcgattag tgcgattaaa acgttgtaca tcctcgcttt taatcatgcc gtcgattaaa 2640
   tcgcgcaatc gagtcaagtg atcaaagtgt ggaataatgt tttctttgta ttcccgagtc 2700
   aagcgcagcg cgtattttaa caaactagcc atcttgtaag ttagtttcat ttaatgcaac 2760
   tttatccaat aatatattat gtatcgcacg tcaagaatta acaatgcgcc cgttgtcgca 2820
   tctcaacacg actatgatag agatcaaata aagcgcgaat taaatagctt gcgacgcaac 2880
   gtgcacgatc tgtgcacgcg ttccggcacg agctttgatt gtaataagtt tttacgaagc 2940
   gatgacatga cccccgtagt gacaacgatc acgcccaaaa gaactgccga ctacaaaatt 3000
   accgagtatg tcggtgacgt taaaactatt aagccatcca atcgaccgtt agtcgaatca 3060
   ggaccgctgg tgcgagaagc cgcgaagtat ggcgaatgca tcgtataacg tgtggagtcc 3120
   gctcattaga gcgtcatgtt tagacaagaa agctacatat ttaattgatc ccgatgattt 3180
   tattgataaa ttgaccctaa ctccatacac ggtattctac aatggcgggg ttttggtcaa 3240
   aatttccgga ctgcgattgt acatgctgtt aacggctccg cccactatta atgaaattaa 3300
   aaattccaat tttaaaaaac gcagcaagag aaacatttgt atgaaagaat gcgtagaagg 3360
   aaagaaaaat gtcgtcgaca tgctgaacaa caagattaat atgcctccgt gtataaaaaa 3420
   aatattgaac gatttgaaag aaaacaatgt accgcgcggc ggtatgtaca ggaagaggtt 3480
   tatactaaac tgttacattg caaacgtggt ttcgtgtgcc aagtgtgaaa accgatgttt 3540
   aatcaaggct ctgacgcatt tctacaacca cgactccaag tgtgtgggtg aagtcatgca 3600
   tcttttaatc aaatcccaag atgtgtataa accaccaaac tgccaaaaaa tgaaaactgt 3660
   cgacaagctc tgtccgtttg ctggcaactg caagggtctc aatcctattt gtaattattg 3720
   aataataaaa caattataaa tgctaaattt gttttttatt aacgatacaa accaaacgca    3780
   acaagaacat ttgtagtatt atctataatt gaaaacgcgt agttataatc gctgaggtaa    3840
   tatttaaaat cattttcaaa tgattcacag ttaatttgcg acaatataat tttattttca    3900
   cataaactag acgccttgtc gtcttcttct tcgtattcct tctctttttc atttttctcc      3960
   tcataaaaat taacatagtt attatcgtat ccatatatgt atctatcgta tagagtaaat    4020
   tttttgttgt cataaatata tatgtctttt ttaatggggt gtatagtacc gctgcgcata    4080
   gtttttctgt aatttacaac agtgctattt tctggtagtt cttcggagtg tgttgcttta    4140
   attattaaat ttatataatc aatgaatttg ggatcgtcgg ttttgtacaa tatgttgccg    4200
   gcatagtacg cagcttcttc tagttcaatt acaccatttt ttagcagcac cggattaaca    4260
   taactttcca aaatgttgta cgaaccgtta aacaaaaaca gttcacctcc cttttctata    4320
   ctattgtctg cgagcagttg tttgttgtta aaaataacag ccattgtaat gagacgcaca    4380
   aactaatatc acaaactgga aatgtctatc aatatatagt tgctgatatc atggagataa    4440
   ttaaaatgat aaccatctcg caaataaata agtattttac tgttttcgta acagttttgt    4500
   aataaaaaaa cctataaata cggatct   atg tcc cct ata cta ggt tat tgg      4551
                                    Met Ser Pro Ile Leu Gly Tyr Trp
                                     1               5
   aaa att aag ggc ctt gtg caa ccc act cga ctt ctt ttg gaa tat ctt      4599
   Lys Ile Lys Gly Leu Val Gln Pro Thr Arg Leu Leu Leu Glu Tyr Leu
           10                  15                  20
   gaa gaa aaa tat gaa gag cat ttg tat gag cgc gat gaa ggt gat aaa      4647
   Glu Glu Lys Tyr Glu Glu His Leu Tyr Glu Arg Asp Glu Gly Asp Lys
   25 30 35 40
   tgg cga aac aaa aag ttt gaa ttg ggt ttg gag ttt ccc aat ctt cct 4695
   Trp Arg Asn Lys Lys Phe Glu Leu Gly Leu Glu Phe Pro Asn Leu Pro
   45 50 55
   tat tat att gat ggt gat gtt aaa tta aca cag tct atg gcc atc ata 4743
   Tyr Tyr Ile Asp Gly Asp Val Lys Leu Thr Gln Ser Met Ala Ile Ile
   60 65 70
   cgt tat ata gct gac aag cac aac atg ttg ggt ggt tgt cca aaa gag 4791
   Arg Tyr Ile Ala Asp Lys His Asn Met Leu Gly Gly Cys Pro Lys Glu
   75 80 85
   cgt gca gag att tca atg ctt gaa gga gcg gtt ttg gat att aga tac 4839
   Arg Ala Glu Ile Ser Met Leu Glu Gly Ala Val Leu Asp Ile Arg Tyr
   90 95 100
   ggt gtt tcg aga att gca tat agt aaa gac ttt gaa act ctc aaa gtt 4887
   Gly Val Ser Arg Ile Ala Tyr Ser Lys Asp Phe Glu Thr Leu Lys Val
   105 110 115 120
   gat ttt ctt agc aag cta cct gaa atg ctg aaa atg ttc gaa gat cgt 4935
   Asp Phe Leu Ser Lys Leu Pro Glu Met Leu Lys Met Phe Glu Asp Arg
   125 130 135
   tta tgt cat aaa aca tat tta aat ggt gat cat gta acc cat cct gac 4983
   Leu Cys His Lys Thr Tyr Leu Asn Gly Asp His Val Thr His Pro Asp
   140 145 150
   ttc atg ttg tat gac gct ctt gat gtt gtt tta tac atg gac cca atg 5031
   Phe Met Leu Tyr Asp Ala Leu Asp Val Val Leu Tyr Met Asp Pro Met
   155 160 165
   tgc ctg gat gcg ttc cca aaa tta gtt tgt ttt aaa aaa cgt att gaa 5079
   Cys Leu Asp Ala Phe Pro Lys Leu Val Cys Phe Lys Lys Arg Ile Glu
   170 175 180
   gct atc cca caa att gat aag tac ttg aaa tcc agc aag tat ata gca 5127
   Ala Ile Pro Gln Ile Asp Lys Tyr Leu Lys Ser Ser Lys Tyr Ile Ala
   185 190 195 200
   tgg cct ttg cag ggc tgg caa gcc acg ttt ggt ggt ggc gac cat cct 5175
   Trp Pro Leu Gln Gly Trp Gln Ala Thr Phe Gly Gly Gly Asp His Pro
   205 210 215
   cca aaa tcggatctg gttccgcgtg gatcccgggg taccacaagt ttgtacaaaa 5230
   Pro Lys
   aagctgaacg agaaacgtaa aatgatataa atatcaatat attaaattag attttgcata 5290
   aaaaacagac tacataatac tgtaaaacac aacatatcca gtcactatgg cggccgcatt 5350
   aggcacccct taggaattcg cttactaaaa gccagataac agtatgcgta tttgcgcgct 5410
   gatttttgcg gtataagaat atatactgat atgtataccc gaagtatgtc aaaaagaggt 5470
   gtgcttctag aatgcagttt aaggtttaca cctataaaag agagagccgt tatcgtctgt 5530
   ttgtggatgt acagagtgat attattgaca cgcccgggcg acggatggtg atccccctgg 5590
   ccagtgcacg tctgctgtca gataaagtct cccgtgaact ttacccggtg gtgcatatcg 5650
   gggatgaaag ctggcgcatg atgaccaccg atatggccag tgtgccggtc tccgttatcg 5710
   gggaagaagt ggctgatctc agccaccgcg aaaatgacat caaaaacgcc attaacctga 5770
   tgttctgggg aatatagaat tcctcaggtc gaccatagtg actggatatg ttgtgtttta 5830
   cagtattatg tagtctgttt tttatgcaaa atctaattta atatattgat atttatatca 5890
   ttttacgttt ctcgttcagc tttcttgtac aaagtggtct cgagttaatt aattgatccg 5950
   ggttattagt acatttatta agcgctagat tctgtgcgtt gttgatttac agacaattgt 6010
   tgtacgtatt ttaataattc attaaattta taatctttag ggtggtatgt tagagcgaaa 6070
   atcaaatgat tttcagcgtc tttatatctg aatttaaata ttaaatcctc aatagatttg 6130
   taaaataggt ttcgattagt ttcaaacaag ggttgttttt ccgaaccgat ggctggacta 6190
   tctaatggat tttcgctcaa cgccacaaaa cttgccaaat cttgtagcag caatctagct 6250
   ttgtcgatat tcgtttgtgt tttgttttgt aataaaggtt cgacgtcgtt caaaatatta 6310
   tgcgcttttg tatttctttc atcactgtcg ttagtgtaca attgactcga cgtaaacacg 6370
   ttaaataaag cttggacata tttaacatcg ggcgtgttag ctttattagg ccgattatcg 6430
   tcgtcgtccc aaccctcgtc gttagaagtt gcttccgaag acgattttgc catagccaca 6490
   cgacgcctat taattgtgtc ggctaacacg tccgcgatca aatttgtagt tgagcttttt 6550
   ggaattattt ctgattgcgg gcgtttttgg gcgggtttca atctaactgt gcccgatttt 6610
   aattcagaca acacgttaga aagcgatggt gcaggcggtg gtaacatttc agacggcaaa 6670
   tctactaatg gcggcggtgg tggagctgat gataaatcta ccatcggtgg aggcgcaggc 6730
   ggggctggcg gcggaggcgg aggcggaggt ggtggcggtg atgcagacgg cggtttaggc 6790
   tcaaatgtct ctttaggcaa cacagtcggc acctcaacta ttgtactggt ttcgggcgcc 6850
   gtttttggtt tgaccggtct gagacgagtg cgattttttt cgtttctaat agcttccaac 6910
   aattgttgtc tgtcgtctaa aggtgcagcg ggttgaggtt ccgtcggcat tggtggagcg 6970
   ggcggcaatt cagacatcga tggtggtggt ggtggtggag gcgctggaat gttaggcacg 7030
   ggagaaggtg gtggcggcgg tgccgccggt ataatttgtt ctggtttagt ttgttcgcgc 7090
   acgattgtgg gcaccggcgc aggcgccgct ggctgcacaa cggaaggtcg tctgcttcga 7150
   ggcagcgctt ggggtggtgg caattcaata ttataattgg aatacaaatc gtaaaaatct 7210
   gctataagca ttgtaatttc gctatcgttt accgtgccga tatttaacaa ccgctcaatg 7270
   taagcaattg tattgtaaag agattgtctc aagctcggat cccgcacgcc gataacaagc 7330
   cttttcattt ttactacagc attgtagtgg cgagacactt cgctgtcgtc gacgtacatg 7390
   tatgctttgt tgtcaaaaac gtcgttggca agctttaaaa tatttaaaag aacatctctg 7450
   ttcagcacca ctgtgttgtc gtaaatgttg tttttgataa tttgcgcttc cgcagtatcg 7510
   acacgttcaa aaaattgatg cgcatcaatt ttgttgttcc tattattgaa taaataagat 7570
   tgtacagatt catatctacg attcgtcatg gccaccacaa atgctacgct gcaaacgctg 7630
   gtacaatttt acgaaaactg caaaaacgtc aaaactcggt ataaaataat caacgggcgc 7690
   tttggcaaaa tatctatttt atcgcacaag cccactagca aattgtattt gcagaaaaca 7750
   atttcggcgc acaattttaa cgctgacgaa ataaaagttc accagttaat gagcgaccac 7810
   ccaaatttta taaaaatcta ttttaatcac ggttccatca acaaccaagt gatcgtgatg 7870
   gactacattg actgtcccga tttatttgaa acactacaaa ttaaaggcga gctttcgtac 7930
   caacttgtta gcaatattat tagacagctg tgtgaagcgc tcaacgattt gcacaagcac 7990
   aatttcatac acaacgacat aaaactcgaa aatgtcttat atttcgaagc acttgatcgc 8050
   gtgtatgttt gcgattacgg attgtgcaaa cacgaaaact cacttagcgt gcacgacggc 8110
   acgttggagt attttagtcc ggaaaaaatt cgacacacaa ctatgcacgt ttcgtttgac 8170
   tggtacgccg tcggcgtgtt aacatacaag ttgctaaccg gcggccgaca cccatttgaa 8230
   aaaagcgaag acgaaatgtt ggacttgaat agcatgaagc gtcgtcagca atacaatgac 8290
   attggcgttt taaaacacgt tcgtaacgtt aacgctcgtg actttgtgta ctgcctaaca 8350
   agatacaaca tagattgtag actcacaaat tacaaacaaa ttataaaaca tgagtttttg 8410
   tcgtaaaaat gccacttgtt ttacgagtag aattctacgt gtaacacacg atctaaaaga 8470
   tgatgtcatt ttttatcaat gactcatttg ttttaaaaca gacttgtttt acgagtagaa 8530
   ttctacgtgt aaagcatgat cgtgagtggt gttaataaaa tcataaaaat tattgtaaat 8590
   gtttattatt taaaaacgat tcaaatatat aataaaaaca atctacatct atttcttcac 8650
   aatccataac acacaacagg tccatcaatg agtttttgtc tttatccgac atactatgtg 8710
   catgtaacaa atcaaataca tcttttaaat ttttatacac atctttacat tgtctaccaa 8770
   aatctttaat aaccctataa caaggaaaag acttttcttc ttgcgtggtt ttgccgcgca 8830
   gatattgaaa taaaatgtgc atgcacgaca acttgtgttt actaaaatgc tccttgccta 8890
   taccgcaaaa ccggccatac atttcggcga ttacacgcgg acaattgtac gattcgtcta 8950
   cgtgtaaacg atcatcataa tcactcttgc gcaaacgaat aaattttttc accgcttccg 9010
   acaaacgagg caccaattcg gcgggcacgc ttcgatacat tattctgtgc acataagtta 9070
   ccacacaaaa tttattgtac caccatccga caacgtcgtt attagggttg aacacgttgg 9130
   cgatgcgcag cagtttcccg tttctcatga aatattcaaa gcggcccaaa ataatttgca 9190
   agcaatccaa catgtcttga gaaatttctc gttcaaaatt gttcaaagag aatatctgcc 9250
   atccgttttg aacgcgcacg ctgacgggaa ccaccgcatc gatttgctcc aacacttcac 9310
   ggacgttatc gtcgatgccc atcgtttcgc tggtgctgaa ccaatgggaa aggctcttga 9370
   tggaatcgcc cgcgtctatc atcttgaccg cttcgtcaaa ggtgcaactg ccgctcttca 9430
   aacgccgcat agcggtcacg tcccgctcta tgcacgacat accgtttacg tacgattctg 9490
   ataggtattc ctgaactata cggtaatggt gatacgactc gccatacacg tcgtgcacct 9550
   cattgtattt agcataataa ttgtaaatta ttaactttgc agcgagagac atgttgtcag 9610
   taaagcggtg ctaggctcaa taatactgat gtacaggcac gcgtgctatt tatatataat 9670
   ttcgcaagga ggggagctgt tatcggttgc tattattaaa gaatggccgt ctgtttttat 9730
   cacaagcttg gcagcctcaa ccatgaagcg tcgtcattgt aaattaaatt ctctgcctca 9790
   agaattattt gacaagattg tcgagtattt atctttatct gattactgca atttggtgct 9850
   tgtctgtaaa agaccttcta gtaaatataa cgtgatattt gatagtacta atcaccaaca 9910
   tttgaaaggc gtgtacaaaa agacagacgt gcaaataaca agctacaacg aatacatcaa 9970
   ctgtatttgc aacgaactga gacaagacga 10000
<210> 5
   <211> 10000
   <212> DNA
   <213> Artificial Sequence
<220>
   <223> Polyhedrin locus of vBacHTS_GFP virus
<220>
   <221> CDS
   <222> (4531)..(5247)
   <223> F EGFP
<220>
   <221> disc-features
   <222> (5521)..(5826)
   <223> F ccdB
<220>
   <221> promoter
   <222> (4428)..(4520)
   <223> F pPolyhedrin
<220>
   <221> misc_feature
   <222> (5843)..(5967)
   <223> R attR2
<220>
   <221> misc_feature
   <222> (5254)..(5378)
   <223> F attR1
<400> 5
   gaattctacc cgtaaagcga gtttagtttt gaaaaacaaa tgacatcatt tgtataatga 60
   catcatcccc tgattgtgtt ttacaagtag aattctatcc gtaaagcgag ttcagttttg 120
   aaaacaaatg agtcatacct aaacacgtta ataatcttct gatatcagct tatgactcaa 180
   gttatgagcc gtgtgcaaaa catgagataa gtttatgaca tcatccactg atcgtgcgtt 240
   acaagtagaa ttctactcgt aaagccagtt cggttatgag ccgtgtgcaa aacatgacat 300
   cagcttatga ctcatacttg attgtgtttt acgcgtagaa ttctactcgt aaagcgagtt 360
   cggttatgag ccgtgtgcaa aacatgacat cagcttatga gtcataatta atcgtgcgtt 420
   acaagtagaa ttctactcgt aaagcgagtt gaaggatcat atttagttgc gtttatgaga 480
   taagattgaa agcacgtgta aaatgtttcc cgcgcgttgg cacaactatt tacaatgcgg 540
   ccaagttata aaagattcta atctgatatg ttttaaaaca cctttgcggc ccgagttgtt 600
   tgcgtacgtg actagcgaag aagatgtgtg gaccgcagaa cagatagtaa aacaaaaccc 660
   tagtattgga gcaataatcg atttaaccaa cacgtctaaa tattatgatg gtgtgcattt 720
   tttgcgggcg ggcctgttat acaaaaaaat tcaagtacct ggccagactt tgccgcctga 780
   aagcatagtt caagaattta ttgacacggt aaaagaattt acagaaaagt gtcccggcat 840
   gttggtgggc gtgcactgca cacacggtat taatcgcacc ggttacatgg tgtgcagata 900
   tttaatgcac accctgggta ttgcgccgca ggaagccata gatagattcg aaaaagccag 960
   aggtcacaaa attgaaagac aaaattacgt tcaagattta ttaatttaat taatattatt 1020
   tgcattcttt aacaaatact ttatcctatt ttcaaattgt tgcgcttctt ccagcgaacc 1080
   aaaactatgc ttcgcttgct ccgtttagct tgtagccgat cagtggcgtt gttccaatcg 1140
   acggtaggat taggccggat attctccacc acaatgttgg caacgttgat gttacgttta 1200
   tgcttttggt tttccacgta cgtcttttgg ccggtaatag ccgtaaacgt agtgccgtcg 1260
   cgcgtcacgc acaacaccgg atgtttgcgc ttgtccgcgg ggtattgaac cgcgcgatcc 1320
   gacaaatcca ccactttggc aactaaatcg gtgacctgcg cgtctttttt ctgcattatt 1380
   tcgtctttct tttgcatggt ttcctggaag ccggtgtaca tgcggtttag atcagtcatg 1440
   acgcgcgtga cctgcaaatc tttggcctcg atctgcttgt ccttgatggc aacgatgcgt 1500
   tcaataaact cttgtttttt aacaagttcc tcggtttttt gcgccaccac cgcttgcagc 1560
   gcgtttgtgt gctcggtgaa tgtcgcaatc agcttagtca ccaactgttt gctctcctcc 1620
   tcccgttgtt tgatcgcggg atcgtacttg ccggtgcaga gcacttgagg aattacttct 1680
   tctaaaagcc attcttgtaa ttctatggcg taaggcaatt tggacttcat aatcagctga 1740
   atcacgccgg atttagtaat gagcactgta tgcggctgca aatacagcgg gtcgcccctt 1800
   ttcacgacgc tgttagaggt agggccccca ttttggatgg tctgctcaaa taacgatttg 1860
   tatttattgt ctacatgaac acgtatagct ttatcacaaa ctgtatattt taaactgtta 1920
   gcgacgtcct tggccacgaa ccggacctgt tggtcgcgct ctagcacgta ccgcaggttg 1980
   aacgtatctt ctccaaattt aaattctcca attttaacgc gagccatttt gatacacgtg 2040
   tgtcgatttt gcaacaacta ttgtttttta acgcaaacta aacttattgt ggtaagcaat 2100
   aattaaatat gggggaacat gcgccgctac aacactcgtc gttatgaacg cagacggcgc 2160
   cggtctcggc gcaagcggct aaaacgtgtt gcgcgttcaa cgcggcaaac atcgcaaaag 2220
   ccaatagtac agttttgatt tgcatattaa cggcgatttt ttaaattatc ttatttaata 2280
   aatagttatg acgcctacaa ctccccgccc gcgttgactc gctgcacctc gagcagttcg 2340
   ttgacgcctt cctccgtgtg gccgaacacg tcgagcgggt ggtcgatgac cagcggcgtg 2400
   ccgcacgcga cgcacaagta tctgtacacc gaatgatcgt cgggcgaagg cacgtcggcc 2460
   tccaagtggc aatattggca aattcgaaaa tatatacagt tgggttgttt gcgcatatct 2520
   atcgtggcgt tgggcatgta cgtccgaacg ttgatttgca tgcaagccga aattaaatca 2580
   ttgcgattag tgcgattaaa acgttgtaca tcctcgcttt taatcatgcc gtcgattaaa 2640
   tcgcgcaatc gagtcaagtg atcaaagtgt ggaataatgt tttctttgta ttcccgagtc 2700
   aagcgcagcg cgtattttaa caaactagcc atcttgtaag ttagtttcat ttaatgcaac 2760
   tttatccaat aatatattat gtatcgcacg tcaagaatta acaatgcgcc cgttgtcgca 2820
   tctcaacacg actatgatag agatcaaata aagcgcgaat taaatagctt gcgacgcaac 2880
   gtgcacgatc tgtgcacgcg ttccggcacg agctttgatt gtaataagtt tttacgaagc 2940
   gatgacatga cccccgtagt gacaacgatc acgcccaaaa gaactgccga ctacaaaatt 3000
   accgagtatg tcggtgacgt taaaactatt aagccatcca atcgaccgtt agtcgaatca 3060
   ggaccgctgg tgcgagaagc cgcgaagtat ggcgaatgca tcgtataacg tgtggagtcc 3120
   gctcattaga gcgtcatgtt tagacaagaa agctacatat ttaattgatc ccgatgattt 3180
   tattgataaa ttgaccctaa ctccatacac ggtattctac aatggcgggg ttttggtcaa 3240
   aatttccgga ctgcgattgt acatgctgtt aacggctccg cccactatta atgaaattaa 3300
   aaattccaat tttaaaaaac gcagcaagag aaacatttgt atgaaagaat gcgtagaagg 3360
   aaagaaaaat gtcgtcgaca tgctgaacaa caagattaat atgcctccgt gtataaaaaa 3420
   aatattgaac gatttgaaag aaaacaatgt accgcgcggc ggtatgtaca ggaagaggtt 3480
   tatactaaac tgttacattg caaacgtggt ttcgtgtgcc aagtgtgaaa accgatgttt 3540
   aatcaaggct ctgacgcatt tctacaacca cgactccaag tgtgtgggtg aagtcatgca 3600
   tcttttaatc aaatcccaag atgtgtataa accaccaaac tgccaaaaaa tgaaaactgt 3660
   cgacaagctc tgtccgtttg ctggcaactg caagggtctc aatcctattt gtaattattg 3720
   aataataaaa caattataaa tgctaaattt gttttttatt aacgatacaa accaaacgca 3780
   acaagaacat ttgtagtatt atctataatt gaaaacgcgt agttataatc gctgaggtaa 3840
   tatttaaaat cattttcaaa tgattcacag ttaatttgcg acaatataat tttattttca 3900
   cataaactag acgccttgtc gtcttcttct tcgtattcct tctctttttc atttttctcc 3960
   tcataaaaat taacatagtt attatcgtat ccatatatgt atctatcgta tagagtaaat 4020
   tttttgttgt cataaatata tatgtctttt ttaatggggt gtatagtacc gctgcgcata 4080
   gtttttctgt aatttacaac agtgctattt tctggtagtt cttcggagtg tgttgcttta 4140
   attattaaat ttatataatc aatgaatttg ggatcgtcgg ttttgtacaa tatgttgccg 4200
   gcatagtacg cagcttcttc tagttcaatt acaccatttt ttagcagcac cggattaaca 4260
   taactttcca aaatgttgta cgaaccgtta aacaaaaaca gttcacctcc cttttctata 4320
   ctattgtctg cgagcagttg tttgttgtta aaaataacag ccattgtaat gagacgcaca 4380
   aactaatatc acaaactgga aatgtctatc aatatatagt tgctgatatc atggagataa      4440
   ttaaaatgat aaccatctcg caaataaata agtattttac tgttttcgta acagttttgt      4500
   aataaaaaaa cctataaata cggatccacc atg gtg agc aag ggc gag gag ctg       4554
                                    Met Val Ser Lys Gly Glu Glu Leu
                                    1               5
   ttc acc ggg gtg gtg ccc atc ctg gtc gag ctg gac ggc gac gta aac        4602
   Phe Thr Gly Val Val Pro Ile Leu Val Glu Leu Asp Gly Asp Val Asn
       10                  15                  20
   ggc cac aag ttc agc gtg tcc ggc gag ggc gag ggc gat gcc acc tac        4650
   Gly His Lys Phe Ser Val Ser Gly Glu Gly Glu Gly Asp Ala Thr Tyr
   25                  30                  35                  40
   ggc aag ctg acc ctg aag ttc atc tgc acc acc ggc aag ctg ccc gtg        4698
   Gly Lys Leu Thr Leu Lys Phe Ile Cys Thr Thr Gly Lys Leu Pro Val
                   45                  50                  55
   ccc tgg ccc acc ctc gtg acc acc ctg acc tac ggc gtg cag tgc ttc        4746
   Pro Trp Pro Thr Leu Val Thr Thr Leu Thr Tyr Gly Val Gln Cys Phe
               60                  65                  70
   agc cgc tac ccc gac cac atg aag cag cac gac ttc ttc aag tcc gcc        4794
   Ser Arg Tyr Pro Asp His Met Lys Gln His Asp Phe Phe Lys Ser Ala
           75                  80                  85
   atg ccc gaa ggc tac gtc cag gag cgc acc atc ttc ttc aag gac gac        4842
   Met Pro Glu Gly Tyr Val Gln Glu Arg Thr Ile Phe Phe Lys Asp Asp
       90                  95                  100
   ggc aac tac aag acc cgc gcc gag gtg aag ttc gag ggc gac acc ctg        4890
   Gly Asn Tyr Lys Thr Arg Ala Glu Val Lys Phe Glu Gly Asp Thr Leu
   105                 110                 115                 120
   gtg aac cgc atc gag ctg aag ggc atc gac ttc aag gag gac ggc aac 4938
   Val Asn Arg Ile Glu Leu Lys Gly Ile Asp Phe Lys Glu Asp Gly Asn
   125 130 135
   atc ctg ggg cac aag ctg gag tac aac tac aac agc cac aac gtc tat 4986
   Ile Leu Gly His Lys Leu Glu Tyr Asn Tyr Asn Ser His Asn Val Tyr
   140 145 150
   atc atg gcc gac aag cag aag aac ggc atc aag gtg aac ttc aag atc 5034
   Ile Met Ala Asp Lys Gln Lys Asn Gly Ile Lys Val Asn Phe Lys Ile
   155 160 165
   cgc cac aac atc gag gac ggc agc gtg cag ctc gcc gac cac tac cag 5082
   Arg His Asn Ile Glu Asp Gly Ser Val Gln Leu Ala Asp His Tyr Gln
   170 175 180
   cag aac acc ccc atc ggc gac ggc ccc gtg ctg ctg ccc gac aac cac 5130
   Gln Asn Thr Pro Ile Gly Asp Gly Pro Val Leu Leu Pro Asp Asn His
   185 190 195 200
   tac ctg agc acc cag tcc gcc ctg agc aaa gac ccc aac gag aag cgc 5178
   Tyr Leu Ser Thr Gln Ser Ala Leu Ser Lys Asp Pro Asn Glu Lys Arg
   205 210 215
   gat cac atg gtc ctg ctg gag ttc gtg acc gcc gcc ggg atc act ctc 5226
   Asp His Met Val Leu Leu Glu Phe Val Thr Ala Ala Gly Ile Thr Leu
   220 225 230
   ggc atg gac gag ctg tac aag ggt accacaagtt tgtacaaaaa agctgaacga 5280
   Gly Met Asp Glu Leu Tyr Lys
   235
   gaaacgtaaa atgatataaa tatcaatata ttaaattaga ttttgcataa aaaacagact 5340
   acataatact gtaaaacaca acatatccag tcactatggc ggccgcatta ggcacccctt 5400
   aggaattcgc ttactaaaag ccagataaca gtatgcgtat ttgcgcgctg atttttgcgg 5460
   tataagaata tatactgata tgtatacccg aagtatgtca aaaagaggtg tgcttctaga 5520
   atgcagttta aggtttacac ctataaaaga gagagccgtt atcgtctgtt tgtggatgta 5580
   cagagtgata ttattgacac gcccgggcga cggatggtga tccccctggc cagtgcacgt 5640
   ctgctgtcag ataaagtctc ccgtgaactt tacccggtgg tgcatatcgg ggatgaaagc 5700
   tggcgcatga tgaccaccga tatggccagt gtgccggtct ccgttatcgg ggaagaagtg 5760
   gctgatctca gccaccgcga aaatgacatc aaaaacgcca ttaacctgat gttctgggga 5820
   atatagaatt cctcaggtcg accatagtga ctggatatgt tgtgttttac agtattatgt 5880
   agtctgtttt ttatgcaaaa tctaatttaa tatattgata tttatatcat tttacgtttc 5940
   tcgttcagct ttcttgtaca aagtggtctc gagttaatta attgatccgg gttattagta 6000
   catttattaa gcgctagatt ctgtgcgttg ttgatttaca gacaattgtt gtacgtattt 6060
   taataattca ttaaatttat aatctttagg gtggtatgtt agagcgaaaa tcaaatgatt 6120
   ttcagcgtct ttatatctga atttaaatat taaatcctca atagatttgt aaaataggtt 6180
   tcgattagtt tcaaacaagg gttgtttttc cgaaccgatg gctggactat ctaatggatt 6240
   ttcgctcaac gccacaaaac ttgccaaatc ttgtagcagc aatctagctt tgtcgatatt 6300
   cgtttgtgtt ttgttttgta ataaaggttc gacgtcgttc aaaatattat gcgcttttgt 6360
   atttctttca tcactgtcgt tagtgtacaa ttgactcgac gtaaacacgt taaataaagc 6420
   ttggacatat ttaacatcgg gcgtgttagc tttattaggc cgattatcgt cgtcgtccca 6480
   accctcgtcg ttagaagttg cttccgaaga cgattttgcc atagccacac gacgcctatt 6540
   aattgtgtcg gctaacacgt ccgcgatcaa atttgtagtt gagctttttg gaattatttc 6600
   tgattgcggg cgtttttggg cgggtttcaa tctaactgtg cccgatttta attcagacaa 6660
   cacgttagaa agcgatggtg caggcggtgg taacatttca gacggcaaat ctactaatgg 6720
   cggcggtggt ggagctgatg ataaatctac catcggtgga ggcgcaggcg gggctggcgg 6780
   cggaggcgga ggcggaggtg gtggcggtga tgcagacggc ggtttaggct caaatgtctc 6840
   tttaggcaac acagtcggca cctcaactat tgtactggtt tcgggcgccg tttttggttt 6900
   gaccggtctg agacgagtgc gatttttttc gtttctaata gcttccaaca attgttgtct 6960
   gtcgtctaaa ggtgcagcgg gttgaggttc cgtcggcatt ggtggagcgg gcggcaattc 7020
   agacatcgat ggtggtggtg gtggtggagg cgctggaatg ttaggcacgg gagaaggtgg 7080
   tggcggcggt gccgccggta taatttgttc tggtttagtt tgttcgcgca cgattgtggg 7140
   caccggcgca ggcgccgctg gctgcacaac ggaaggtcgt ctgcttcgag gcagcgcttg 7200
   gggtggtggc aattcaatat tataattgga atacaaatcg taaaaatctg ctataagcat 7260
   tgtaatttcg ctatcgttta ccgtgccgat atttaacaac cgctcaatgt aagcaattgt 7320
   attgtaaaga gattgtctca agctcggatc ccgcacgccg ataacaagcc ttttcatttt 7380
   tactacagca ttgtagtggc gagacacttc gctgtcgtcg acgtacatgt atgctttgtt 7440
   gtcaaaaacg tcgttggcaa gctttaaaat atttaaaaga acatctctgt tcagcaccac 7500
   tgtgttgtcg taaatgttgt ttttgataat ttgcgcttcc gcagtatcga cacgttcaaa 7560
   aaattgatgc gcatcaattt tgttgttcct attattgaat aaataagatt gtacagattc 7620
   atatctacga ttcgtcatgg ccaccacaaa tgctacgctg caaacgctgg tacaatttta 7680
   cgaaaactgc aaaaacgtca aaactcggta taaaataatc aacgggcgct ttggcaaaat 7740
   atctatttta tcgcacaagc ccactagcaa attgtatttg cagaaaacaa tttcggcgca 7800
   caattttaac gctgacgaaa taaaagttca ccagttaatg agcgaccacc caaattttat 7860
   aaaaatctat tttaatcacg gttccatcaa caaccaagtg atcgtgatgg actacattga 7920
   ctgtcccgat ttatttgaaa cactacaaat taaaggcgag ctttcgtacc aacttgttag 7980
   caatattatt agacagctgt gtgaagcgct caacgatttg cacaagcaca atttcataca 8040
   caacgacata aaactcgaaa atgtcttata tttcgaagca cttgatcgcg tgtatgtttg 8100
   cgattacgga ttgtgcaaac acgaaaactc acttagcgtg cacgacggca cgttggagta 8160
   ttttagtccg gaaaaaattc gacacacaac tatgcacgtt tcgtttgact ggtacgccgt 8220
   cggcgtgtta acatacaagt tgctaaccgg cggccgacac ccatttgaaa aaagcgaaga 8280
   cgaaatgttg gacttgaata gcatgaagcg tcgtcagcaa tacaatgaca ttggcgtttt 8340
   aaaacacgtt cgtaacgtta acgctcgtga ctttgtgtac tgcctaacaa gatacaacat 8400
   agattgtaga ctcacaaatt acaaacaaat tataaaacat gagtttttgt cgtaaaaatg 8460
   ccacttgttt tacgagtaga attctacgtg taacacacga tctaaaagat gatgtcattt 8520
   tttatcaatg actcatttgt tttaaaacag acttgtttta cgagtagaat tctacgtgta 8580
   aagcatgatc gtgagtggtg ttaataaaat cataaaaatt attgtaaatg tttattattt 8640
   aaaaacgatt caaatatata ataaaaacaa tctacatcta tttcttcaca atccataaca 8700
   cacaacaggt ccatcaatga gtttttgtct ttatccgaca tactatgtgc atgtaacaaa 8760
   tcaaatacat cttttaaatt tttatacaca tctttacatt gtctaccaaa atctttaata 8820
   accctataac aaggaaaaga cttttcttct tgcgtggttt tgccgcgcag atattgaaat 8880
   aaaatgtgca tgcacgacaa cttgtgttta ctaaaatgct ccttgcctat accgcaaaac 8940
   cggccataca tttcggcgat tacacgcgga caattgtacg attcgtctac gtgtaaacga 9000
   tcatcataat cactcttgcg caaacgaata aattttttca ccgcttccga caaacgaggc 9060
   accaattcgg cgggcacgct tcgatacatt attctgtgca cataagttac cacacaaaat 9120
   ttattgtacc accatccgac aacgtcgtta ttagggttga acacgttggc gatgcgcagc 9180
   agtttcccgt ttctcatgaa atattcaaag cggcccaaaa taatttgcaa gcaatccaac 9240
   atgtcttgag aaatttctcg ttcaaaattg ttcaaagaga atatctgcca tccgttttga 9300
   acgcgcacgc tgacgggaac caccgcatcg atttgctcca acacttcacg gacgttatcg 9360
   tcgatgccca tcgtttcgct ggtgctgaac caatgggaaa ggctcttgat ggaatcgccc 9420
   gcgtctatca tcttgaccgc ttcgtcaaag gtgcaactgc cgctcttcaa acgccgcata 9480
   gcggtcacgt cccgctctat gcacgacata ccgtttacgt acgattctga taggtattcc 9540
   tgaactatac ggtaatggtg atacgactcg ccatacacgt cgtgcacctc attgtattta 9600
   gcataataat tgtaaattat taactttgca gcgagagaca tgttgtcagt aaagcggtgc 9660
   taggctcaat aatactgatg tacaggcacg cgtgctattt atatataatt tcgcaaggag 9720
   gggagctgtt atcggttgct attattaaag aatggccgtc tgtttttatc acaagcttgg 9780
   cagcctcaac catgaagcgt cgtcattgta aattaaattc tctgcctcaa gaattatttg 9840
   acaagattgt cgagtattta tctttatctg attactgcaa tttggtgctt gtctgtaaaa 9900
   gaccttctag taaatataac gtgatatttg atagtactaa tcaccaacat ttgaaaggcg 9960
   tgtacaaaaa gacagacgtg caaataacaa gctacaacga 10000
<210> 6
   <211> 21
   <212> DNA
   <213> Artificial Sequence
<220>
   <223> Baculo-Forward primer
<400> 6
   actgttttcg taacagtttt g 21
<210> 7
   <211> 19
   <212> DNA
   <213> Artificial Sequence
<220>
   <223> Baculo-Reverse primer
<400> 7
   acaacgcaca gaatctagc 19
<210> 8
   <211> 14000
   <212> DNA
   <213> Artificial Sequence
<220>
   <223> Polyhedrin locus of vBacHTS2
<220>
   <221> source
   <222> (4687)..(11207)
   <223> F pBACe3.6
<220>
   <221> misc_feature
   <222> (10307)..(10966)
   <223> F CmR
<220>
   <221> promoter
   <222> (4428)..(4520)
   <223> F pPolyhedrin
<220>
   <221> misc_feature
   <222> (11220)..(11344)
   <223> R attR2
<220>
   <221> misc_feature
   <222> (4537)..(4661)
   <223> F attR1
<400> 8
   gaattctacc cgtaaagcga gtttagtttt gaaaaacaaa tgacatcatt tgtataatga 60
   catcatcccc tgattgtgtt ttacaagtag aattctatcc gtaaagcgag ttcagttttg 120
   aaaacaaatg agtcatacct aaacacgtta ataatcttct gatatcagct tatgactcaa 180
   gttatgagcc gtgtgcaaaa catgagataa gtttatgaca tcatccactg atcgtgcgtt 240
   acaagtagaa ttctactcgt aaagccagtt cggttatgag ccgtgtgcaa aacatgacat 300
   cagcttatga ctcatacttg attgtgtttt acgcgtagaa ttctactcgt aaagcgagtt 360
   cggttatgag ccgtgtgcaa aacatgacat cagcttatga gtcataatta atcgtgcgtt 420
   acaagtagaa ttctactcgt aaagcgagtt gaaggatcat atttagttgc gtttatgaga 480
   taagattgaa agcacgtgta aaatgtttcc cgcgcgttgg cacaactatt tacaatgcgg 540
   ccaagttata aaagattcta atctgatatg ttttaaaaca cctttgcggc ccgagttgtt 600
   tgcgtacgtg actagcgaag aagatgtgtg gaccgcagaa cagatagtaa aacaaaaccc 660
   tagtattgga gcaataatcg atttaaccaa cacgtctaaa tattatgatg gtgtgcattt 720
   tttgcgggcg ggcctgttat acaaaaaaat tcaagtacct ggccagactt tgccgcctga 780
   aagcatagtt caagaattta ttgacacggt aaaagaattt acagaaaagt gtcccggcat 840
   gttggtgggc gtgcactgca cacacggtat taatcgcacc ggttacatgg tgtgcagata 900
   tttaatgcac accctgggta ttgcgccgca ggaagccata gatagattcg aaaaagccag 960
   aggtcacaaa attgaaagac aaaattacgt tcaagattta ttaatttaat taatattatt 1020
   tgcattcttt aacaaatact ttatcctatt ttcaaattgt tgcgcttctt ccagcgaacc 1080
   aaaactatgc ttcgcttgct ccgtttagct tgtagccgat cagtggcgtt gttccaatcg 1140
   acggtaggat taggccggat attctccacc acaatgttgg caacgttgat gttacgttta 1200
   tgcttttggt tttccacgta cgtcttttgg ccggtaatag ccgtaaacgt agtgccgtcg 1260
   cgcgtcacgc acaacaccgg atgtttgcgc ttgtccgcgg ggtattgaac cgcgcgatcc 1320
   gacaaatcca ccactttggc aactaaatcg gtgacctgcg cgtctttttt ctgcattatt 1380
   tcgtctttct tttgcatggt ttcctggaag ccggtgtaca tgcggtttag atcagtcatg 1440
   acgcgcgtga cctgcaaatc tttggcctcg atctgcttgt ccttgatggc aacgatgcgt 1500
   tcaataaact cttgtttttt aacaagttcc tcggtttttt gcgccaccac cgcttgcagc 1560
   gcgtttgtgt gctcggtgaa tgtcgcaatc agcttagtca ccaactgttt gctctcctcc 1620
   tcccgttgtt tgatcgcggg atcgtacttg ccggtgcaga gcacttgagg aattacttct 1680
   tctaaaagcc attcttgtaa ttctatggcg taaggcaatt tggacttcat aatcagctga 1740
   atcacgccgg atttagtaat gagcactgta tgcggctgca aatacagcgg gtcgcccctt 1800
   ttcacgacgc tgttagaggt agggccccca ttttggatgg tctgctcaaa taacgatttg 1860
   tatttattgt ctacatgaac acgtatagct ttatcacaaa ctgtatattt taaactgtta 1920
   gcgacgtcct tggccacgaa ccggacctgt tggtcgcgct ctagcacgta ccgcaggttg 1980
   aacgtatctt ctccaaattt aaattctcca attttaacgc gagccatttt gatacacgtg 2040
   tgtcgatttt gcaacaacta ttgtttttta acgcaaacta aacttattgt ggtaagcaat 2100
   aattaaatat gggggaacat gcgccgctac aacactcgtc gttatgaacg cagacggcgc 2160
   cggtctcggc gcaagcggct aaaacgtgtt gcgcgttcaa cgcggcaaac atcgcaaaag 2220
   ccaatagtac agttttgatt tgcatattaa cggcgatttt ttaaattatc ttatttaata 2280
   aatagttatg acgcctacaa ctccccgccc gcgttgactc gctgcacctc gagcagttcg 2340
   ttgacgcctt cctccgtgtg gccgaacacg tcgagcgggt ggtcgatgac cagcggcgtg 2400
   ccgcacgcga cgcacaagta tctgtacacc gaatgatcgt cgggcgaagg cacgtcggcc 2460
   tccaagtggc aatattggca aattcgaaaa tatatacagt tgggttgttt gcgcatatct 2520
   atcgtggcgt tgggcatgta cgtccgaacg ttgatttgca tgcaagccga aattaaatca 2580
   ttgcgattag tgcgattaaa acgttgtaca tcctcgcttt taatcatgcc gtcgattaaa 2640
   tcgcgcaatc gagtcaagtg atcaaagtgt ggaataatgt tttctttgta ttcccgagtc 2700
   aagcgcagcg cgtattttaa caaactagcc atcttgtaag ttagtttcat ttaatgcaac 2760
   tttatccaat aatatattat gtatcgcacg tcaagaatta acaatgcgcc cgttgtcgca 2820
   tctcaacacg actatgatag agatcaaata aagcgcgaat taaatagctt gcgacgcaac 2880
   gtgcacgatc tgtgcacgcg ttccggcacg agctttgatt gtaataagtt tttacgaagc 2940
   gatgacatga cccccgtagt gacaacgatc acgcccaaaa gaactgccga ctacaaaatt 3000
   accgagtatg tcggtgacgt taaaactatt aagccatcca atcgaccgtt agtcgaatca 3060
   ggaccgctgg tgcgagaagc cgcgaagtat ggcgaatgca tcgtataacg tgtggagtcc 3120
   gctcattaga gcgtcatgtt tagacaagaa agctacatat ttaattgatc ccgatgattt 3180
   tattgataaa ttgaccctaa ctccatacac ggtattctac aatggcgggg ttttggtcaa 3240
   aatttccgga ctgcgattgt acatgctgtt aacggctccg cccactatta atgaaattaa 3300
   aaattccaat tttaaaaaac gcagcaagag aaacatttgt atgaaagaat gcgtagaagg 3360
   aaagaaaaat gtcgtcgaca tgctgaacaa caagattaat atgcctccgt gtataaaaaa 3420
   aatattgaac gatttgaaag aaaacaatgt accgcgcggc ggtatgtaca ggaagaggtt 3480
   tatactaaac tgttacattg caaacgtggt ttcgtgtgcc aagtgtgaaa accgatgttt 3540
   aatcaaggct ctgacgcatt tctacaacca cgactccaag tgtgtgggtg aagtcatgca 3600
   tcttttaatc aaatcccaag atgtgtataa accaccaaac tgccaaaaaa tgaaaactgt 3660
   cgacaagctc tgtccgtttg ctggcaactg caagggtctc aatcctattt gtaattattg 3720
   aataataaaa caattataaa tgctaaattt gttttttatt aacgatacaa accaaacgca 3780
   acaagaacat ttgtagtatt atctataatt gaaaacgcgt agttataatc gctgaggtaa 3840
   tatttaaaat cattttcaaa tgattcacag ttaatttgcg acaatataat tttattttca 3900
   cataaactag acgccttgtc gtcttcttct tcgtattcct tctctttttc atttttctcc 3960
   tcataaaaat taacatagtt attatcgtat ccatatatgt atctatcgta tagagtaaat 4020
   tttttgttgt cataaatata tatgtctttt ttaatggggt gtatagtacc gctgcgcata 4080
   gtttttctgt aatttacaac agtgctattt tctggtagtt cttcggagtg tgttgcttta 4140
   attattaaat ttatataatc aatgaatttg ggatcgtcgg ttttgtacaa tatgttgccg 4200
   gcatagtacg cagcttcttc tagttcaatt acaccatttt ttagcagcac cggattaaca 4260
   taactttcca aaatgttgta cgaaccgtta aacaaaaaca gttcacctcc cttttctata 4320
   ctattgtctg cgagcagttg tttgttgtta aaaataacag ccattgtaat gagacgcaca 4380
   aactaatatc acaaactgga aatgtctatc aatatatagt tgctgatatc atggagataa 4440
   ttaaaatgat aaccatctcg caaataaata agtattttac tgttttcgta acagttttgt 4500
   aataaaaaaa cctataaata cggatcccgg ggtaccacaa gtttgtacaa aaaagctgaa 4560
   cgagaaacgt aaaatgatat aaatatcaat atattaaatt agattttgca taaaaaacag 4620
   actacataat actgtaaaac acaacatatc cagtcactat ggcggccgca ttaggcaccc 4680
   cttaggtagg gtcaccgtcg acagcgacac acttgcatcg gatgcagccc ggttaacgtg 4740
   ccggcacggc ctgggtaacc aggtattttg tccacataac cgtgcgcaaa atgttgtgga 4800
   taagcaggac acagcagcaa tccacagcag gcatacaacc gcacaccgag gttactccgt 4860
   tctacaggtt acgacgacat gtcaatactt gcccttgaca ggcattgatg gaatcgtagt 4920
   ctcacgctga tagtctgatc gacaatacaa gtgggaccgt ggtcccagac cgataatcag 4980
   accgacaaca cgagtgggat cgtggtccca gactaataat cagaccgacg atacgagtgg 5040
   gaccgtggtc ccagactaat aatcagaccg acgatacgag tgggaccgtg gttccagact 5100
   aataatcaga ccgacgatac gagtgggacc gtggtcccag actaataatc agaccgacga 5160
   tacgagtggg accatggtcc cagactaata atcagaccga cgatacgagt gggaccgtgg 5220
   tcccagtctg attatcagac cgacgatacg agtgggaccg tggtcccaga ctaataatca 5280
   gaccgacgat acgagtggga ccgtggtccc agactaataa tcagaccgac gatacgagtg 5340
   ggaccgtggt cccagtctga ttatcagacc gacgatacaa gtggaacagt gggcccagag 5400
   agaatattca ggccagttat gctttctggc ctgtaacaaa ggacattaag taaagacaga 5460
   taaacgtaga ctaaaacgtg gtcgcatcag ggtgctggct tttcaagttc cttaagaatg 5520
   gcctcaattt tctctataca ctcagttgga acacgagacc tgtccaggtt aagcaccatt 5580
   ttatcgccct tatacaatac tgtcgctcca ggagcaaact gatgtcgtga gcttaaacta 5640
   gttcttgatg cagatgacgt tttaagcaca gaagttaaaa gagtgataac ttcttcagct 5700
   tcaaatatca ccccagcttt tttctgctca tgaaggttag atgcctgctg cttaagtaat 5760
   tcctctttat ctgtaaaggc tttttgaagt gcatcacctg accgggcaga tagttcaccg 5820
   gggtgagaaa aaagagcaac aactgattta ggcaatttgg cggtgttgat acagcgggta 5880
   ataatcttac gtgaaatatt ttccgcatca gccagcgcag aaatatttcc agcaaattca 5940
   ttctgcaatc ggcttgcata acgctgacca cgttcataag cacttgttgg gcgataatcg 6000
   ttacccaatc tggataatgc agccatctgc tcatcatcca gctcgccaac cagaacacga 6060
   taatcacttt cggtaagtgc agcagcttta cgacggcgac tcccatcggc aatttctatg 6120
   acaccagata ctcttcgacc gaacgccggt gtctgttgac cagtcagtag aaaagaaggg 6180
   atgagatcat ccagtgcgtc ctcagtaagc agctcctggt cacgttcatt acctgaccat 6240
   acccgagagg tcttctcaac actatcaccc cggagcactt caagagtaaa cttcacatcc 6300
   cgaccacata caggcaaagt aatggcatta ccgcgagcca ttactcctac gcgcgcaatt 6360
   aacgaatcca ccatcggggc agctggtgtc gataacgaag tatcttcaac cggttgagta 6420
   ttgagcgtat gttttggaat aacaggcgca cgcttcatta tctaatctcc cagcgtggtt 6480
   taatcagacg atcgaaaatt tcattgcaga caggttccca aatagaaaga gcatttctcc 6540
   aggcaccagt tgaagagcgt tgatcaatgg cctgttcaaa aacagttctc atccggatct 6600
   gacctttacc aacttcatcc gtttcacgta caacattttt tagaaccatg cttccccagg 6660
   catcccgaat ttgctcctcc atccacgggg actgagagcc attactattg ctgtatttgg 6720
   taagcaaaat acgtacatca ggctcgaacc ctttaagatc aacgttcttg agcagatcac 6780
   gaagcatatc gaaaaactgc agtgcggagg tgtagtcaaa caactcagca ggcgtgggaa 6840
   caatcagcac atcagcagca catacgacat taatcgtgcc gatacccagg ttaggcgcgc 6900
   tgtcaataac tatgacatca tagtcatgag caacagtttc aatggccagt cggagcatca 6960
   ggtgtggatc ggtgggcagt ttaccttcat caaatttgcc cattaactca gtttcaatac 7020
   ggtgcagagc cagacaggaa ggaataatgt caagccccgg ccagcaagtg ggctttattg 7080
   cataagtgac atcgtccttt tccccaagat agaaaggcag gagagtgtct tctgcatgaa 7140
   tatgaagatc tggtacccat ccgtgataca ttgaggctgt tccctggggg tcgttacctt 7200
   ccacgagcaa aacacgtagc cccttcagag ccagatcctg agcaagatga acagaaactg 7260
   aggttttgta aacgccacct ttatgggcag caaccccgat caccggtgga aatacgtctt 7320
   cagcacgtcg caatcgcgta ccaaacacat cacgcatatg attaatttgt tcaattgtat 7380
   aaccaacacg ttgctcaacc cgtcctcgaa tttccatatc cgggtgcggt agtcgccctg 7440
   ctttctcggc atctctgata gcctgagaag aaaccccaac taaatccgct gcttcaccta 7500
   ttctccagcg ccgggttatt ttcctcgctt ccgggctgtc atcattaaac tgtgcaatgg 7560
   cgatagcctt cgtcatttca tgaccagcgt ttatgcactg gttaagtgtt tccatgagtt 7620
   tcattctgaa catcctttaa tcattgcttt gcgttttttt attaaatctt gcaatttact 7680
   gcaaagcaac aacaaaatcg caaagtcatc aaaaaaccgc aaagttgttt aaaataagag 7740
   caacactaca aaaggagata agaagagcac atacctcagt cacttattat cactagcgct 7800
   cgccgcagcc gtgtaaccga gcatagcgag cgaactggcg aggaagcaaa gaagaactgt 7860
   tctgtcagat agctcttacg ctcagcgcaa gaagaaatat ccaccgtggg aaaaactcca 7920
   ggtagaggta cacacgcgga tagccaattc agagtaataa actgtgataa tcaaccctca 7980
   tcaatgatga cgaactaacc cccgatatca ggtcacatga cgaagggaaa gagaaggaaa 8040
   tcaactgtga caaactgccc tcaaatttgg cttccttaaa aattacagtt caaaaagtat 8100
   gagaaaatcc atgcaggctg aaggaaacag caaaactgtg acaaattacc ctcagtaggt 8160
   cagaacaaat gtgacgaacc accctcaaat ctgtgacaga taaccctcag actatcctgt 8220
   cgtcatggaa gtgatatcgc ggaaggaaaa tacgatatga gtcgtctggc ggcctttctt 8280
   tttctcaatg tatgagaggc gcattggagt tctgctgttg atctcattaa cacagacctg 8340
   caggaagcgg cggcggaagt caggcatacg ctggtaactt tgaggcagct ggtaacgctc 8400
   tatgatccag tcgattttca gagagacgat gcctgagcca tccggcttac gatactgaca 8460
   cagggattcg tataaacgca tggcatacgg attggtgatt tcttttgttt cactaagccg 8520
   aaactgcgta aaccggttct gtaacccgat aaagaaggga atgagatatg ggttgatatg 8580
   tacactgtaa agccctctgg atggactgtg cgcacgtttg ataaaccaag gaaaagattc 8640
   atagcctttt tcatcgccgg catcctcttc agggcgataa aaaaccactt ccttccccgc 8700
   gaaactcttc aatgcctgcc gtatatcctt actggcttcc gcagaggtca atccgaatat 8760
   ttcagcatat ttagcaacat ggatctcgca gataccgtca tgttcctgta gggtgccatc 8820
   agattttctg atctggtcaa cgaacagata cagcatacgt tttgatcccc gggagagact 8880
   atatgccgcc tcagtgaggt cgtttgactg gacgattcgc gggctatttt tacgtttctt 8940
   gtgattgata accgctgttt ccgccatgac agatccatgt gaagtgtgac aagtttttag 9000
   attgtcacac taaataaaaa agagtcaata agcagggata actttgtgaa aaaacagctt 9060
   cttctgaggg caatttgtca cagggttaag ggcaatttgt cacagacagg actgtcattt 9120
   gagggtgatt tgtcacactg aaagggcaat ttgtcacaac accttctcta gaaccagcat 9180
   ggataaaggc ctacaaggcg ctctaaaaaa gaagatctaa aaactataaa aaaaataatt 9240
   ataaaaatat ccccgtggat aagtggataa ccccaaggga agttttttca ggcatcgtgt 9300
   gtaagcagaa tatataagtg ctgttccctg gtgcttcctc gctcactcga gggcttcgcc 9360
   ctgtcgctca actgcggcga gcactactgg ctgtaaaagg acagaccaca tcatggttct 9420
   gtgttcatta ggttgttctg tccattgctg acataatccg ctccacttca acgtaacacc 9480
   gcacgaagat ttctattgtt cctgaaggca tattcaaatc gttttcgtta ccgcttgcag 9540
   gcatcatgac agaacactac ttcctataaa cgctacacag gctcctgaga ttaataatgc 9600
   ggatctctac gataatggga gattttcccg actgtttcgt tcgcttctca gtggataaca 9660
   gccagcttct ctgtttaaca gacaaaaaca gcatatccac tcagttccac atttccatat 9720
   aaaggccaag gcatttattc tcaggataat tgtttcagca tcgcaaccgc atcagactcc 9780
   ggcatcgcaa actgcacccg gtgccgggca gccacatcca gcgcaaaaac cttcgtgtag 9840
   acttccgttg aactgatgga cttatgtccc atcaggcttt gcagaacttt cagcggtata 9900
   ccggcataca gcatgtgcat cgcataggaa tggcggaacg tatgtggtgt gaccggaaca 9960
   gagaacgtca caccgtcagc agcagcggcg gcaaccgcct ccccaatcca ggtcctgacc 10020
   gttctgtccg tcacttccca gatccgcgct ttctctgtcc ttcctgtgcg acggttacgc 10080
   cgctccatga gcttatcgcg aataaatacc tgtgacggaa gatcacttcg cagaataaat 10140
   aaatcctggt gtccctgttg ataccgggaa gccctgggcc aacttttggc gaaaatgaga 10200
   cgttgatcgg cacgtaagag gttccaactt tcaccataat gaaataagat cactaccggg 10260
   cgtatttttt gagttatcga gattttcagg agctaaggaa gctaaaatgg agaaaaaaat 10320
   cactggatat accaccgttg atatatccca atggcatcgt aaagaacatt ttgaggcatt 10380
   tcagtcagtt gctcaatgta cctataacca gaccgttcag ctggatatta cggccttttt 10440
   aaagaccgta aagaaaaata agcacaagtt ttatccggcc tttattcaca ttcttgcccg 10500
   cctgatgaat gctcatccgg agttccgtat ggcaatgaaa gacggtgagc tggtgatatg 10560
   ggatagtgtt cacccttgtt acaccgtttt ccatgagcaa actgaaacgt tttcatcgct 10620
   ctggagtgaa taccacgacg atttccggca gtttctacac atatattcgc aagatgtggc 10680
   gtgttacggt gaaaacctgg cctatttccc taaagggttt attgagaata tgtttttcgt 10740
   ctcagccaat ccctgggtga gtttcaccag ttttgattta aacgtggcca atatggacaa 10800
   cttcttcgcc cccgttttca ccatgggcaa atattatacg caaggcgaca aggtgctgat 10860
   gccgctggcg attcaggttc atcatgccgt ttgtgatggc ttccatgtcg gcagaatgct 10920
   taatgaatta caacagtact gcgatgagtg gcagggcggg gcgtaatttt tttaaggcag 10980
   ttattggtgc ccttaaacgc ctggttgcta cgcctgaata agtgataata agcggatgaa 11040
   tggcagaaat tcgatgataa gctgtcaaac atgagaattg gtcgacggcg cgccaaagct 11100
   tgcatgcctg cagccgcgta acctggcaaa atcggttacg gttgagtaat aaatggatgc 11160
   cctgcgtaag cggggcacat ttcattacct ctttctccgc acccgaccct caggtcgacc 11220
   atagtgactg gatatgttgt gttttacagt attatgtagt ctgtttttta tgcaaaatct 11280
   aatttaatat attgatattt atatcatttt acgtttctcg ttcagctttc ttgtacaaag 11340
   tggtctcgag ttaattaatt gatccgggtt attagtacat ttattaagcg ctagattctg 11400
   tgcgttgttg atttacagac aattgttgta cgtattttaa taattcatta aatttataat 11460
   ctttagggtg gtatgttaga gcgaaaatca aatgattttc agcgtcttta tatctgaatt 11520
   taaatattaa atcctcaata gatttgtaaa ataggtttcg attagtttca aacaagggtt 11580
   gtttttccga accgatggct ggactatcta atggattttc gctcaacgcc acaaaacttg 11640
   ccaaatcttg tagcagcaat ctagctttgt cgatattcgt ttgtgttttg ttttgtaata 11700
   aaggttcgac gtcgttcaaa atattatgcg cttttgtatt tctttcatca ctgtcgttag 11760
   tgtacaattg actcgacgta aacacgttaa ataaagcttg gacatattta acatcgggcg 11820
   tgttagcttt attaggccga ttatcgtcgt cgtcccaacc ctcgtcgtta gaagttgctt 11880
   ccgaagacga ttttgccata gccacacgac gcctattaat tgtgtcggct aacacgtccg 11940
   cgatcaaatt tgtagttgag ctttttggaa ttatttctga ttgcgggcgt ttttgggcgg 12000
   gtttcaatct aactgtgccc gattttaatt cagacaacac gttagaaagc gatggtgcag 12060
   gcggtggtaa catttcagac ggcaaatcta ctaatggcgg cggtggtgga gctgatgata 12120
   aatctaccat cggtggaggc gcaggcgggg ctggcggcgg aggcggaggc ggaggtggtg 12180
   gcggtgatgc agacggcggt ttaggctcaa atgtctcttt aggcaacaca gtcggcacct 12240
   caactattgt actggtttcg ggcgccgttt ttggtttgac cggtctgaga cgagtgcgat 12300
   ttttttcgtt tctaatagct tccaacaatt gttgtctgtc gtctaaaggt gcagcgggtt 12360
   gaggttccgt cggcattggt ggagcgggcg gcaattcaga catcgatggt ggtggtggtg 12420
   gtggaggcgc tggaatgtta ggcacgggag aaggtggtgg cggcggtgcc gccggtataa 12480
   tttgttctgg tttagtttgt tcgcgcacga ttgtgggcac cggcgcaggc gccgctggct 12540
   gcacaacgga aggtcgtctg cttcgaggca gcgcttgggg tggtggcaat tcaatattat 12600
   aattggaata caaatcgtaa aaatctgcta taagcattgt aatttcgcta tcgtttaccg 12660
   tgccgatatt taacaaccgc tcaatgtaag caattgtatt gtaaagagat tgtctcaagc 12720
   tcggatcccg cacgccgata acaagccttt tcatttttac tacagcattg tagtggcgag 12780
   acacttcgct gtcgtcgacg tacatgtatg ctttgttgtc aaaaacgtcg ttggcaagct 12840
   ttaaaatatt taaaagaaca tctctgttca gcaccactgt gttgtcgtaa atgttgtttt 12900
   tgataatttg cgcttccgca gtatcgacac gttcaaaaaa ttgatgcgca tcaattttgt 12960
   tgttcctatt attgaataaa taagattgta cagattcata tctacgattc gtcatggcca 13020
   ccacaaatgc tacgctgcaa acgctggtac aatttacgga aaactgcaaa aacgtcaaaa 13080
   ctcggtataa aataatcaac gggcgctttg gcaaaatatc tattttatcg cacaagccca 13140
   ctagcaaatt gtatttgcag aaaacaattt cggcgcacaa ttttaacgct gacgaaataa 13200
   aagttcacca gttaatgagc gaccacccaa attttataaa aatctatttt aatcacggtt 13260
   ccatcaacaa ccaagtgatc gtgatggact acattgactg tcccgattta tttgaaacac 13320
   tacaaattaa aggcgagctt tcgtaccaac ttgttagcaa tattattaga cagctgtgtg 13380
   aagcgctcaa cgatttgcac aagcacaatt tcatacacaa cgacataaaa ctcgaaaatg 13440
   tcttatattt cgaagcactt gatcgcgtgt atgtttgcga ttacggattg tgcaaacacg 13500
   aaaactcact tagcgtgcac gacggcacgt tggagtattt tagtccggaa aaaattcgac 13560
   acacaactat gcacgtttcg tttgactggt acgccgtcgg cgtgttaaca tacaagttgc 13620
   taaccggcgg ccgacaccca tttgaaaaaa gcgaagacga aatgttggac ttgaatagca 13680
   tgaagcgtcg tcagcaatac aatgacattg gcgttttaaa acacgttcgt aacgttaacg 13740
   ctcgtgactt tgtgtactgc ctaacaagat acaacataga ttgtagactc acaaattaca 13800
   aacaaattat aaaacatgag tttttgtcgt aaaaatgcca cttgttttac gagtagaatt 13860
   ctacgtgtaa cacacgatct aaaagatgat gtcatttttt atcaatgact catttgtttt 13920
   aaaacagact tgttttacga gtagaattct acgtgtaaag catgatcgtg agtggtgtta 13980
   ataaaatcat aaaaattatt 14000
<210> 9
   <211> 13100
   <212> DNA
   <213> Artificial Sequence
<220>
   <223> Polyhedrin locus of vBacHTS2_GFP
<220>
   <221> source
   <222> (4523)..(5252)
   <223> F EGFP
<220>
   <221> source
   <222> (5404)..(11924)
   <223> F pBACe3.6
<220>
   <221> CDS
   <222> (4531)..(5247)
   <223> F EGFP
<220>
   <221> misc_feature
   <222> (11024)..(11683)
   <223> F CmR
<220>
   <221> promoter
   <222> (4428)..(4520)
   <223> F pPolyhedrin
<220>
   <221> misc_feature
   <222> (11937)..(12061)
   <223> R attR2
<220>
   <221> misc_feature
   <222> (5254)..(5378)
   <223> F attR1
<400> 9
   gaattctacc cgtaaagcga gtttagtttt gaaaaacaaa tgacatcatt tgtataatga 60
   catcatcccc tgattgtgtt ttacaagtag aattctatcc gtaaagcgag ttcagttttg 120
   aaaacaaatg agtcatacct aaacacgtta ataatcttct gatatcagct tatgactcaa 180
   gttatgagcc gtgtgcaaaa catgagataa gtttatgaca tcatccactg atcgtgcgtt 240
   acaagtagaa ttctactcgt aaagccagtt cggttatgag ccgtgtgcaa aacatgacat 300
   cagcttatga ctcatacttg attgtgtttt acgcgtagaa ttctactcgt aaagcgagtt 360
   cggttatgag ccgtgtgcaa aacatgacat cagcttatga gtcataatta atcgtgcgtt 420
   acaagtagaa ttctactcgt aaagcgagtt gaaggatcat atttagttgc gtttatgaga 480
   taagattgaa agcacgtgta aaatgtttcc cgcgcgttgg cacaactatt tacaatgcgg 540
   ccaagttata aaagattcta atctgatatg ttttaaaaca cctttgcggc ccgagttgtt 600
   tgcgtacgtg actagcgaag aagatgtgtg gaccgcagaa cagatagtaa aacaaaaccc 660
   tagtattgga gcaataatcg atttaaccaa cacgtctaaa tattatgatg gtgtgcattt 720
   tttgcgggcg ggcctgttat acaaaaaaat tcaagtacct ggccagactt tgccgcctga 780
   aagcatagtt caagaattta ttgacacggt aaaagaattt acagaaaagt gtcccggcat 840
   gttggtgggc gtgcactgca cacacggtat taatcgcacc ggttacatgg tgtgcagata 900
   tttaatgcac accctgggta ttgcgccgca ggaagccata gatagattcg aaaaagccag 960
   aggtcacaaa attgaaagac aaaattacgt tcaagattta ttaatttaat taatattatt 1020
   tgcattcttt aacaaatact ttatcctatt ttcaaattgt tgcgcttctt ccagcgaacc 1080
   aaaactatgc ttcgcttgct ccgtttagct tgtagccgat cagtggcgtt gttccaatcg 1140
   acggtaggat taggccggat attctccacc acaatgttgg caacgttgat gttacgttta 1200
   tgcttttggt tttccacgta cgtcttttgg ccggtaatag ccgtaaacgt agtgccgtcg 1260
   cgcgtcacgc acaacaccgg atgtttgcgc ttgtccgcgg ggtattgaac cgcgcgatcc 1320
   gacaaatcca ccactttggc aactaaatcg gtgacctgcg cgtctttttt ctgcattatt 1380
   tcgtctttct tttgcatggt ttcctggaag ccggtgtaca tgcggtttag atcagtcatg 1440
   acgcgcgtga cctgcaaatc tttggcctcg atctgcttgt ccttgatggc aacgatgcgt 1500
   tcaataaact cttgtttttt aacaagttcc tcggtttttt gcgccaccac cgcttgcagc 1560
   gcgtttgtgt gctcggtgaa tgtcgcaatc agcttagtca ccaactgttt gctctcctcc 1620
   tcccgttgtt tgatcgcggg atcgtacttg ccggtgcaga gcacttgagg aattacttct 1680
   tctaaaagcc attcttgtaa ttctatggcg taaggcaatt tggacttcat aatcagctga 1740
   atcacgccgg atttagtaat gagcactgta tgcggctgca aatacagcgg gtcgcccctt 1800
   ttcacgacgc tgttagaggt agggccccca ttttggatgg tctgctcaaa taacgatttg 1860
   tatttattgt ctacatgaac acgtatagct ttatcacaaa ctgtatattt taaactgtta 1920
   gcgacgtcct tggccacgaa ccggacctgt tggtcgcgct ctagcacgta ccgcaggttg 1980
   aacgtatctt ctccaaattt aaattctcca attttaacgc gagccatttt gatacacgtg 2040
   tgtcgatttt gcaacaacta ttgtttttta acgcaaacta aacttattgt ggtaagcaat 2100
   aattaaatat gggggaacat gcgccgctac aacactcgtc gttatgaacg cagacggcgc 2160
   cggtctcggc gcaagcggct aaaacgtgtt gcgcgttcaa cgcggcaaac atcgcaaaag 2220
   ccaatagtac agttttgatt tgcatattaa cggcgatttt ttaaattatc ttatttaata 2280
   aatagttatg acgcctacaa ctccccgccc gcgttgactc gctgcacctc gagcagttcg 2340
   ttgacgcctt cctccgtgtg gccgaacacg tcgagcgggt ggtcgatgac cagcggcgtg 2400
   ccgcacgcga cgcacaagta tctgtacacc gaatgatcgt cgggcgaagg cacgtcggcc 2460
   tccaagtggc aatattggca aattcgaaaa tatatacagt tgggttgttt gcgcatatct 2520
   atcgtggcgt tgggcatgta cgtccgaacg ttgatttgca tgcaagccga aattaaatca 2580
   ttgcgattag tgcgattaaa acgttgtaca tcctcgcttt taatcatgcc gtcgattaaa 2640
   tcgcgcaatc gagtcaagtg atcaaagtgt ggaataatgt tttctttgta ttcccgagtc 2700
   aagcgcagcg cgtattttaa caaactagcc atcttgtaag ttagtttcat ttaatgcaac 2760
   tttatccaat aatatattat gtatcgcacg tcaagaatta acaatgcgcc cgttgtcgca 2820
   tctcaacacg actatgatag agatcaaata aagcgcgaat taaatagctt gcgacgcaac 2880
   gtgcacgatc tgtgcacgcg ttccggcacg agctttgatt gtaataagtt tttacgaagc 2940
   gatgacatga cccccgtagt gacaacgatc acgcccaaaa gaactgccga ctacaaaatt 3000
   accgagtatg tcggtgacgt taaaactatt aagccatcca atcgaccgtt agtcgaatca 3060
   ggaccgctgg tgcgagaagc cgcgaagtat ggcgaatgca tcgtataacg tgtggagtcc 3120
   gctcattaga gcgtcatgtt tagacaagaa agctacatat ttaattgatc ccgatgattt 3180
   tattgataaa ttgaccctaa ctccatacac ggtattctac aatggcgggg ttttggtcaa 3240
   aatttccgga ctgcgattgt acatgctgtt aacggctccg cccactatta atgaaattaa 3300
   aaattccaat tttaaaaaac gcagcaagag aaacatttgt atgaaagaat gcgtagaagg 3360
   aaagaaaaat gtcgtcgaca tgctgaacaa caagattaat atgcctccgt gtataaaaaa 3420
   aatattgaac gatttgaaag aaaacaatgt accgcgcggc ggtatgtaca ggaagaggtt 3480
   tatactaaac tgttacattg caaacgtggt ttcgtgtgcc aagtgtgaaa accgatgttt 3540
   aatcaaggct ctgacgcatt tctacaacca cgactccaag tgtgtgggtg aagtcatgca 3600
   tcttttaatc aaatcccaag atgtgtataa accaccaaac tgccaaaaaa tgaaaactgt 3660
   cgacaagctc tgtccgtttg ctggcaactg caagggtctc aatcctattt gtaattattg      3720
   aataataaaa caattataaa tgctaaattt gttttttatt aacgatacaa accaaacgca     3780
   acaagaacat ttgtagtatt atctataatt gaaaacgcgt agttataatc gctgaggtaa      3840
   tatttaaaat cattttcaaa tgattcacag ttaatttgcg acaatataat tttattttca      3900
   cataaactag acgccttgtc gtcttcttct tcgtattcct tctctttttc atttttctcc      3960
   tcataaaaat taacatagtt attatcgtat ccatatatgt atctatcgta tagagtaaat      4020
   tttttgttgt cataaatata tatgtctttt ttaatggggt gtatagtacc gctgcgcata      4080
   gtttttctgt aatttacaac agtgctattt tctggtagtt cttcggagtg tgttgcttta      4140
   attattaaat ttatataatc aatgaatttg ggatcgtcgg ttttgtacaa tatgttgccg      4200
   gcatagtacg cagcttcttc tagttcaatt acaccatttt ttagcagcac cggattaaca      4260
   taactttcca aaatgttgta cgaaccgtta aacaaaaaca gttcacctcc cttttctata      4320
   ctattgtctg cgagcagttg tttgttgtta aaaataacag ccattgtaat gagacgcaca      4380
   aactaatatc acaaactgga aatgtctatc aatatatagt tgctgatatc atggagataa      4440
   ttaaaatgat aaccatctcg caaataaata agtattttac tgttttcgta acagttttgt       4500
   aataaaaaaa cctataaata cggatccacc atg gtg agc aag ggc gag gag ctg       4554
                                    Met Val Ser Lys Gly Glu Glu Leu
                                    1               5
   ttc acc ggg gtg gtg ccc atc ctg gtc gag ctg gac ggc gac gta aac        4602
   Phe Thr Gly Val Val Pro Ile Leu Val Glu Leu Asp Gly Asp Val Asn
       10                  15                  20
   ggc cac aag ttc agc gtg tcc ggc gag ggc gag ggc gat gcc acc tac 4650
   Gly His Lys Phe Ser Val Ser Gly Glu Gly Glu Gly Asp Ala Thr Tyr
   25 30 35 40
   ggc aag ctg acc ctg aag ttc atc tgc acc acc ggc aag ctg ccc gtg 4698
   Gly Lys Leu Thr Leu Lys Phe Ile Cys Thr Thr Gly Lys Leu Pro Val
   45 50 55
   ccc tgg ccc acc ctc gtg acc acc ctg acc tgg ggc gtg cag tgc ttc 4746
   Pro Trp Pro Thr Leu Val Thr Thr Leu Thr Trp Gly Val Gln Cys Phe
   60 65 70
   agc cgc tac ccc gac cac atg aag cag cac gac ttc ttc aag tcc gcc 4794
   Ser Arg Tyr Pro Asp His Met Lys Gln His Asp Phe Phe Lys Ser Ala
   75 80 85
   atg ccc gaa ggc tac gtc cag gag cgc acc atc ttc ttc aag gac gac 4842
   Met Pro Glu Gly Tyr Val Gln Glu Arg Thr Ile Phe Phe Lys Asp Asp
   90 95 100
   ggc aac tac aag acc cgc gcc gag gtg aag ttc gag ggc gac acc ctg 4890
   Gly Asn Tyr Lys Thr Arg Ala Glu Val Lys Phe Glu Gly Asp Thr Leu
   105 110 115 120
   gtg aac cgc atc gag ctg aag ggc atc gac ttc aag gag gac ggc aac 4938
   Val Asn Arg Ile Glu Leu Lys Gly Ile Asp Phe Lys Glu Asp Gly Asn
   125 130 135
   atc ctg ggg cac aag ctg gag tac aac tac aac agc cac aac gtc tat 4986
   Ile Leu Gly His Lys Leu Glu Tyr Asn Tyr Asn Ser His Asn Val Tyr
   140 145 150
   atc atg gcc gac aag cag aag aac ggc atc aag gtg aac ttc aag atc 5034
   Ile Met Ala Asp Lys Gln Lys Asn Gly Ile Lys Val Asn Phe Lys Ile
   155 160 165
   cgc cac aac atc gag gac ggc agc gtg cag ctc gcc gac cac tac cag          5082
   Arg His Asn Ile Glu Asp Gly Ser Val Gln Leu Ala Asp His Tyr Gln
       170                 175                 180
   cag aac acc ccc atc ggc gac ggc ccc gtg ctg ctg ccc gac aac cac          5130
   Gln Asn Thr Pro Ile Gly Asp Gly Pro Val Leu Leu Pro Asp Asn His
   185                 190                 195                 200
   tac ctg agc acc cag tcc gcc ctg agc aaa gac ccc aac gag aag cgc          5178
   Tyr Leu Ser Thr Gln Ser Ala Leu Ser Lys Asp Pro Asn Glu Lys Arg
                   205                 210                 215
   gat cac atg gtc ctg ctg gag ttc gtg acc gcc gcc ggg atc act ctc          5226
   Asp His Met Val Leu Leu Glu Phe Val Thr Ala Ala Gly Ile Thr Leu
               220                 225                 230
   ggc atg gac gag ctg tac aag  ggt accacaagtt tgtacaaaaa agctgaacga        5280
   Gly Met Asp Glu Leu Tyr Lys
           235
   gaaacgtaaa atgatataaa tatcaatata ttaaattaga ttttgcataa aaaacagact        5340
   acataatact gtaaaacaca acatatccag tcactatggc ggccgcatta ggcacccctt        5400
   aggtagggtc accgtcgaca gcgacacact tgcatcggat gcagcccggt taacgtgccg        5460
   gcacggcctg ggtaaccagg tattttgtcc acataaccgt gcgcaaaatg ttgtggataa        5520
   gcaggacaca gcagcaatcc acagcaggca tacaaccgca caccgaggtt actccgttct        5580
   acaggttacg acgacatgtc aatacttgcc cttgacaggc attgatggaa tcgtagtctc        5640
   acgctgatag tctgatcgac aatacaagtg ggaccgtggt cccagaccga taatcagacc        5700
   gacaacacga gtgggatcgt ggtcccagac taataatcag accgacgata cgagtgggac        5760
   cgtggtccca gactaataat cagaccgacg atacgagtgg gaccgtggtt ccagactaat 5820
   aatcagaccg acgatacgag tgggaccgtg gtcccagact aataatcaga ccgacgatac 5880
   gagtgggacc atggtcccag actaataatc agaccgacga tacgagtggg accgtggtcc 5940
   cagtctgatt atcagaccga cgatacgagt gggaccgtgg tcccagacta ataatcagac 6000
   cgacgatacg agtgggaccg tggtcccaga ctaataatca gaccgacgat acgagtggga 6060
   ccgtggtccc agtctgatta tcagaccgac gatacaagtg gaacagtggg cccagagaga 6120
   atattcaggc cagttatgct ttctggcctg taacaaagga cattaagtaa agacagataa 6180
   acgtagacta aaacgtggtc gcatcagggt gctggctttt caagttcctt aagaatggcc 6240
   tcaattttct ctatacactc agttggaaca cgagacctgt ccaggttaag caccatttta 6300
   tcgcccttat acaatactgt cgctccagga gcaaactgat gtcgtgagct taaactagtt 6360
   cttgatgcag atgacgtttt aagcacagaa gttaaaagag tgataacttc ttcagcttca 6420
   aatatcaccc cagctttttt ctgctcatga aggttagatg cctgctgctt aagtaattcc 6480
   tctttatctg taaaggcttt ttgaagtgca tcacctgacc gggcagatag ttcaccgggg 6540
   tgagaaaaaa gagcaacaac tgatttaggc aatttggcgg tgttgataca gcgggtaata 6600
   atcttacgtg aaatattttc cgcatcagcc agcgcagaaa tatttccagc aaattcattc 6660
   tgcaatcggc ttgcataacg ctgaccacgt tcataagcac ttgttgggcg ataatcgtta 6720
   cccaatctgg ataatgcagc catctgctca tcatccagct cgccaaccag aacacgataa 6780
   tcactttcgg taagtgcagc agctttacga cggcgactcc catcggcaat ttctatgaca 6840
   ccagatactc ttcgaccgaa cgccggtgtc tgttgaccag tcagtagaaa agaagggatg 6900
   agatcatcca gtgcgtcctc agtaagcagc tcctggtcac gttcattacc tgaccatacc 6960
   cgagaggtct tctcaacact atcaccccgg agcacttcaa gagtaaactt cacatcccga 7020
   ccacatacag gcaaagtaat ggcattaccg cgagccatta ctcctacgcg cgcaattaac 7080
   gaatccacca tcggggcagc tggtgtcgat aacgaagtat cttcaaccgg ttgagtattg 7140
   agcgtatgtt ttggaataac aggcgcacgc ttcattatct aatctcccag cgtggtttaa 7200
   tcagacgatc gaaaatttca ttgcagacag gttcccaaat agaaagagca tttctccagg 7260
   caccagttga agagcgttga tcaatggcct gttcaaaaac agttctcatc cggatctgac 7320
   ctttaccaac ttcatccgtt tcacgtacaa cattttttag aaccatgctt ccccaggcat 7380
   cccgaatttg ctcctccatc cacggggact gagagccatt actattgctg tatttggtaa 7440
   gcaaaatacg tacatcaggc tcgaaccctt taagatcaac gttcttgagc agatcacgaa 7500
   gcatatcgaa aaactgcagt gcggaggtgt agtcaaacaa ctcagcaggc gtgggaacaa 7560
   tcagcacatc agcagcacat acgacattaa tcgtgccgat acccaggtta ggcgcgctgt 7620
   caataactat gacatcatag tcatgagcaa cagtttcaat ggccagtcgg agcatcaggt 7680
   gtggatcggt gggcagttta ccttcatcaa atttgcccat taactcagtt tcaatacggt 7740
   gcagagccag acaggaagga ataatgtcaa gccccggcca gcaagtgggc tttattgcat 7800
   aagtgacatc gtccttttcc ccaagataga aaggcaggag agtgtcttct gcatgaatat 7860
   gaagatctgg tacccatccg tgatacattg aggctgttcc ctgggggtcg ttaccttcca 7920
   cgagcaaaac acgtagcccc ttcagagcca gatcctgagc aagatgaaca gaaactgagg 7980
   ttttgtaaac gccaccttta tgggcagcaa ccccgatcac cggtggaaat acgtcttcag 8040
   cacgtcgcaa tcgcgtacca aacacatcac gcatatgatt aatttgttca attgtataac 8100
   caacacgttg ctcaacccgt cctcgaattt ccatatccgg gtgcggtagt cgccctgctt 8160
   tctcggcatc tctgatagcc tgagaagaaa ccccaactaa atccgctgct tcacctattc 8220
   tccagcgccg ggttattttc ctcgcttccg ggctgtcatc attaaactgt gcaatggcga 8280
   tagccttcgt catttcatga ccagcgttta tgcactggtt aagtgtttcc atgagtttca 8340
   ttctgaacat cctttaatca ttgctttgcg tttttttatt aaatcttgca atttactgca 8400
   aagcaacaac aaaatcgcaa agtcatcaaa aaaccgcaaa gttgtttaaa ataagagcaa 8460
   cactacaaaa ggagataaga agagcacata cctcagtcac ttattatcac tagcgctcgc 8520
   cgcagccgtg taaccgagca tagcgagcga actggcgagg aagcaaagaa gaactgttct 8580
   gtcagatagc tcttacgctc agcgcaagaa gaaatatcca ccgtgggaaa aactccaggt 8640
   agaggtacac acgcggatag ccaattcaga gtaataaact gtgataatca accctcatca 8700
   atgatgacga actaaccccc gatatcaggt cacatgacga agggaaagag aaggaaatca 8760
   actgtgacaa actgccctca aatttggctt ccttaaaaat tacagttcaa aaagtatgag 8820
   aaaatccatg caggctgaag gaaacagcaa aactgtgaca aattaccctc agtaggtcag 8880
   aacaaatgtg acgaaccacc ctcaaatctg tgacagataa ccctcagact atcctgtcgt 8940
   catggaagtg atatcgcgga aggaaaatac gatatgagtc gtctggcggc ctttcttttt 9000
   ctcaatgtat gagaggcgca ttggagttct gctgttgatc tcattaacac agacctgcag 9060
   gaagcggcgg cggaagtcag gcatacgctg gtaactttga ggcagctggt aacgctctat 9120
   gatccagtcg attttcagag agacgatgcc tgagccatcc ggcttacgat actgacacag 9180
   ggattcgtat aaacgcatgg catacggatt ggtgatttct tttgtttcac taagccgaaa 9240
   ctgcgtaaac cggttctgta acccgataaa gaagggaatg agatatgggt tgatatgtac 9300
   actgtaaagc cctctggatg gactgtgcgc acgtttgata aaccaaggaa aagattcata 9360
   gcctttttca tcgccggcat cctcttcagg gcgataaaaa accacttcct tccccgcgaa 9420
   actcttcaat gcctgccgta tatccttact ggcttccgca gaggtcaatc cgaatatttc 9480
   agcatattta gcaacatgga tctcgcagat accgtcatgt tcctgtaggg tgccatcaga 9540
   ttttctgatc tggtcaacga acagatacag catacgtttt tgatcccggg agagactata 9600
   tgccgcctca gtgaggtcgt ttgactggac gattcgcggg ctatttttac gtttcttgtg 9660
   attgataacc gctgtttccg ccatgacaga tccatgtgaa gtgtgacaag tttttagatt 9720
   gtcacactaa ataaaaaaga gtcaataagc agggataact ttgtgaaaaa acagcttctt 9780
   ctgagggcaa tttgtcacag ggttaagggc aatttgtcac agacaggact gtcatttgag 9840
   ggtgatttgt cacactgaaa gggcaatttg tcacaacacc ttctctagaa ccagcatgga 9900
   taaaggccta caaggcgctc taaaaaagaa gatctaaaaa ctataaaaaa aataattata 9960
   aaaatatccc cgtggataag tggataaccc caagggaagt tttttcaggc atcgtgtgta 10020
   agcagaatat ataagtgctg ttccctggtg cttcctcgct cactcgaggg cttcgccctg 10080
   tcgctcaact gcggcgagca ctactggctg taaaaggaca gaccacatca tggttctgtg 10140
   ttcattaggt tgttctgtcc attgctgaca taatccgctc cacttcaacg taacaccgca 10200
   cgaagatttc tattgttcct gaaggcatat tcaaatcgtt ttcgttaccg cttgcaggca 10260
   tcatgacaga acactacttc ctataaacgc tacacaggct cctgagatta ataatgcgga 10320
   tctctacgat aatgggagat tttcccgact gtttcgttcg cttctcagtg gataacagcc 10380
   agcttctctg tttaacagac aaaaacagca tatccactca gttccacatt tccatataaa 10440
   ggccaaggca tttattctca ggataattgt ttcagcatcg caaccgcatc agactccggc 10500
   atcgcaaact gcacccggtg ccgggcagcc acatccagcg caaaaacctt cgtgtagact 10560
   tccgttgaac tgatggactt atgtcccatc aggctttgca gaactttcag cggtataccg 10620
   gcatacagca tgtgcatcgc ataggaatgg cggaacgtat gtggtgtgac cggaacagag 10680
   aacgtcacac cgtcagcagc agcggcggca accgcctccc caatccaggt cctgaccgtt 10740
   ctgtccgtca cttcccagat ccgcgctttc tctgtccttc ctgtgcgacg gttacgccgc 10800
   tccatgagct tatcgcgaat aaatacctgt gacggaagat cacttcgcag aataaataaa 10860
   tcctggtgtc cctgttgata ccgggaagcc ctgggccaac ttttggcgaa aatgagacgt 10920
   tgatcggcac gtaagaggtt ccaactttca ccataatgaa ataagatcac taccgggcgt 10980
   attttttgag ttatcgagat tttcaggagc taaggaagct aaaatggaga aaaaaatcac 11040
   tggatatacc accgttgata tatcccaatg gcatcgtaaa gaacattttg aggcatttca 11100
   gtcagttgct caatgtacct ataaccagac cgttcagctg gatattacgg cctttttaaa 11160
   gaccgtaaag aaaaataagc acaagtttta tccggccttt attcacattc ttgcccgcct 11220
   gatgaatgct catccggagt tccgtatggc aatgaaagac ggtgagctgg tgatatggga 11280
   tagtgttcac ccttgttaca ccgttttcca tgagcaaact gaaacgtttt catcgctctg 11340
   gagtgaatac cacgacgatt tccggcagtt tctacacata tattcgcaag atgtggcgtg 11400
   ttacggtgaa aacctggcct atttccctaa agggtttatt gagaatatgt ttttcgtctc 11460
   agccaatccc tgggtgagtt tcaccagttt tgatttaaac gtggccaata tggacaactt 11520
   cttcgccccc gttttcacca tgggcaaata ttatacgcaa ggcgacaagg tgctgatgcc 11580
   gctggcgatt caggttcatc atgccgtttg tgatggcttc catgtcggca gaatgcttaa 11640
   tgaattacaa cagtactgcg atgagtggca gggcggggcg taattttttt aaggcagtta 11700
   ttggtgccct taaacgcctg gttgctacgc ctgaataagt gataataagc ggatgaatgg 11760
   cagaaattcg atgataagct gtcaaacatg agaattggtc gacggcgcgc caaagcttgc 11820
   atgcctgcag ccgcgtaacc tggcaaaatc ggttacggtt gagtaataaa tggatgccct 11880
   gcgtaagcgg ggcacatttc attacctctt tctccgcacc cgaccctcag gtcgaccata 11940
   gtgactggat atgttgtgtt ttacagtatt atgtagtctg ttttttatgc aaaatctaat 12000
   ttaatatatt gatatttata tcattttacg tttctcgttc agctttcttg tacaaagtgg 12060
   tctcgagtta attaattgat ccgggttatt agtacattta ttaagcgcta gattctgtgc 12120
   gttgttgatt tacagacaat tgttgtacgt attttaataa ttcattaaat ttataatctt 12180
   tagggtggta tgttagagcg aaaatcaaat gattttcagc gtctttatat ctgaatttaa 12240
   atattaaatc ctcaatagat ttgtaaaata ggtttcgatt agtttcaaac aagggttgtt 12300
   tttccgaacc gatggctgga ctatctaatg gattttcgct caacgccaca aaacttgcca 12360
   aatcttgtag cagcaatcta gctttgtcga tattcgtttg tgttttgttt tgtaataaag 12420
   gttcgacgtc gttcaaaata ttatgcgctt ttgtatttct ttcatcactg tcgttagtgt 12480
   acaattgact cgacgtaaac acgttaaata aagcttggac atatttaaca tcgggcgtgt 12540
   tagctttatt aggccgatta tcgtcgtcgt cccaaccctc gtcgttagaa gttgcttccg 12600
   aagacgattt tgccatagcc acacgacgcc tattaattgt gtcggctaac acgtccgcga 12660
   tcaaatttgt agttgagctt tttggaatta tttctgattg cgggcgtttt tgggcgggtt 12720
   tcaatctaac tgtgcccgat tttaattcag acaacacgtt agaaagcgat ggtgcaggcg 12780
   gtggtaacat ttcagacggc aaatctacta atggcggcgg tggtggagct gatgataaat 12840
   ctaccatcgg tggaggcgca ggcggggctg gcggcggagg cggaggcgga ggtggtggcg 12900
   gtgatgcaga cggcggttta ggctcaaatg tctctttagg caacacagtc ggcacctcaa 12960
   ctattgtact ggtttcgggc gccgtttttg gtttgaccgg tctgagacga gtgcgatttt 13020
   tttcgtttct aatagcttcc aacaattgtt gtctgtcgtc taaaggtgca gcgggttgag 13080
   gttccgtcgg cattggtgga 13100